# EUROPEAN PATENT SPECIFICATION

(11) **EP 1 726 247 B1**
(45) Date of publication and mention of the grant of the patent: **19.10.2011**
(21) Application number: 05720564.3
(22) Date of filing: 11.03.2005
(51) Int. Cl.: A61B 1/00

(54) **ENDOSCOPE INSERTION AIDING DEVICE**
HILFSVORRICHTUNG FÜR DIE ENDOSKOP-EINFÜHRUNG
DISPOSITIF AUXILIAIRE D'INSERTION D'ENDOSCOPE

(30) Priority: 15.03.2004 JP 2004073581; 05.04.2004 JP 2004111521; 27.07.2004 JP 2004219214
(43) Date of publication of application: 29.11.2006
(73) Proprietor: Olympus Corporation, Tokyo 151-0072 (JP)
(72) Inventor: TANAKA, Shinsuke c/o Olympus Intellectual, Hachioji-shi, Tokyo 192-8512 (JP); TAKIZAWA, Hironobu c/o Olympus Intellectual, Hachioji-shi, Tokyo 192-8512 (JP); AOKI, Isao c/o Olympus Intellectual, Hachioji-shi, Tokyo 192-8512 (JP); KAWANO, Hironao c/o Olympus Intellectual, Hachioji-shi, Tokyo 192-8512 (JP)
(74) Representative: von Hellfeld, Axel
(86) International application number: PCT/JP2005/004293
(87) International publication number: WO 2005/087082

(56) References cited:
- JP-A- 2 141 657
- JP-A- 7 079 909
- JP-A- 10 155 733
- JP-A- 11 128 230
- JP-A- 2002 507 922
- JP-A- 2003 210 392
- JP-A- 2003 260 026

## Description

### Technical Field

The present invention relates to an endoscope insertion aiding device that aids the insertion of an endoscope by using a spiral structure.

### Background Art

Recently, an endoscope is widely used in the medical and industrial fields. The endoscope uses an endoscope insertion aiding device to smoothly insert the endoscope into a winding portion in the body cavity.

For example, as a first conventional art, Japanese Unexamined Patent Application Publication No. 54-78884 discloses a fiber scope comprising a spiral inserting portion, which facilitates the insertion in the large intestine by twisting the inserting portion on the hand side.

Further, as a second conventional art, Japanese Unexamined Utility Model Registration Application Publication No. 51-73884 discloses an endoscope insertion aiding device comprising a large number of cylinders and rings connected via rivets and a spiral member on the outer side, in which a fiber scope is inserted therein to facilitate the insertion to the large intestine.

JP 02141657 A discloses a moving unit of a self-propelling apparatus composed of a main body on a front side and a main body on a rear side, which are connected via a hard tube. An insertion portion of an endoscope is inserted into an inner opening of the moving unit. The elastic body used for forward and backward driving movement is provided so as to cover an outer periphery of the tube. The elastic body comprises an elastic tube and a restricting tubular member for restricting expansion of the elastic body. In an air-tight opening between the tube and the elastic tube an air feeding tube is arranged. On the outer side of the elastic body a compressing spring is arranged in a compressed state. Both ends of the compressing spring are mounted to the main body on the front side and the main body on the rear side. The main body on the front side and the main body on the rear side are provided with a front balloon and a rear balloon, respectively, for engaging an inner wall of the tubular opening. Tightly closed spaces and of the balloons and are connected to communicate with the air feeding tubes, which are connected to the fluid supplying apparatus for supplying pressurized fluid. On the outer peripheral surfaces of the balloons and many anti-slipping projections are provided.

JP 10155733 A discloses and endoscope inserting auxiliary tool according to the preamble of claim 1 having a guide tube, into which an insertion portion of an endoscope is inserted. A balloon is mounted to the outer peripheral surface at a distal end portion of the guide tube. A spiral portion is provided on the outer peripheral surface of the guide tube and partly covers the guide tube.

JP 7079909 A discloses an endoscope insertion guide comprising a guide body and a fluid supply means. The guide body has a closed distal end and comprises a tubular body, in which a hollow member is spirally arranged. The inner and the outer peripheral surfaces of the tubular body are covered with covering layers, respectively.

JP 2003210392 A discloses a catheter tube having a hollow form and comprising a magnetic body on its distal end. By utilizing a magnetic section of an external magnetic device for an observation subject, an endoscope insertion portion is guided in a predetermined direction together with the catheter tube.

The first conventional art as mentioned above is disadvantageous in that it is not available to aid the insertion of a usual endoscope of which the inserting portion becomes rectilinear. The second conventional art as mentioned above is disadvantageous in that the constitution thereof is complex and the costs becomes high.

The second conventional art as mentioned above is disadvantageous also in that it does not well come in contact with body cavities which may be of various diameters because the outer diameter thereof is not variable so that the thrust may become insufficient when rotated.

Further in the second conventional art, when it is to be removed, the uneven configuration of the spiral member may prevent it from being removed smoothly.

The present invention, which has been made under the circumstances, is to provide an endoscope insertion aiding device which ensures a smooth thrusting operation by rotation.

### Disclosure of Invention

### Means for Solving the Problem

According to the present invention, an endoscope insertion aiding device comprises: a flexible tube;
a distal-end member that is arranged to the distal end of the tube and has the outer diameter equal to the outer diameter of the tube or more; and
a spiral structure arranged onto the outer circumferential surface of the tube, and also arranged to the outer circumferential surface of the distal-end member.

By the constitution as mentioned above, the distal-end member that has the outer diameter equal to the outer diameter of the tube or more comes in contact with the inner wall of the lumen, whereupon it is possible to have a large contact area of the distal-end member with the inner wall of the lumen so that the tube may be inserted in the lumen without being captured to haustra. Further, the spiral structure provided to the tube comes in contact with the inner wall of the lumen and rotates, thereby to thrust the tube and ensure the smooth insertion.

### Brief Description of the Drawings

Fig. 1 is a diagram showing the entire structure of an endoscope device according to a first embodiment of the present invention.
Fig. 2 is a perspective view showing the appearance of an endoscope insertion aiding device according to the first embodiment.
Fig. 3 is a diagram showing the structure of the distal end shown in Fig. 2.
Fig. 4 is a sectional view showing the structure of a rotation driving device shown in Fig. 1.
Fig. 5 is a diagram showing a relationship between a rotating direction and an advancing direction.
Fig. 6 is a diagram showing a state of inserting an inserting portion of the endoscope into the endoscope insertion aiding device.
Fig. 7 is a diagram showing a state of bending the inserting portion of the endoscope by a bending mechanism of the endoscope while inserting the inserting portion.
Fig. 8 is a sectional view showing a state of injecting a fluid in the space between the endoscope and the endoscope insertion aiding device.
Fig. 9A is an explanatory diagram of a state of inserting the endoscope into the large intestine by using the endoscope insertion aiding device.
Fig. 9B is a diagram showing a just-after state of insertion into the anus.
Fig. 9C is an explanatory diagram of a state of insertion into the deep part of the winding lumen.
Fig. 10 is a perspective view showing a rotation driving device according to a first modification.
Fig. 11A is a perspective view exploding and showing a rotation driving device and the like according to a second modification.
Fig. 11B is a diagram showing a motor having a hollow rotating shaft.
Fig. 12A is a sectional view showing a rotation driving device according to a third modification.
Fig. 12B is a sectional view of the rotation driving device along the line A-A shown in Fig. 12A.
Fig. 13 is a diagram showing the schematic structure of an endoscope insertion aiding device according to a fourth modification.
Fig. 14A is a diagram showing a state of inserting a distal-end member into the inserting portion.
Fig. 14B is a diagram showing a state of blowing a balloon in the state shown in Fig. 14A.
Fig. 15 is a schematic diagram showing the internal structure according to a fifth modification.
Fig. 16 is a schematic diagram showing the internal structure according to a sixth modification.
Fig. 17 is a diagram showing the entire structure of an endoscope insertion aiding device according to a second embodiment of the present invention.
Fig. 18A is a diagram showing a state of blowing and projecting a tube forming a spiral structure.
Fig. 18B is a diagram showing a state in which the tube forming the spiral structure is not blown.
Fig. 18C is a diagram showing a state of further blowing the tube as compared with the case shown in Fig. 18A.
Fig. 19 is a diagram showing the entire structure of an endoscope insertion aiding device according to the first modification.
Fig. 20 is a diagram showing a state of flattening a projected height of a spiral structure comprising a hollow tube according to the first modification.
Fig. 21 is a perspective view showing the structure of a bending portion according to the second embodiment.
Fig. 22 is a perspective view showing the structure of a bending portion according to the modification.
Fig. 23A is a diagram showing the bending shape on the distal-end side in the case of controlling the bending operation.
Fig. 23B is a diagram showing a state of rotating a bent tube.
Fig. 24A is an explanatory diagram of the operation of a torque limiter.
Fig. 24B is a diagram showing a state of the operation of the torque at a predetermined level or more in Fig. 24A.
Fig. 25 is a diagram showing a spiral structure comprising a close-coiling member with a fine diameter according to the second modification.
Fig. 26A is a diagram showing a tube structure according to the third modification.
Fig. 26B is a diagram showing a state of injecting the air to an external tube in Fig. 26A.
Fig. 27A is a diagram showing a tube structure according to the fourth modification.
Fig. 27B is a diagram showing a state of blowing the tube in Fig. 27A.
Fig. 28A is a diagram showing a tube structure according to the fifth structure.
Fig. 28B is a diagram showing a state of detaching the spiral structure from the tube in Fig. 28A.
Fig. 29A is a diagram showing a rotation regulating mechanism according to the sixth modification.
Fig. 29B is a diagram showing a state of the operation of torque at a predetermined level or more in Fig. 29A.
Fig. 30 is a diagram showing the structure of a rotation regulating mechanism according to the seventh modification.
Fig. 31A is a diagram showing the arrangement of a torque limiter.
Fig. 31B is a diagram showing the case of arranging the torque limiter at the position different from that shown in Fig. 31A.
Fig. 31C is a diagram showing the case of arranging the torque limiter at the position different from those shown in Figs. 31A and 31B.
Fig. 32 is a diagram showing the partial structure of a rotation regulating mechanism according to the eighth modification.
Fig. 33A is an explanatory diagram of the operation of insertion into the body cavity according to the ninth modification.
Fig. 33B is a diagram showing a state of insertion into the deeper side as compared with the case shown in Fig. 33A.
Fig. 33C is a diagram showing a state of insertion into the deeper side as compared with the case shown in Fig. 33B.
Fig. 34A is a diagram showing the distal-end side according to the tenth modification.
Fig. 34B is a diagram showing a state of bending a distal-end member.
Fig. 35 is a perspective view showing the structure of a distal-end side according to the third embodiment of the present invention.
Fig. 36A is a diagram showing the structure of a thrusting holder according to the first modification.
Fig. 36B is a diagram showing the internal structure of the thrusting holder.
Fig. 37 is a perspective view schematically showing the structure of a thrusting holder according to the second modification.
Fig. 38 is a diagram showing the internal structure of the thrusting holder shown in Fig. 37.
Fig. 39 is a perspective view showing the periphery of a thrusting holder attached to an endoscope according to the third modification.
Fig. 40 is a perspective view showing the schematic structure of the thrusting holder shown in Fig. 39.
Fig. 41 is a diagram showing the internal structure of the thrusting holder shown in Fig. 40.
Fig. 42 is a perspective view showing a distal-end side inserted into a channel of a dedicated endoscope according to the fourth modification.
Fig. 43A is a perspective view showing the appearance of the periphery of the distal-end portion of the dedicated endoscope.
Fig. 43B is a front view of Fig. 43A.
Fig. 44 is a diagram showing a state of inserting a treatment tool in a hollow portion according to the fourth modification.
Fig. 45 is a perspective view showing the structure of a distal-end side according to the fourth embodiment of the present invention.
Fig. 46 is a perspective view showing the structure of a distal-end side according to the first modification.
Fig. 47 is a perspective view showing the structure of a distal-end side according to the second modification.
Fig. 48 is a perspective view showing the structure of a distal-end side according to the third modification.
Fig. 49 is a perspective view showing the structure of a distal-end side according to the fourth modification.
Fig. 50 is a perspective view showing the structure of a distal-end side of an endoscope insertion aiding device having a distal-end member with the outer diameter equal to that of a tube.
Fig. 51 is a diagram showing the entire structure of an endoscope insertion aiding system according to the fifth embodiment.
Fig. 52 is a perspective view showing a distal-end side of an inserting portion of an endoscope and a distal-end side of a spiral thrusting probe shown in Fig. 51.
Fig. 53 is a sectional view showing the internal structure of a spiral thrusting portion shown in Fig. 52.
Fig. 54 is an explanatory diagram of a spiral driving portion shown in Fig. 51.
Fig. 55 is an explanatory diagram of the connection between a motor-unit portion and flexible shaft shown in Fig. 54.
Fig. 56 is a first explanatory diagram of the operation of the inserting portion of the endoscope and the spiral thrusting probe.
Fig. 57 is an explanatory diagram of the operation of the spiral thrusting portion of the spiral thrusting probe shown in Fig. 56.
Fig. 58 is a second explanatory diagram of the operation of the inserting portion of the endoscope and the spiral thrusting probe.
Fig. 59 is an explanatory diagram of a spiral thrusting portion according to the first modification.
Fig. 60 is a sectional view showing the internal structure of the spiral thrusting portion shown in Fig. 59.
Fig. 61 is an explanatory diagram of a spiral thrusting portion according to the second modification.
Fig. 62 is a sectional view showing the internal structure of the spiral thrusting portion shown in Fig. 61.
Fig. 63 is an explanatory diagram of a spiral thrusting portion according to the third modification.
Fig. 64 is a sectional view showing a spiral thrusting portion according to the fourth modification.
Fig. 65 is an explanatory diagram of the spiral thrusting portion when a taper balloon shown in Fig. 64 is blown.
Fig. 66 is a front view showing the taper balloon shown in Fig. 65.
Fig. 67 is a sectional view showing a spiral thrusting portion according to the fifth modification.
Fig. 68 is a front view showing a planetary gear shown in Fig. 67.
Fig. 69 is an explanatory diagram in the case of attaching the spiral thrusting portion shown in Fig. 67 to a flexible rotating shaft.
Fig. 70 is a sectional view showing a spiral thrusting portion according to the sixth modification.
Fig. 71 is a sectional view showing a spiral thrusting portion according to the seventh modification.
Fig. 72 is a perspective view showing a distal-end side of a spiral thrusting probe forming an endoscope insertion aiding system and a distal-end side of an inserting portion of an endoscope according to the sixth embodiment of the present invention.
Fig. 73 is an explanatory diagram of a spiral thrusting portion when a balloon on the proximal-end side shown in Fig. 72 is blown.
Fig. 74 is a first explanatory diagram of the operation of the inserting portion of the endoscope and the spiral thrusting probe.
Fig. 75 is a second explanatory diagram of the operation of the inserting portion of the endoscope and the spiral thrusting probe.
Fig. 76 is a third explanatory diagram of the operation of the inserting portion of the endoscope and the spiral thrusting probe.
Fig. 77 is a perspective view showing an endoscope insertion aiding device and a distal-end side of an inserting portion of an endoscope according to the first modification.
Fig. 78 is an explanatory diagram of the endoscope insertion aiding device and the distal-end side of the inserting portion of the endoscope shown in Fig. 77.
Fig. 79 is a perspective view showing an endoscope insertion aiding device and a distal-end side of an inserting portion of an endoscope according to the second modification.
Fig. 80 is a perspective view showing an operating portion of a spiral thrusting probe shown in Fig. 79.
Fig. 81 is a perspective view showing an endoscope insertion aiding device and a distal-end side of an inserting portion of an endoscope according to the third modification.
Fig. 82 is a perspective view showing a distal-end side of an inserting portion of an endoscope forming an endoscope insertion aiding system and a distal-end side of a spiral thrusting probe according to the seventh embodiment of the present invention.
Fig. 83 is an explanatory diagram of the structure of an advance and retreat mechanism unit shown in Fig. 82.
Fig. 84 is an explanatory diagram of an endoscope insertion aiding device and a distal-end side of an inserting portion of an endoscope according to the first modification.
Fig. 85 is a front view showing a spiral thrusting portion shown in Fig. 84.
Fig. 86 is a perspective view showing an endoscope insertion aiding device and a distal-end side of an inserting portion of an endoscope according to the second modification.
Fig. 87 is an explanatory diagram of an attachable/detachable unit and the distal-end side of the inserting portion of the endoscope shown in Fig. 86.
Fig. 88 is a sectional view showing the structure of a thrusting device for endoscope attached to an endoscope according to the eighth embodiment of the present invention.
Fig. 89 is a side view of Fig. 88.
Fig. 90 is a front view of Fig. 88.
Fig. 91 is a principle diagram of a rotation driving system.
Fig. 92 is a diagram showing a using example in the body cavity.
Fig. 93 is a transverse sectional view showing a magnetic field applying member arranged in a channel according to the first modification.
Fig. 94 is a longitudinal sectional view showing the magnetic field applying member arranged in the channel according to the first modification.
Fig. 95 is a transverse sectional view showing a magnetic field applying member arranged in a channel according to the second modification.
Fig. 96 is a sectional view showing the structure of attachment to an endoscope according to the third modification.
Fig. 97 is a sectional view showing the structure of attachment to an endoscope according to the fourth modification.
Fig. 98 is a sectional view showing the structure of attachment to an endoscope according to the fifth modification.
Fig. 99 is a sectional view showing the structure of attachment to an endoscope according to the sixth modification.
Fig. 100 is a sectional view showing the structure of attachment to an endoscope according to the seventh modification.
Fig. 101 is an explanatory diagram of maintaining a rotating member in freely rotatable state by the magnetic suspension caused by the magnets at a distal-end side and the rotating member side.
Fig. 102 is a sectional view showing the structure of attachment to an endoscope according to the eighth modification.
Fig. 103 is an explanatory diagram of the operation according to the eighth modification.
Fig. 104 is a diagram showing a part according to the ninth modification.
Fig. 105 is a front view showing the structure of attachment to an endoscope according to the tenth modification.
Fig. 106 is a perspective view showing an attaching state to a distal-end portion of the endoscope.
Fig. 107 is a sectional view showing the structure of attachment to an endoscope according to the eleventh modification.
Fig. 108 is a sectional view partly showing a state of attachment to an endoscope according to the twelfth modification.
Fig. 109 is a perspective view partly showing a state of attachment to an endoscope according to the thirteenth modification.
Fig. 110 is a sectional view showing a state of attachment to an endoscope according to the fourteenth modification.
Fig. 111 is a sectional view showing a state of attachment to an endoscope according to the fifteenth modification.
Fig. 112 is a sectional view showing the structure of attachment to an endoscope according to the sixteenth modification.
Fig. 113 is a sectional view showing the structure according to the ninth embodiment of the present invention.
Fig. 114 is a diagram showing the operation principle of rotational drive.
Fig. 115 is a sectional view showing the structure according to the tenth embodiment of the present invention.
Fig. 116 is a front view of Fig. 115.
Fig. 117 is a perspective view showing a state of attachment to an endoscope.
Fig. 118 is a diagram showing the operation principle of rotation.
Fig. 119 is a sectional view showing a state of attachment to an endoscope according to the first modification.
Fig. 120 is a perspective view showing a state of being attaching to the endoscope according to the first modification.
Fig. 121 is a sectional view showing a state of attachment to an endoscope according to the second modification.
Fig. 122 is a perspective view showing a state of being attaching to the endoscope according to the second modification.
Fig. 123 is an explanatory diagram of the thrusting operation by rotating a wheel.

### Best Mode for Carrying Out the Invention

Hereinbelow, embodiments of the present invention will be described with reference to the drawings.

### (First embodiment)

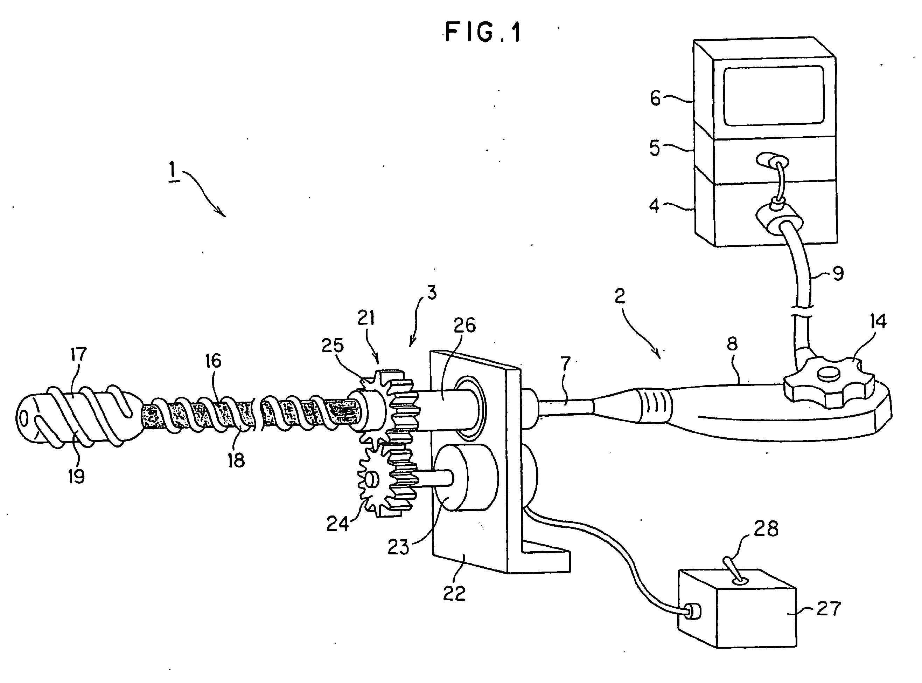

A first embodiment of the present invention will be described with reference to Figs. 1 to 16.

Referring to Fig. 1, an endoscope device 1 according to the first embodiment comprises: an endoscope 2 for endoscope examination; an endoscope insertion aiding device 3 for inserting the endoscope 2 therein and for aiding the insertion of the endoscope 2; a light source device 4 for supplying illumination beam to the endoscope 2; a camera control unit (abbreviated to a CCU) 5 for signal processing of an image pick-up element included in the endoscope 2; and a monitor 6 for receiving a video signal outputted by the CCU 5 and displaying an endoscope image picked-up by the image pick-up element.

The endoscope 2 comprises: an inserting portion 7 which is inserted in the body cavity with flexibility; an operating portion 8 arranged to the proximal end of the inserting portion 7; and a cable portion 9 extended from the side portion of the operating portion 8. The terminal end of the cable portion 9 is connected to the light source device 4 and the CCU 5.

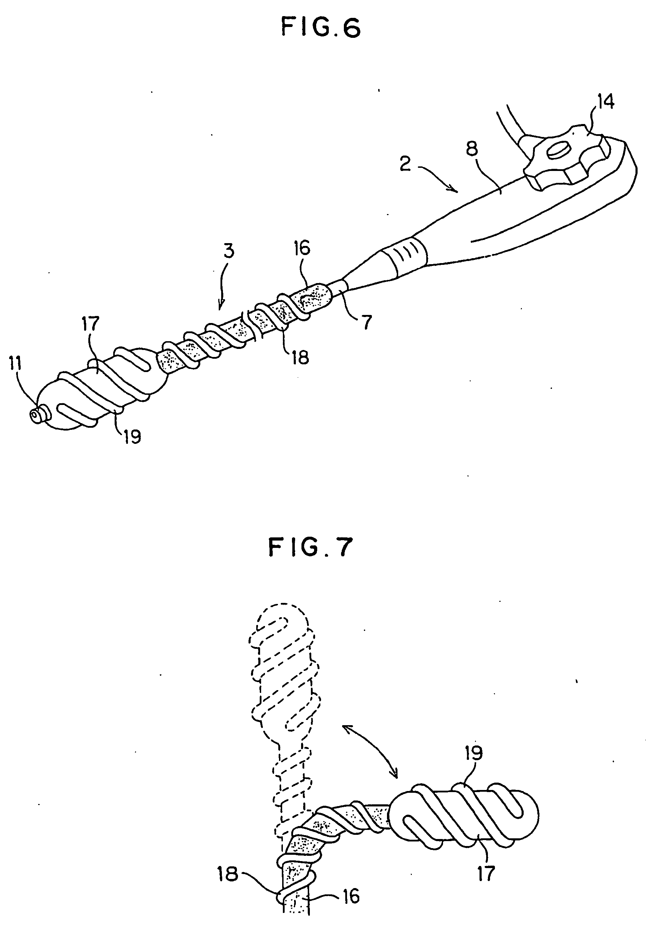

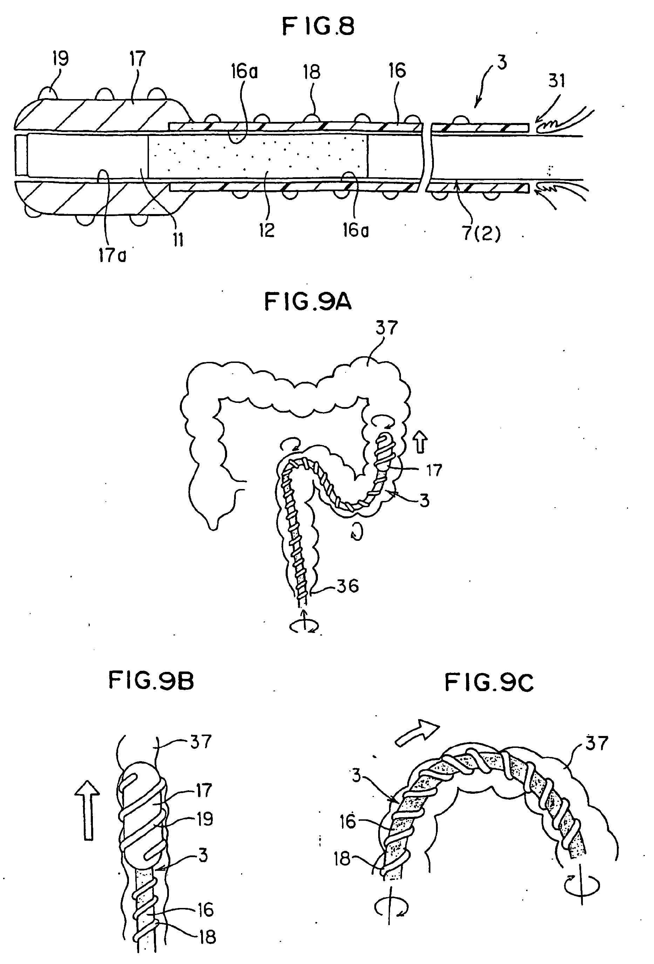

The inserting portion 7 comprises a rigid distal-end portion 11 (refer to Figs. 6 and 8) having an illuminating window and an observing window at the distal end thereof, and a bending portion 12 (refer to Fig. 8) which is arranged to the proximal end of the distal-end portion 11 and is freely bent. The bending portion 12 is bent in the desired direction by operating a bending knob 14 arranged to the operating portion 8.

The light source device 4 supplies illumination beam to a light guide (not shown) of the endoscope 2. The supplied illumination beam is outputted from the illuminating window to illuminate the body cavity. An image of the light reflected or scattered in the illuminated body cavity is formed, as an optical image, onto a solid-state image pick-up element arranged at the image forming position via an objective lens attached to the observing window, and is photoelectrically converted onto the image pick-up surface. The signal photoelectrically-converted by the solid-state image pick-up element is subjected to signal processing by the CCU 5, is converted into a standard video signal, and is sent to the monitor 6. The optical image formed onto the solid-state image pick-up element is displayed, as the endoscope image, on a display surface of the monitor 6.

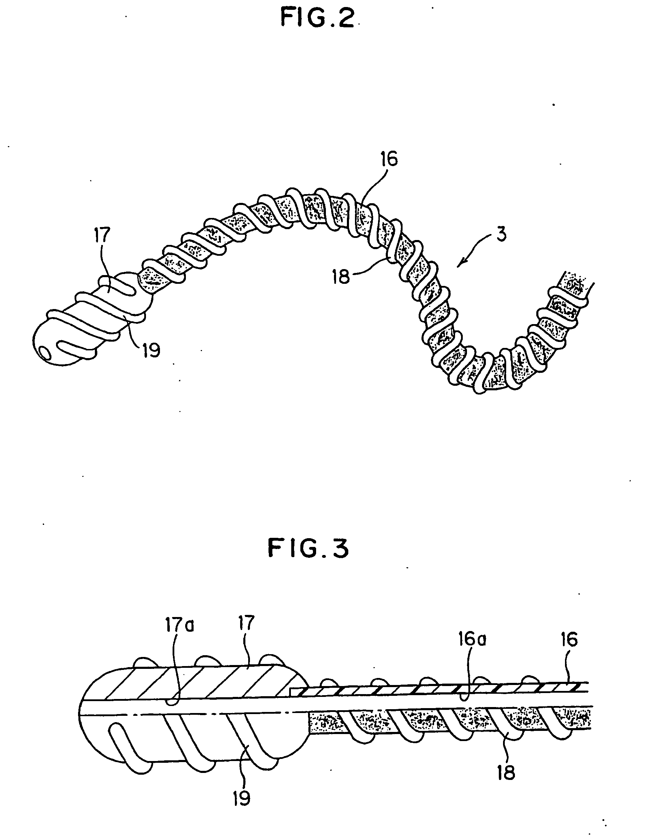

Referring to Figs. 1 and 2, the endoscope insertion aiding device 3 according to the first embodiment has a flexible (soft) tube 16. The tube 16 has, at the distal end thereof, a distal-end member 17 with proper rigidity containing a soft member such as resin and with the diameter thicker than that of the tube 16.

The tube 16 has, on the outer surface thereof, a spiral structure 18 formed by spirally attaching hollow or solid resin like a string with a fine diameter and then by spirally projecting the attached portion from the outer surface. Similarly, a spiral structure 19 is arranged onto the cylindrical outer surface of the distal-end member 17. The spiral structures 18 and 19 may be connected.

According to the first embodiment, the spiral structure 18 is arranged onto the outer circumferential surface of the tube 16, the distal-end member 17 with the thicker diameter is arranged at the distal end of the tube 16, the spiral structure 19 is arranged onto the outer circumferential surface of the distal-end member 17, and the tube 16 is rotated, thereby enabling the thrusting operation with large thrust caused by the spiral structure 19 arranged on the outer circumferential surface of the distal-end member 17.

Referring to Fig. 3, a hollow portion 16a in the tube 16 is communicated with a through-hole 17a arranged along the central axis of the distal-end member 17. The inserting portion 7 of the endoscope 2 is inserted from the proximal end of the hollow portion 16a, the distal-end portion 11 of the inserting portion 7 is arranged in the through-hole 17a, and the illuminating window and the observing window of the endoscope 2 are exposed at the opening at the distal end of the through-hole 17a, thereby observing the body cavity.

Referring to Fig. 1 again, the tube 16 has, at the proximal end thereof, a rotation driving device 21 that rotates the tube 16.

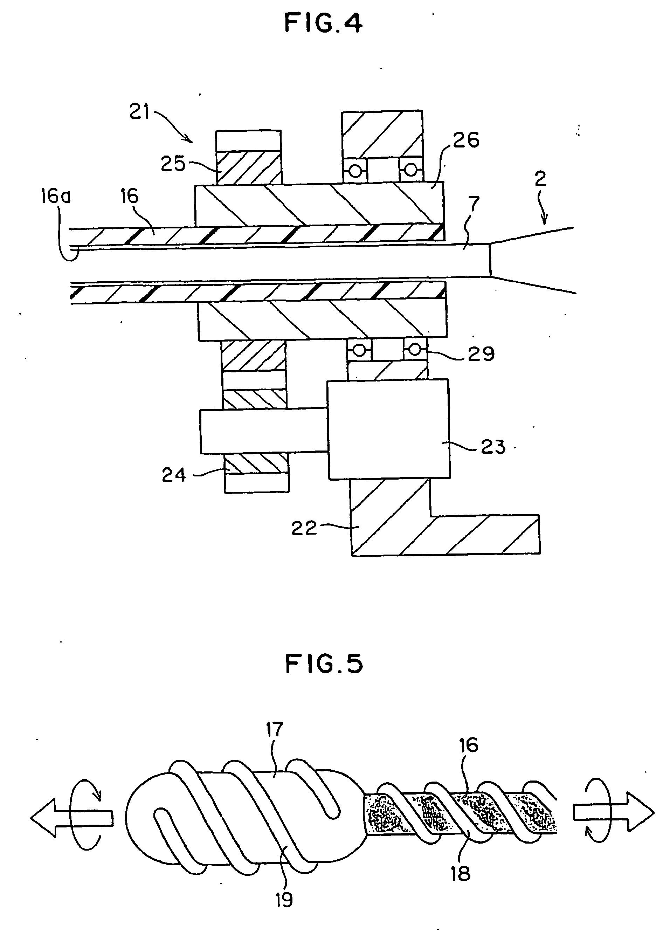

Referring to Figs. 1 and 4, the rotation driving device 21 comprises: a motor 23 that is attached to a holder 22; a gear 24 attached to a rotating shaft of the motor 23; and a gear 25 attached to the distal end of a cylinder 26 that holds the proximal end of the tube 16. The gear 25 is engaged with the gear 24 attached to the rotating shaft of the motor 23. The gear 25 is rotated by rotating the motor 23 and thus the cylinder 26 and the tube 16 are rotated.

The motor 23 is connected to a motor driving device 27 via a cable. The motor driving device 27 includes a driving battery and a control circuit that controls the number of rotations and the rotating direction of the motor 23. Further, the motor driving device 27 has, on the top thereof, an operating knob 28.

A user inclines the operating knob 28 forward and thus the tube 16 is moved forward. That is, the motor 23 is rotated in the thrusting direction. The operating knob 28 is inclined backward and thus the tube 16 is moved backward. That is, the motor 23 is rotated in the returning direction.

Referring to Fig. 4, the proximal end of the tube 16 is attached to the inner circumferential surface of the cylinder 26. The cylinder 26 is freely rotatably held to the holder 22 via a roller bearing 29 that freely rotatably supports the cylinder 26.

Fig. 5 shows a relationship between the rotating direction and the advancing direction. Referring to Fig. 5, the spiral structures 18 and 19 are right-screwed, and the tube 16 is rotated in the clockwise direction, thereby advancing the tube 16. The tube 16 is rotated in the counterclockwise direction, thereby moving the tube 16 backward.

As described above, as shown in Fig. 6, the inserting portion 7 of the endoscope 2 is inserted into the hollow portion 16a of the tube 16. That is, the distal-end side of the inserting portion 7 of the endoscope 2 with the fine diameter is inserted from the terminal end of the tube 16, and the inserting portion 7 is inserted up to the distal-end member 17. Fig. 6 shows a state of slightly projecting the distal-end portion 11 of the inserting portion 7 from the through-hole 17a of the distal-end member 17. The distal-end surface of the endoscope 2 is slightly projected to the opening of the distal end of the through-hole 17a, thereby enabling an observing function.

Since the endoscope 2 has the bending portion 12, the tube 16 is bent by using a bending mechanism of the endoscope 2 shown in Fig. 7 when the inserting portion 7 of the endoscope 2 is inserted in the tube 16 as shown in Fig. 1 or 6.

That is, according to the first embodiment, the observing function and the bending function of the endoscope 2 are used in the inserting state of the endoscope 2. As a consequence, the endoscope insertion aiding device 3 according to the first embodiment has a mechanism for smoothly aiding the insertion of the endoscope 2 with the simple structure.

Referring to Fig. 8, a fluid 31 such as water or air serving as a lubrication agent may be injected into the space between the tube 16 and the distal-end member 17 from the end of the tube 16 so as to improve a function (smoothly rotating function) for smoothly rotating the tube 16 and the distal-end member 17 on the outer circumferential side of the endoscope 2 without rotating the inserting portion 7 of the endoscope 2.

As described above, the fluid 31 is injected in the space between them and thus the inserting portion 7 of the endoscope 2 can smoothly be inserted without rotating the inserting portion 7 of the endoscope 2 upon rotatably driving the tube 16 so as to thrust the inserting portion 7.

A description of the operation for inserting the endoscope 2 into the body cavity by using the endoscope insertion aiding device 3 with the above-described structure according to the first embodiment is given.

Fig. 9A shows a state of inserting the inserting portion 7 of the endoscope 2 into the deep portion of a large intestine 37 from an anus 36 by using the endoscope insertion aiding device 3 according to the first embodiment while the inserting portion 7 of the endoscope 2 is inserted into a hollow portion of the endoscope insertion aiding device 3.

In the case of inserting the inserting portion 7 of the endoscope 2 into the deeper portion of the large intestine 37, the inserting portion 7 is inserted into the anus 36 from the distal-end member 17 of the endoscope insertion aiding device 3 while the inserting portion 7 is inserted in the endoscope insertion aiding device 3 according to the first embodiment.

Fig. 9B shows an immediate post insertion state in the anus 36. Referring to Fig. 9B, the straight large intestine 37 does not need the bending operation, and the distal-end member 17 can advance to the deep portion of the large intestine 37 by rotating the proximal end of the tube 16 with the rotation driving device 21 on the hand side. That is, according to the first embodiment, the spiral structure 19 is arranged on the outer circumferential surface (outer surface) of the distal-end member 17 with the outer diameter thicker than that of the tube 16, at the distal end of the tube 16. Therefore, the distal-end member 17 is rotated with the operation of friction force caused by the contact state with the inner wall surface of the large intestine 37 and thus the spiral structure 19 sequentially and spirally comes into contact with the inner wall surface of the large intestine 37.

In accordance with the spiral moving locus, the distal-end member 17 effectively advances to the deep portion.

At the bent portion such as the sigmoid colon, referring to Fig. 9C, the rotation of the rotation driving device 21 enables the distal-end member 17 to pass through the bent portion so that the distal-end member 17 is bent in the direction for bending the bending portion 12 of the endoscope 2 under the observation using the endoscope 2.

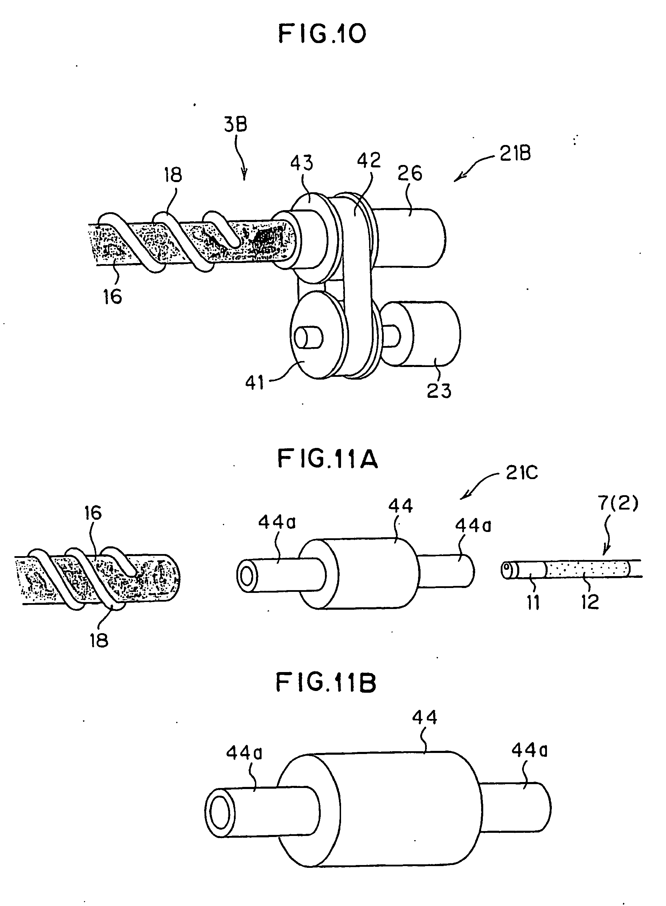

Referring to Fig. 9A, the distal-end member 17 is thrust to the deep portion of the large intestine 37. Further, the insertion into the deeper portion is smooth. Fig. 10 shows the structure of a rotation driving device 21B in an endoscope insertion aiding device 3B according to the first modification. In the rotation driving device 21B, a pulley 41 is attached to a rotating shaft of the motor 23 and a pulley 43 attached to the cylinder 26 for holding the proximal end of the tube 16 via a belt 42 is rotated.

Referring to Fig. 10, for the purpose of a brief description, the holder 22 for holding the cylinder 26 and the motor 23 shown in Figs. 1 and 4 is omitted. The operations and advantages according to a first modification are the same as those of using the gears 24 and 25 shown in Figs. 1 and 4.

Fig. 11A explodes and shows a rotation driving device 21C according to a second modification. The rotation driving device 21C uses a motor 44 having a hollow rotating shaft 44a shown in Fig. 11B. The motor 44 has the hollow rotating shaft 44a and thus the rotatable driving force of the motor 44 is directly transmitted to the tube 16.

That is, the proximal end of the tube 16 is attached to the tip end of the hollow rotating shaft 44a of the motor 44, and the inserting portion 7 of the endoscope 2 is inserted into the hollow portion of the rotating shaft 44a from the proximal end.

The use of the rotation driving device 21C according to the second modification reduces the transmitting loss with the simple structure and low costs.

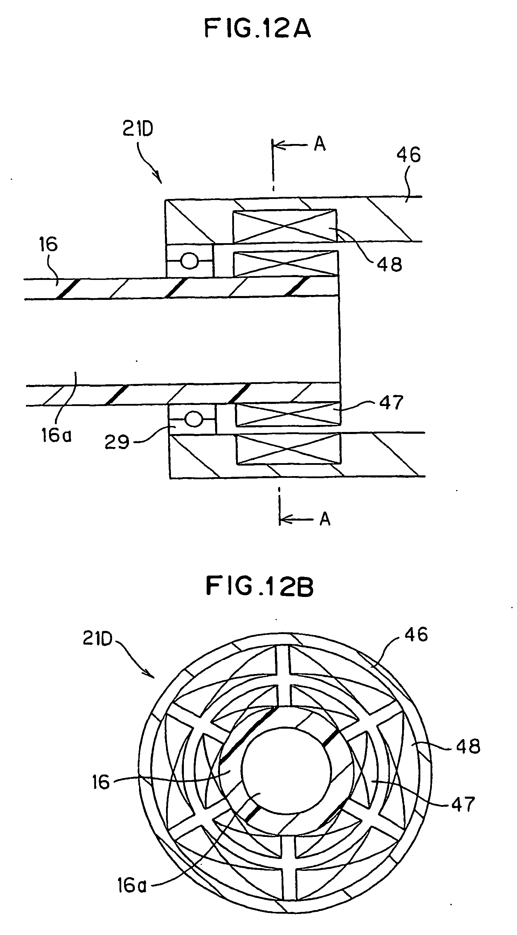

Fig. 12A is a longitudinal sectional view of a rotation driving device 21D according to a third modification. Fig. 12B is a sectional view of an A-A line shown in Fig. 12A.

The periphery of the proximal end of the tube 16 is freely rotatably held to a holding cylindrical member 46 via the roller bearing 29. A coil (or electromagnet) 47 is attached to the outer circumferential surface of the proximal end of the tube 16. A coil (or electromagnet) 48 is attached to the inner circumferential surface of the holding cylindrical member 46 facing the outer circumference of the coil 47.

Referring to Fig. 12B, both the coils 47 and 48 are divided in the circumferential directions. Further, it is set that the AC current with the deviated phases is applied between the coils 47 and 48 which a power device (not shown) faces. Thus, for the coil 48 fixed to the inner circumferential surface of the holding cylindrical member 46, the rotating magnetic field is relatively applied to the coil 47, thereby rotating the coil 47 and the tube 16.

The third modification has approximately the same advantages as those according to the second modification with reference to Fig. 11A. According to the third modification, one of the coils 47 and 48 may be replaced with a magnet. For example, the coil 47 that is rotated is replaced with the magnet, the structure including a contact for supplying current to the coil 47 is not necessary.

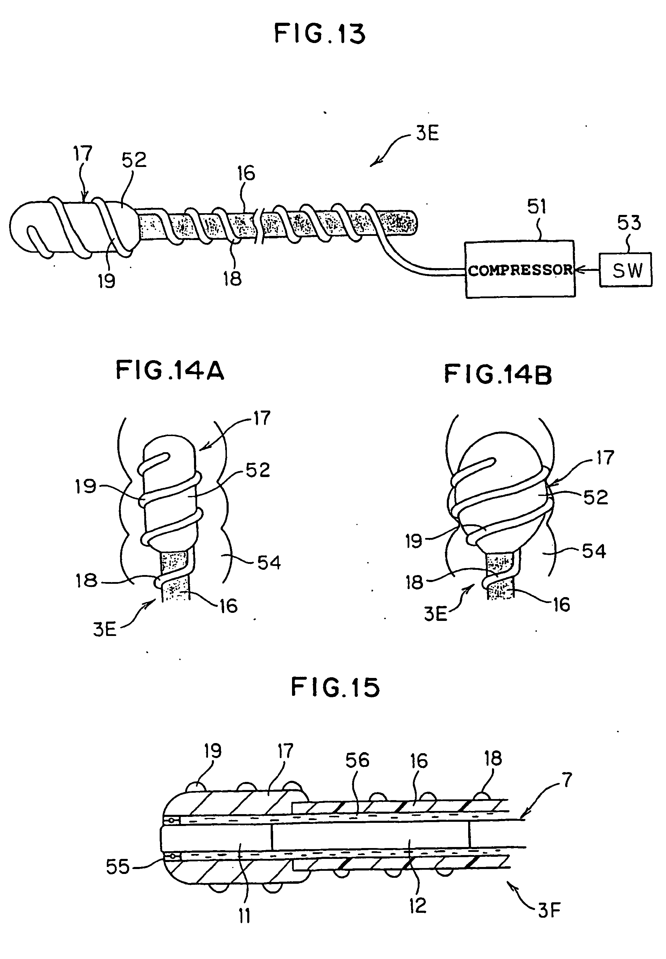

Fig. 13 schematically shows an endoscope insertion aiding device 3E according to a fourth modification. The endoscope insertion aiding device 3E has a compressor 51, serving as a fluid feed and discharge device, which feeds and discharges compressed air (as fluid). According to the fourth modification, the spiral structure 18 arranged to the tube 16 comprises a hollow tube, and the proximal end of the hollow tube is connected to the compressor 51.

The distal end of the hollow tube forming the spiral structure 18 is connected to a balloon 52 arranged on the outer circumferential surface of the distal-end member 17. In this case, the spiral structure 19 contains an elastic member such as rubber, which is arranged on the outer circumferential surface of the balloon 52 for covering the outer circumferential surface of the distal-end member 17.

The compressed air is fed into the balloon 52 via the hollow tube from the compressor 51, thereby blowing the balloon 52.

The user switches a switch 53 from OFF to ON, thereby feeding the compressed air to the balloon 52 from the compressor 51.

Figs. 14A and 14B are explanatory diagrams of the operation of the endoscope insertion aiding device 3E.

Referring to Fig. 14A, in the case of inserting the endoscope insertion aiding device 3E into a body cavity 54, if the inner diameter of the body cavity 54 is larger than the outer diameter of the distal-end member 17, the thrust is not sufficiently obtained by rotating the distal-end member 17.

In this case, the user switches-on the switch 53, thereby operating the compressor 51. Thus, the compressed air is fed to the balloon 52 and, referring to Fig. 14B, the balloon 52 is blown.

The spiral structure 19 on the outer circumferential surface of the balloon 52 comes into contact with the inner wall of the body cavity 54. The endoscope insertion aiding device 3E is rotated in this state and thus the state of generating the higher thrust is set and the thrusting operation in the body cavity 54 is smooth.

The hollow tube used for the spiral structure 18 may be arranged up to the distal end of the distal-end member 17, thereby supplying the fluid such as the air or water to the distal end of the distal-end member 17 from the proximal end of the hollow tube. With the above-described structure, the observing window at the distal end of the endoscope 2 inserted in the endoscope insertion aiding device 3 is cleaned by the fed water, or the air is fed by expanding the body cavity so as to ensure the field of view.

Fig. 15 schematically shows the inner structure of an endoscope insertion aiding device 3F according to a fifth modification. According to the fifth modification, in order to improve the lubricating property between the tube 16 and the inserting portion 7 of the endoscope 2, a circular roller bearing 55 such as a bearing is arranged for rotatable sealing operation between the outer circumferential surface of the distal-end portion 11 of the inserting portion 7 and the inner circumferential surface of the distal-end member 17. A lubrication agent 56 such as oil is filled in the sealed portion.

Thus, the tube 16 on the outer circumferential surface and the distal-end member 17 are rotated without the rotation of the endoscope 2.

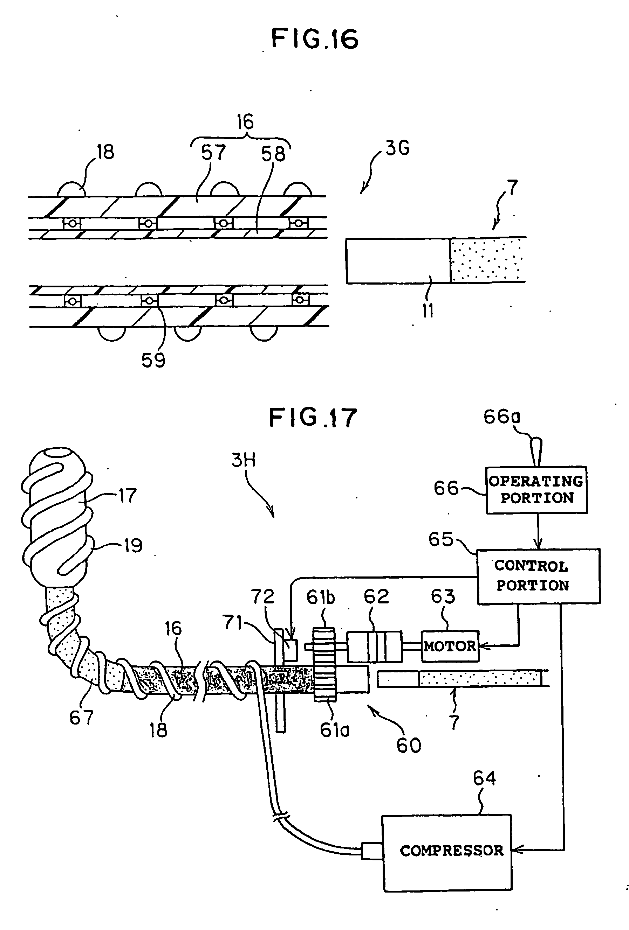

Fig. 16 schematically shows the inner modification of an endoscope insertion aiding device 3G according to a sixth modification. According to the sixth modification, in order to improve the lubricating property between the tube 16 and the inserting portion 7 of the endoscope 2, the tube 16 comprises double sheaths 57 and 58.

The inserting portion 7 to be inserted of the endoscope 2 just fits to the inner sheath 58, and a roller bearing 59 is arranged between the sheaths 57 and 58 at the proper interval.

With the above-described structure, only the outer sheath 57 is easily rotated.

### (Second embodiment)

Next, a second embodiment of the present invention will be described.

Fig. 17 schematically shows an endoscope insertion aiding device 3H according to the second embodiment of the present invention. The endoscope insertion aiding device 3H has the rotation driving device 60 on the proximal-end side of the tube 16.

The rotation driving device 60 comprises: a gear 61a attached to the proximal end of the tube 16; and a gear 61b which is engaged with the gear 61a and is connected to a motor 63 via a torque limiter 62 serving as rotation regulating means.

The spiral structure 18 arranged to the outer circumferential surface of the tube 16 constitutes a hollow tube. The distal end of the hollow tube is closed and the proximal end thereof is connected to a compressor 64.

The motor 63 and the compressor 64 are connected to a control portion 65. The control portion 65 is connected to an operating portion 66. The operation of the operating portion 66 controls the driving and stop of rotation and the rotating speed of the motor 63, and further controls the on/off operation of the operation for feeding the compressed air from the compressor 64.

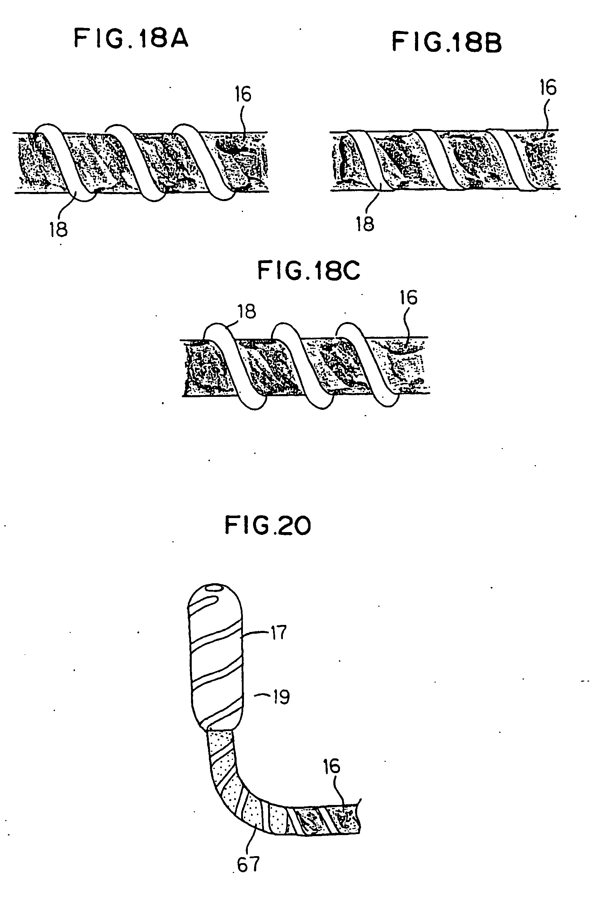

The operation of the operating portion 66 sets the compressor 64 to set a state in which the compressed air is fed. Thus, referring to Figs. 17 or 18A, the spiral structure 18 comprising the flexible hollow tube is projected from the outer diameter of the tube 16.

On the other hand, the operation of the operating portion 66 sets the compressor 64 to set a state in which the compressed air is not fed. Referring to Fig. 18B, the hollow tube forming the spiral structure 18 is not blown and the non-blowing portion has the outer diameter as that of the tube 16.

By adjusting the amount of fed compressed air, it is possible to adjust the height projected from the surface of the tube 16 of the hollow tube forming the spiral structure 18.

For example, by feeding the larger amount of compressed air as compared with that in the state shown in Fig. 18A, referring to Fig. 18C, the height projected from the outer surface of the tube 16 of the spiral structure 18 is higher.

According to the second embodiment, by controlling the feed and the feed stop of compressed air into the hollow tube forming the spiral structure 18, it is possible to select the forming state of the spiral structure 18 is set and the non-forming state thereof. Further, the height of the spiral structure 18 projected from the surface of the tube 16 is adjusted.

Upon inserting the tube 16 into the body cavity, referring to Fig. 18A or 18C, the height for projecting the spiral structure 18 from the outer surface of the tube 16 is set. Further, upon pulling-out the tube 16, referring to Fig. 18B, the surface of the tube 16 is flat for smooth pull-out operation for a short time.

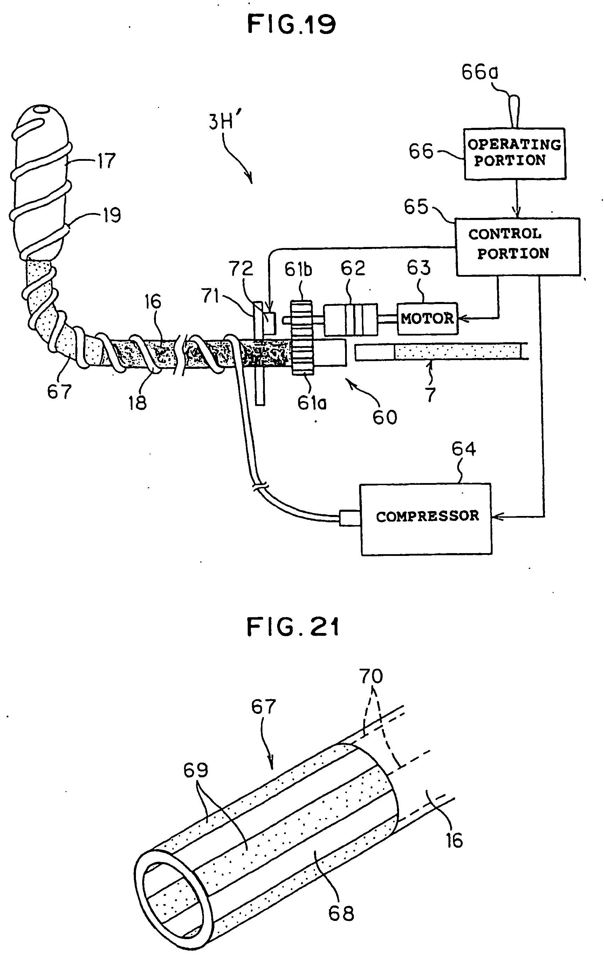

Referring to Fig. 19, in an endoscope insertion aiding device 3H' according to a first modification, a hollow portion is communicated by connecting the distal end of the hollow tube forming the spiral structure 18 arranged to the outer circumferential surface of the tube 16 to the hollow tube forming the spiral structure 19 arranged to the outer circumferential surface of the distal-end member 17.

In this case, since the distal end of the hollow tube forming the spiral structure 19 is closed, the projected spiral structure 18 is formed onto the outer circumferential surface by feeding the compressed air by the compressor 64 as shown in Fig. 19. Further, the projected spiral structure 19 is formed onto the outer circumferential surface of the distal-end member 17.

By discharging the compressed air, referring to Fig. 20, the outer circumferential surface of the distal-end member 17 becomes flat and the outer circumferential surface of the tube 16 also becomes flat. The height of projected portion from the outer circumferences of the spiral structures 18 and 19 is controlled by changing the amount of fed compressed air.

According to the first modification, in the communication of the spiral structures 18 and 19 comprising the hollow tubes on the outer circumferential surface of the tube 16 and the outer circumferential surface of the distal-end member 17, the height of the projected portion from the outer circumferential surface is controlled, thereby smoothly executing the insertion and the pull-out operation.

According to the second embodiment (including the first modification), a bending portion (bending means) 67 is formed at the portion near the distal end of the tube 16, namely, at the portion adjacent to the proximal end of the distal-end member 17. The bending portion 67 contains, for example, an electro active polymer artificial muscle (abbreviated to an EPAM) which is compressed/decompressed by applying a voltage.

Referring to Fig. 21, a tube EPAM 68 with the same dimension is connected to the periphery of the distal end of the tube 16 for integration. Both surfaces of band portions corresponding to the up, down, right, and left portions of the tube EPAM 68 have electrodes 69 respectively.

The electrode 69 is connected to one end of a signal line 70 passing through the inside of the tube 16. Referring to Fig. 17, another end of the signal line 70 is connected to a coaxial contact of a hollow disc contact member 71 on the rotor attached to the outer circumferential surface of the proximal end of the tube 16, and is further connected to the control portion 65 via a contact member 72 on the side of a stator in contact with the coaxial contact.

By inclining a joystick 66a, serving as bending-direction instructing operating means, arranged to the operating portion 66, the control portion 65 applies a driving voltage to the electrode 69 of the EPAM 68 in accordance with the inclining operation and the bending portion 67 is bent in the inclining direction (of the joystick 66a).

When the joystick 66a is inclined in the up direction, the largest driving voltage is applied to the corresponding electrode 69 in the down direction, and the EPAM 68 corresponding to the portion is inclined at the highest level. Further, the proper driving voltage is applied to the right and left electrodes 69 so as to expand the EPAM 68, thereby bending the bending portion 67 in the up direction in which the EPAM 68 is not expanded.

The EPAM 68 has the characteristic serving as the amount of strain in proportional to a value obtained by raising the strength of electric field of the applied voltage to the second power.

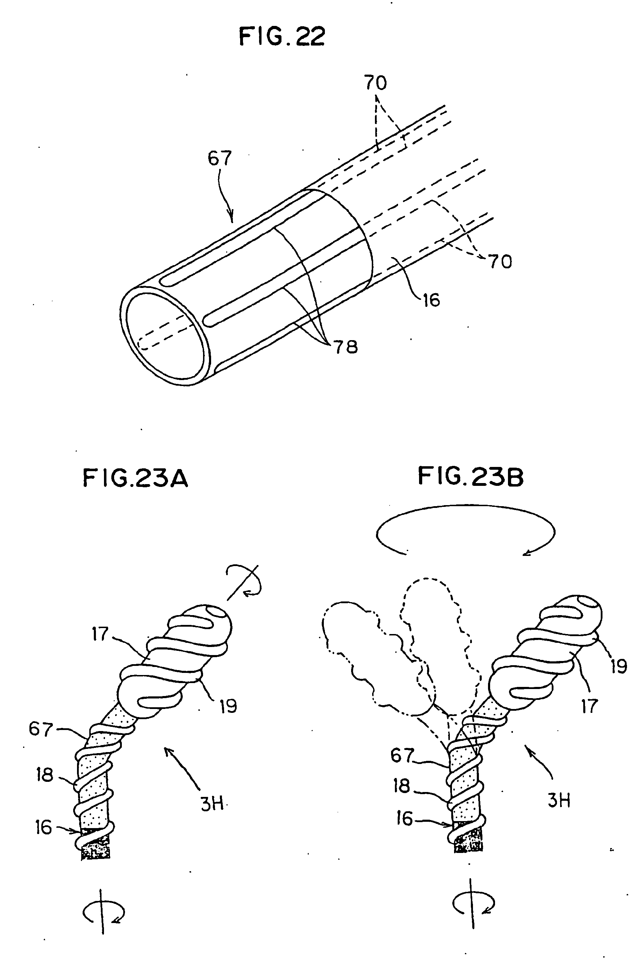

Means other than the EPAM 68 can be used as bending means for bending the bending portion 67. In place of the EPAM 68, referring to Fig. 22, an SMA (shape memory alloy, hereinafter, abbreviated to an SMA) 78 that contracts by the energization may be used.

The SMA wire 78 is arranged at the portions corresponding to the up, down, right, and left portions of the bending portion 67 so that the parallel line is folded on the distal-end side. Further, the SMA wire 78 is connected to the signal line 70 near the proximal end of the bending portion 67.

The proximal-end side of the signal line 70 has the same structure as that of the EPAM 68. The bending portion 67 is bent by energizing the SMA wire 78 in the bending direction.

In addition, a wire connected to the bending portion 67 may comprise means that is mechanically pulled. As described above, some means and methods for bending the bending portion 67 may be selected and used.

The endoscope insertion aiding device 3H according to the second embodiment has the bending mechanism of the tube 16. Therefore, when the inserting portion 7 of the endoscope 2 is not inserted, the distal-end side of the tube 16 can be bent. That is, when the inserting portion 7 of the endoscope 2 is inserted, the tube 16 is bent by using the bending function of the endoscope 2 as shown in Fig. 7 according to the first embodiment. However, according to the second embodiment, the distal-end side of the tube 16 can be bent without inserting the inserting portion 7.

According to the second embodiment, referring to Fig. 23A, the distal-end side of the tube 16 can be bent in the desired direction (without inserting the endoscope). If the tube 16 is rotated while being bent, the distal-end side is oscillated as shown in Fig. 23B. Therefore, when the tube 16 is rotated, referring to Fig. 23A, the bending portion 67 may be controlled so that the bending shape of the tube 16 maintains only in one direction.

According to the second embodiment, when the tube 16 is rotated by rotating the motor 63, the spiral structures 18 and 19 smoothly thrust the tube 16 side. However, the torque at a predetermined level or more is applied to the spiral structures 18 and 19, the torque limiter 62 as serving as the rotation regulating means prevents the rotation of the tube 16 side.

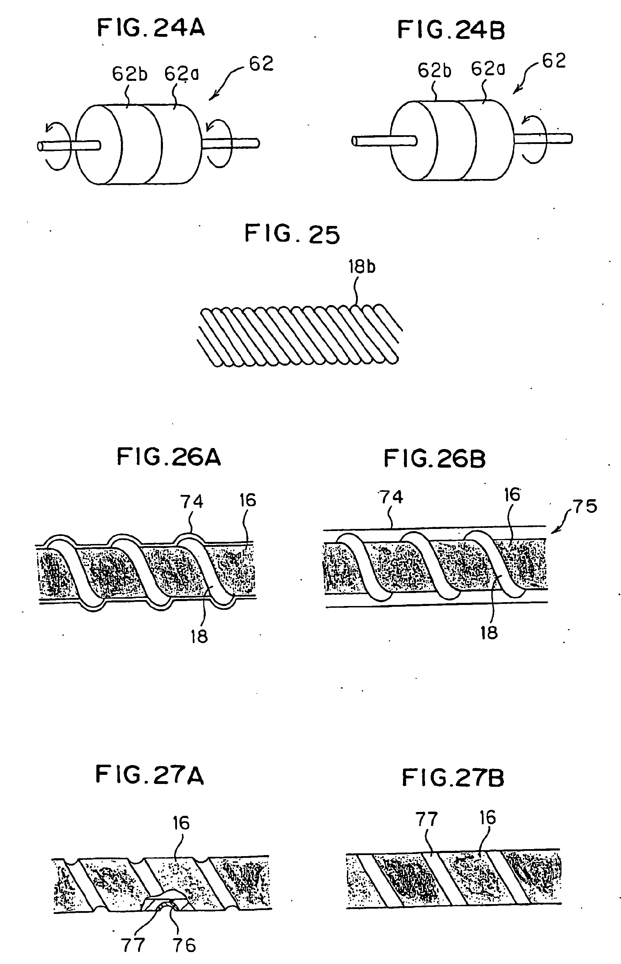

The torque limiter 62 has a slip structure using a clutch. Referring to Fig. 24A, friction surfaces of two discs 62a and 62b for transmitting the rotation having the friction surfaces face each other, and come into contact with each other in the state of applying a proper pressure.

In the operation of torque having predetermined force or more to one of the discs 62a and 62b, referring to Fig. 24B, the two discs 62a and 62b do not transmit the rotating force. According to the second embodiment, the disc 62a connected to the motor 63 is rotated and, however, the other disc 62b is not rotated.

The torque limiter 62 prevents the application of the force at predetermined value or more to the spiral structures 18 and 19 from the inner wall of the body cavity by the rotation of the spiral structures 18 and 19.

According to the second embodiment, similarly to the first embodiment, the spiral structures 18 and 19 are arranged onto the outer circumferential surface between the tube 16 and the distal-end member 17. The same operations and advantages as those according to the first embodiment are obtained by arranging the rotation driving mechanism for rotating the tube 16.

According to the second embodiment, (including the first modification), the tube 16 and the distal-end member 17 smoothly inserted or pulled-out by changing the heights of (projected from the surfaces of) the spiral structures 18 and 19.

The torque limiter 62 serving as the rotation regulating means prevents the application of the force at a predetermined value or more to the spiral structures 18 and 19 from the inner wall of the body cavity by the rotation of the spiral structures 18 and 19.

According to the second embodiment, the bending portion 67 enables the distal end of the inserting portion 7 of the endoscope 2 to be inserted into the body cavity by using the distal end of the inserting portion 7 of the endoscope 2 as a guide wire without the insertion up to the distal-end member 17.

Fig. 25 shows a spiral structure 18b according to a second modification. According to the second modification, the height of spiral portion is reduced because the tube 16 is smoothly pulled-out. Referring to Fig. 25, the spiral structure 18b is arranged like close coiling with the fine diameter (the tube 16 (not shown) is arranged in the spiral structure 18b). The spiral structure 18b has a small spiral structure and, however, a large number of spiral structures 18b are arranged per length as a unit. Therefore, the rotation maintains predetermined thrust.

In the pull-out operation, the spiral structure 18b has the spiral structure with minute concaved and convexed portions, thereby smoothly pulling-out the tube 16.

Figs. 26A and 26B show examples of the tube structure according to a third modification. According to the third modification, for the same purpose as that of Fig. 25, the surface of the tube 16 is covered with a thin external tube 74. The proximal-end side of the external tube 74 is connected to the compressor 64, thereby feeding air 75 into the external tube 74 and discharging the fed air.

In the insertion of the tube 16, the air is discharged and, referring to Fig. 26A, the external tube 74 is firmly attached to the outer surfaces of the spiral structure 18 and the tube 16, thereby forming the spiral structure.

In the pull-out operation, the air 75 is injected into the external tube 74 for blowing. Thus, referring to Fig. 26B, the flat surface structure is formed. In this state, the tube 16 is smoothly pulled-out for a short time.

Figs. 27A and 27B show examples of the tube structure according to a fourth modification. According to the fourth modification, similarly to Fig. 25, the spiral structure comprises a spiral groove 76 arranged onto the outer surface of the tube 16 so as to improve the mobility of the tube 16 as shown in Fig. 27A.

A soft and thin tube 77 is attached to the groove 76, thereby feed and discharging the air from the proximal end of the tube 77. In the insertion, the tube 16 is set to a state shown in Fig. 27A.

In the pull-out operation, the air is fed to the tube 77 arranged along the groove 76, thereby blowing-up the tube 77. Thus, the flat surface is formed as shown in Fig. 27B. In this state, the tube 16 is smoothly pulled-out.

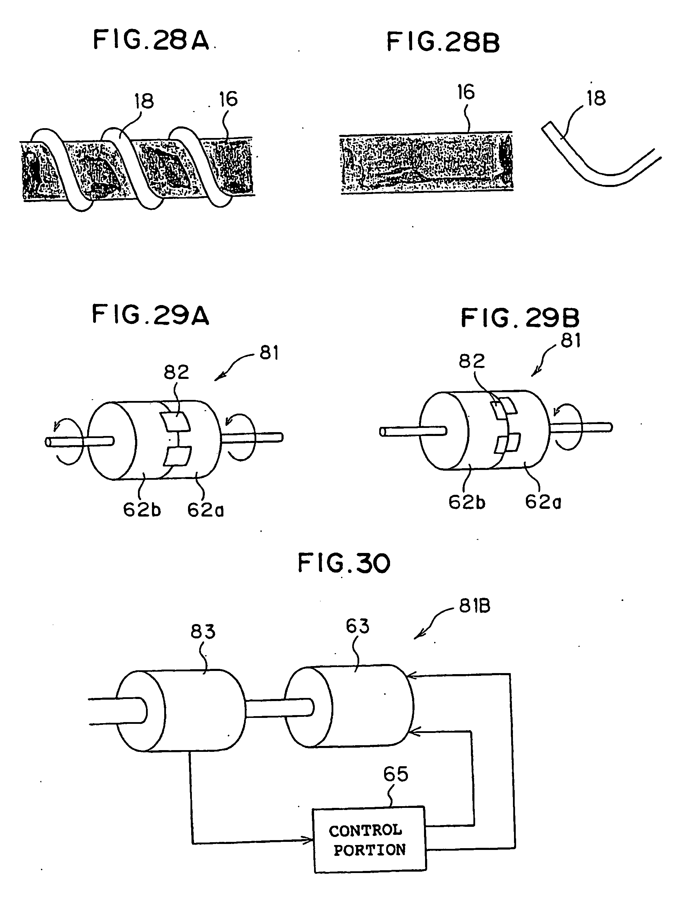

In addition, referring to Figs. 28A and 28B, in the tube structures according to a fifth modification, in order to improve the property of pull-out operation, after inserting the tube 16, the spiral structure 18 is detached from the tube 16. That is, according to the fifth modification, referring to Fig. 28A, the spiral structure 18 is fixed to the distal end and the proximal end of the tube 16 by the adhesion or the like.

In the pull-out operation of the tube 16, the proximal end of the spiral structure 18 is pulled by force of a predetermined value or more, thereby resetting the fixing of the distal end by the adhesion. Referring to Fig. 28B, the spiral structure 18 is detached from the tube 16.

Fig. 29A shows a rotation regulating mechanism 81 according to a sixth modification. According to the sixth modification, e.g., an adhesive tape 82 is adhered to the two discs 62a and 62b so as to maintain the connecting state thereof.

Referring to Fig. 29A, the rotation of the disc 62a on the motor side allows the disc 62b to rotate by predetermined torque or less.

Referring to Fig. 29B, the connection is broken by separating or breaking the adhesive tape 82 by predetermined torque or more. Thus, the disc 62a on the motor side rotates and, however, the disc 62b does not rotate. As described above, the operation of operation torque or more regulates the rotation. An adhesive for connection is not limited to the adhesive tape 82 and may be another means. For example, such connecting means may connect the discs 62a and 62b by magnet, and may separate the connection therebetween by predetermined torque or more.

Fig. 30 shows a rotation regulating mechanism 81B according to a seventh modification. According to the seventh modification, a torque sensor 83 for detecting the torque is connected to the rotating shaft of the motor 63. That is, according to the seventh modification, the rotation regulating mechanism 81B uses the torque sensor 83, in place of arranging the torque limiter 62 to the rotating shaft of the motor 63 as shown in Fig. 17.

The torque sensor 83 outputs a torque detecting signal to the control portion 65. The control portion 65 monitors whether or not the torque detecting signal indicates a predetermined torque value or more, and stops the rotation of the motor 63 when the torque detecting signal indicates a predetermined torque value. Alternatively, rotating speed control means having a function reducing the rotating speed may be arranged to prevent the state in which the torque detecting signal indicates the predetermined value or more.

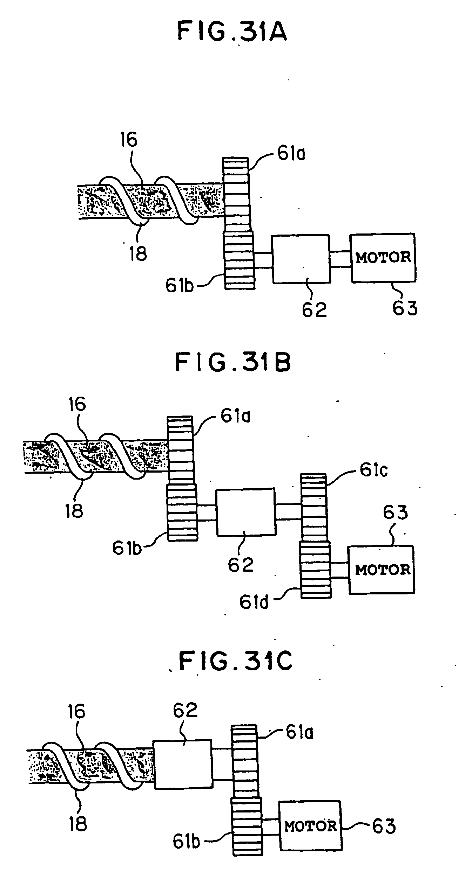

Figs. 31A and the like show examples of arrangement places of the torque limiter 62 shown in Fig. 24A and the like. Properly, the torque limiter 62 is installed between the motor 63 and a gear 61b, between gears 61a and 61b and gears 61c and 61d, or between the gear 61a and the tube 16. Figs. 31A to 31C specifically show the install places. Referring to Fig. 31A, the torque limiter 62 is arranged similarly to that shown in Fig. 17.

That is, the gear 61b engaged with the gear 61a attached to the proximal end of the tube 16 is connected to the motor 63 via the torque limiter 62.

According to the modification, referring to Fig. 31B, the gear 61c and the gear 61d are inserted between the torque limiter 62 and the motor 63 shown in Fig. 31A.

According to the modification, referring to Fig. 31C, the torque limiter 62 with the hollow structure is attached to the proximal end of the tube 16, the gear 61a is attached to the hollow shaft of the torque limiter 62, and the gear 61a is engaged with the gear 61b attached to the rotating shaft of the motor 63.

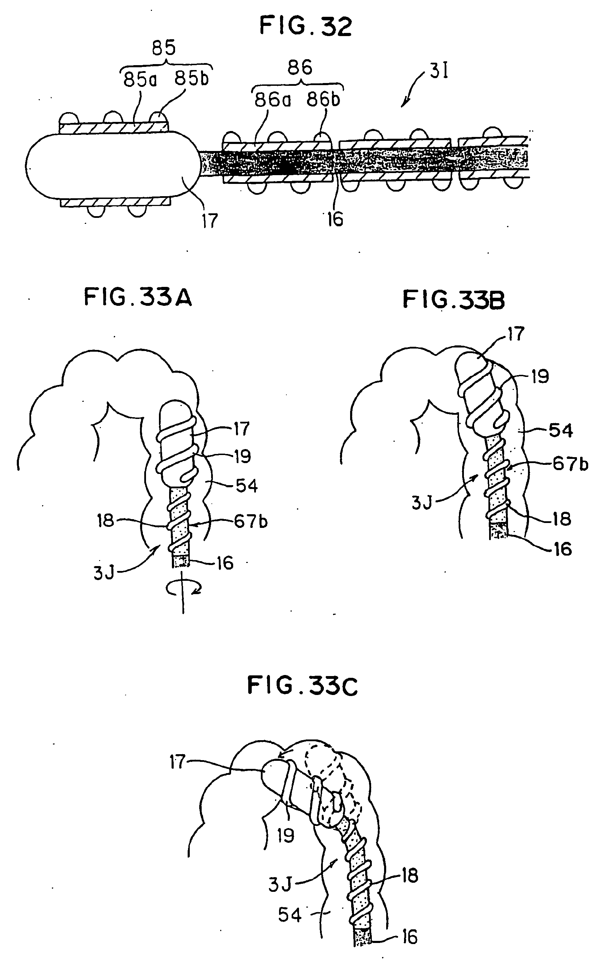

Referring to Fig. 31A, etc., the torque at a predetermined value or more operates and then the torque limiter 62 regulates the transmission of rotation. Fig. 32 shows the structure of partly regulating the rotation, differently from those shown in Fig. 31A, etc.

In an endoscope insertion aiding device 3I according to an eight modification, cylindrical structures 85 and 86, serving as rotation regulating mechanisms, having cylindrical members 85a and 86a with proper lengths having a spiral structure 85b and a spiral structure 86b are fit into the distal-end member 17 and the tube 16.

The friction between the outer circumferential surface of the tube 16 and the inner circumferential surface of the cylindrical member 86a allows the tube 16 to cause slip to the cylindrical structure 86 when rotation with predetermined torque or more is tried (when the outer circumferential surface of the cylindrical structure 86 comes into contact with the inner wall of the body cavity). By dividing the cylindrical structure 86 into a plurality of sections, at position where the resistance for rotation is high, specifically where the cylindrical structure 86 is strongly in contact with the inner wall of the peripheral body cavity and is difficult to rotate, the rotation of the cylindrical structure 86 will stop, while at other positions cylindrical structure 86 will rotate, and then obtains the thrust. The distal-end member 17 side has the similar operation. That is, the friction between the outer circumferential surface of the distal-end member 17 and the inner circumferential surface of the cylindrical member 85a allows the distal-end member 17 to rotate to the cylindrical structure 85 by predetermined torque or more, thereby causing the slip.

When the cylindrical structure 85 strongly comes into contact with the inner wall of the body cavity and does not rotate, the rotation of the cylindrical structure 85 stops. Since the distal-end member 17 has the length shorter than the tube 16, only one cylindrical structure 85 is arranged. However, the cylindrical structure 85 may be divided into a plurality of sections.

Next, a ninth modification will be described. The bending mechanism for bending operation in the four up, down, right, and left directions is arranged as shown in Fig. 17. However, an endoscope insertion aiding device 3J according to the ninth modification has a bending portion 67b for bending operation only in one direction. In this case, in the insertion, the insertion into the bent body cavity is smooth by the following.

That is, Figs. 33A to 33C show states of insertion into the body cavity 54 such as the large intestine. Referring to Fig. 33A, in the insertion into the straight body cavity 54, the insertion is possible by rotating in the straight state. Referring to Fig. 33B, when the endoscope reaches the bent portion, the rotation first stops, the bending portion is bent in one direction, and an image of the inserted endoscope is viewed to check the current bending direction. When the direction is different from the desired direction (bending direction of the body cavity), the rotation slowly restarts so that the bending direction matches the advancing direction. In the state, the bending function is reset, the rotation starts at the normal speed, and the endoscope is inserted in accordance with the bent portion.

The repetition of the above operation enables the insertion into the deep portion as shown in Fig. 33C.

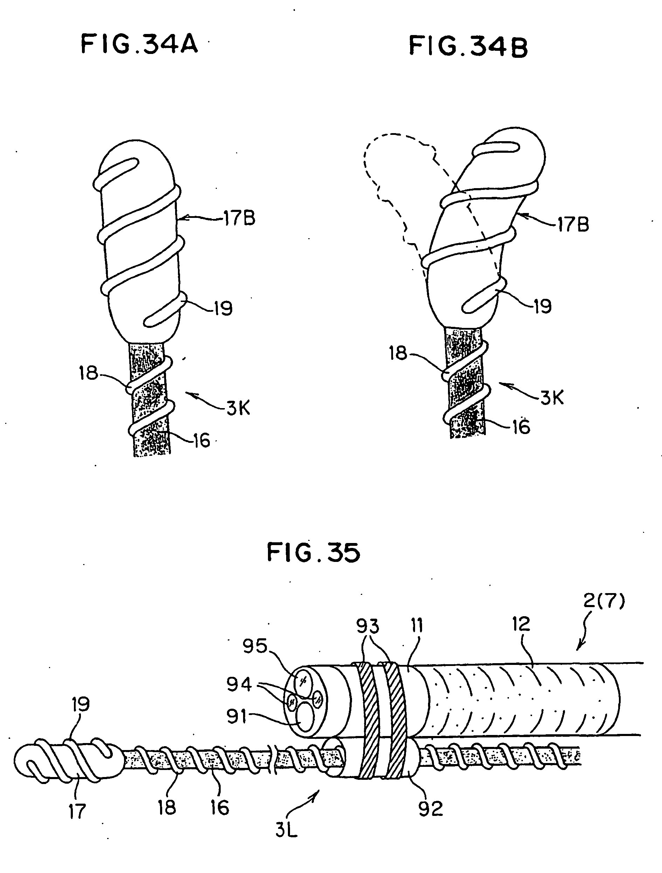

Next, a tenth modification will be described. Figs. 34A and 34B show a distal-end side of an endoscope insertion aiding device 3K according to the tenth modification. According to the tenth modification, a distal-end member 17B, in place of the distal end of the tube 16, is formed by using the EPAM described with reference to Fig. 21. The distal-end member 17B is bent in four directions or at least one direction.

The bendable distal-end member 17B is formed, thereby bending the distal-end member 17B as shown in Fig. 34B from the straight state as shown in Fig. 34A. The bending structure of the distal-end member 17B facilitates the smooth insertion.

That is, since the distal-end member 17B contains a soft material and has the bending function, the rigid length is short. Upon inserting the distal-end member 17B in the body cavity, the distal-end member 17B can be bent in accordance with the bent portion and thus the insertability is preferably ensured.

Further, the distal-end member 17B may not have the bending function and may contain a soft material to be bent in accordance with the applied force.

In this case, the distal-end member is passively bent along the bending portion of the intestine, thereby preferably ensuring the insertability.

### (Third embodiment)

Next, a third embodiment of the present invention will be described. Fig. 35 schematically shows an endoscope insertion aiding device 3L according to the third embodiment of the present invention. The endoscope insertion aiding device 3L is attached to the outer circumferential surface of the endoscope 2, thereby supporting the insertion.

The endoscope insertion aiding devices 3 to 3K according to the first and second embodiments have the hollow portion for inserting the inserting portion 7 of the endoscope 2 and the inserting portion 7 inserted into the hollow portion has a fine diameter. Then, although the endoscope insertion aiding devices 3 to 3K substantially observe the image, the endoscope insertion aiding devices 3 to 3K are limited to ones without any channels for inserting the treatment tool. In this case, the treatment is not possible.

Then, according to the third embodiment, the endoscope insertion aiding device 3 can be applied to the endoscope 2 having a channel 91 in which the treatment tool can be inserted.

Thus, according to the third embodiment, the insertion is aided by attaching the endoscope 2 onto the outer circumferential surface as described above.

The endoscope inserting aiding device 3L is inserted, like a guide wire, into the body cavity such as the large intestine for insertion (in advance of the endoscope 2). After inserting the endoscope inserting aiding device 3L, the inserting portion 7 of the endoscope 2 having a channel that cannot be inserted is easily inserted.

In the endoscope insertion aiding device 3L according to the third embodiment, the spiral structures 18 and 19 onto the outer circumferential surfaces of the tube 16 and the distal-end member 17 arranged to the distal end thereof pass through a cylinder 92 serving as a thrusting holder. Further, in the endoscope insertion aiding device 3L, a tape 93 fixes the cylinder 92 to the distal-end portion 11 of the endoscope 2.

The tube 16 having the spiral structure 18 freely movably passes through the cylinder 92.

According to the third embodiment, the tube 16 and the distal-end member 17 have a hollow portion 16a and a through-hole 17a which are used for inserting the treatment tool therein with the fine diameter. However, the hollow portion 16a and the through-hole 17a may have the solid string-structure.

As described above according to the second embodiment with reference to Fig. 17, the proximal end of the tube 16 is connected to the rotation driving device 60. The proximal end of the tube 16 is rotated, thereby smoothly thrusting the tube 16.

The proximal end of the spiral structure 18 is connected to the compressor 64 according to the second embodiment shown in Fig. 17. By feeding and discharging the air, the concaved and convexed portions of the spiral structure 18 having the hollow tube can be adjusted as described with reference to Fig. 18A and the like.

The distal-end portion 11 of the endoscope 2 comprises an illuminating window 94 and an observing window 95.

With the structure according to the third embodiment, referring to Fig. 35, the endoscope insertion aiding device 3L is inserted into the cylinder 92, and the cylinder 92 is fixed to the distal-end portion 11 of the endoscope 2 for endoscope examination or therapeutic treatment.

The distal-end member 17 of the endoscope insertion aiding device 3L projected in front of the distal-end portion 11 of the endoscope 2 is inserted in the large intestine in advance. The proximal end of the tube 16 is rotated by the rotation driving mechanism, thereby smoothly thrusting the endoscope insertion aiding device 3L and inserting it into the deep portion in the body cavity such as the large intestine.

After inserting the endoscope insertion aiding device 3L, the proximal end of the endoscope 2 is pressed, thereby smoothly inserting the distal end of the inserting portion 7 of the endoscope 2 into the deep portion in the body cavity such as the large intestine by using the endoscope insertion aiding device 3L as a guiding device.

Upon inserting the distal end of the inserting portion 7 of the endoscope 2 into the deep portion in the body cavity such as the large intestine, the air is discharge by the compressor 64 in the endoscope insertion aiding device 3L. Thus, the surface of the tube 16 is flat as shown in Fig. 18B and then the endoscope 2 is smoothly inserted.

According to the third embodiment, the endoscope insertion aiding device can be used not only for the endoscope 2 having the inserting portion 7 with the fine diameter without the channel but also for the endoscope 2 having the inserting portion 7 with the thick diameter having the channel 91, for aiding the insertion of the endoscope 2.

In addition to the structures shown in Figs. 18A to 18C, the similar operations and advantages are obtained by inserting the endoscope 2 by the structures according to modifications with reference to Figs. 25 to 28B.

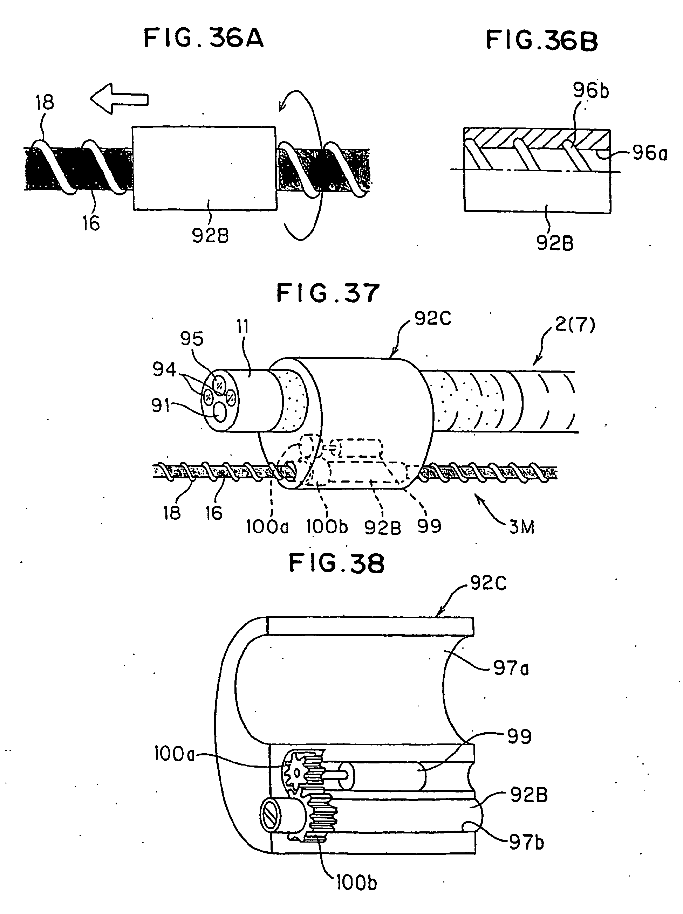

Fig. 36A shows a thrusting holder according to a first modification. The thrusting holder comprises a nut guide 92B comprising a hole 96a through which the tube 16 passes as shown in Fig. 36B and a spiral groove 96b which has a groove matching the pitch of the spiral structure 18 arranged onto the outer circumferential surface of the tube 16 and which accommodates therein the spiral structure 18.

According to the first modification, the endoscope 2 having the thick inserting portion 7 with the channel 91 is effectively thrust.

A thrusting holder 92C shown in Fig. 37 has a hole 97a for passage of the periphery of the distal-end portion 11 of the inserting portion 7 of the endoscope 2 as shown in the cutting view shown in Fig. 38, and a hole 97b for freely rotatably holding the nut guide 92B for passage of the tube 16 having the spiral structure 18.

The thrusting holder 92C has a motor 99 for rotational drive. A gear 100a attached to a rotating shaft of the motor 99 is engaged with a gear 100b attached onto the outer circumferential surface of the nut guide 92B. The thrusting holder 92C around the gears 100a and 100b is notched so as to rotate the gears 100a and 100b.

The motor 99 is connected to the control portion 65 on the hand side via a signal line (not shown). The rotation and the stop of the motor 99 is controlled by operating the operating portion 66.

A user such as an operator operates the operating portion 66, thereby driving the motor 99. Thus, the nut guide 92B is rotationally driven. The nut guide 92B has, on the inner circumferential surface thereof, the spiral groove for passage of a hole for passage of the tube 16 and the spiral structure 18 that is engaged with the hole described with reference to Fig. 36B.

With the above-described structure, the motor 99 for rotational drive attached to the thrusting holder 92C is rotated after inserting the tube 16 into the body cavity such as the large intestine, thereby thrusting the distal end of the endoscope 2 along the tube 16 that automatically functions as a guide wire.

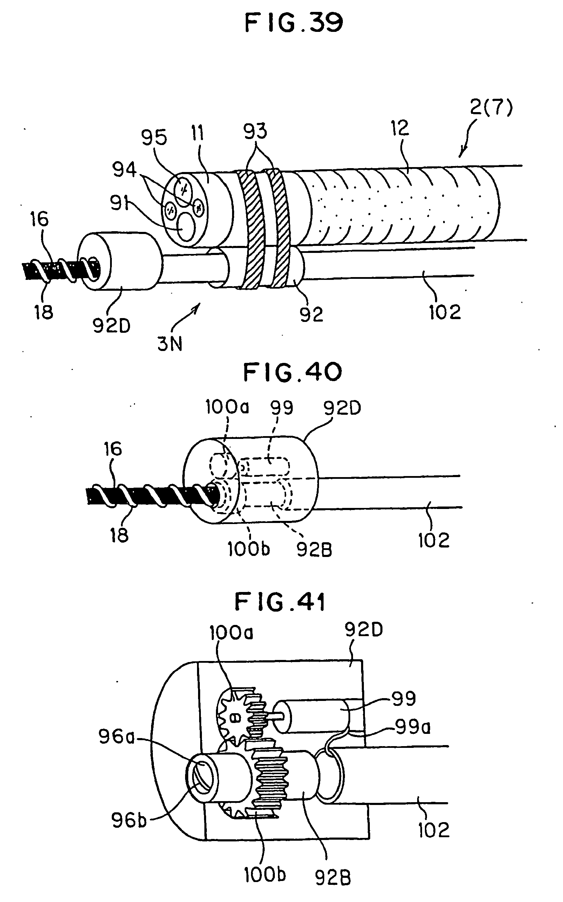

Fig. 39 shows a state of attaching, to the endoscope 2, the distal end of an endoscope insertion aiding device 3N according to a second modification. Although the tube 16 having the spiral structure 18 arranged in the cylinder 92 passes through the endoscope insertion aiding device 3L, according to the second modification, a sheath 102 which covers the tube 16 having the spiral structure 18 passes through the endoscope insertion aiding device 3N.

Further, according to the second modification, a thrusting holder 92D is arranged to the distal end of the sheath 102. Fig. 40 shows the thrusting holder 92D. Fig. 41 shows the internal structure of the thrusting holder 92D. The thrusting holder 92D has the similar structure to that shown in Fig. 38.

Referring to Fig. 41, the thrusting holder 92D includes the motor 99 for rotational drive, the gear 100a attached to the rotating shaft of the motor 99, the gear 100b, and the nut guide 92B having the gear 100b.

The user such as the operator operates the operating portion 66 after inserting the tube 16 into the deep portion in the body cavity to rotate the motor 99. Thus, the nut guide 92B freely rotatably held in the thrusting holder 92D is rotationally driven, thereby thrusting the sheath 102 to the distal end of the tube 16.

According to the second modification, the sheath 102 having the flat outer circumferential surface covers the tube 16 having the spiral structure 18 onto the outer circumferential surface and, advantageously, the inserting operation of the endoscope 2 is smooth.

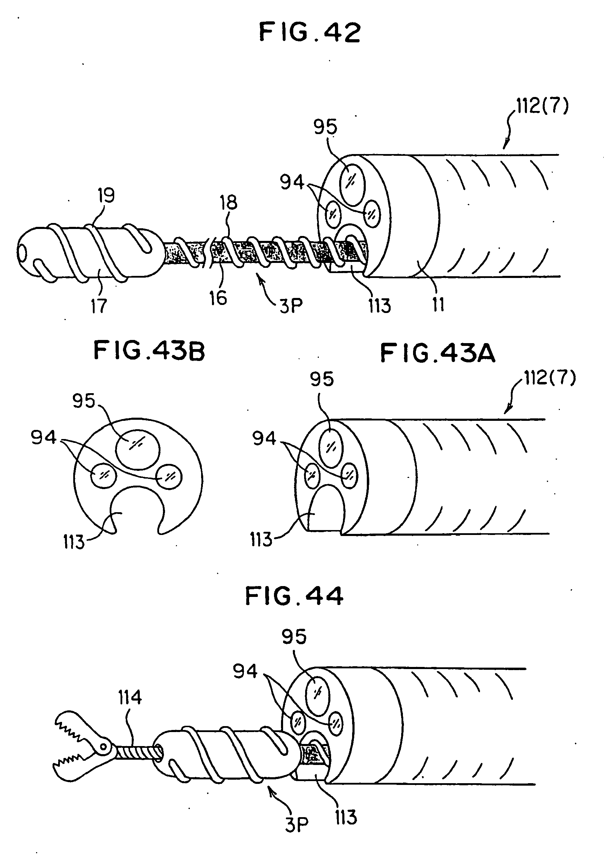

Fig. 42 shows a state of inserting, into a dedicated endoscope 112, the distal end of an endoscope insertion aiding device 3P according to a third modification. According to the third modification, referring to Fig. 43A, the endoscope insertion aiding device 3P uses a distal-end opening 113 (and channel having the same cross-sectional shape as that of the distal-end opening 113) that is inserted and pulled-out from the down direction. The endoscope insertion aiding device 3P is projected forward from the distal-end opening 113 for aiding the insertion.

Fig. 43A shows a perspective view showing the distal end of the endoscope 112. Fig. 43B shows a front view.

The endoscope 112 has the inserting portion 7 and other portions having the same structure as that of the endoscope 2.

According to the third modification, the endoscope insertion aiding device 3P is used like a guide wire.

Referring to Fig. 44, a treatment tool 114 is inserted in the hollow portion of the tube 16 for therapeutic treatment in the endoscope insertion aiding device 3P.

Although not shown, it is possible to utilize a using method for inserting, from the distal end of the endoscope, the endoscope insertion aiding device into the channel of the endoscope for treatment tool having a channel with the thick diameter or a plurality of channels.

### (Fourth embodiment)

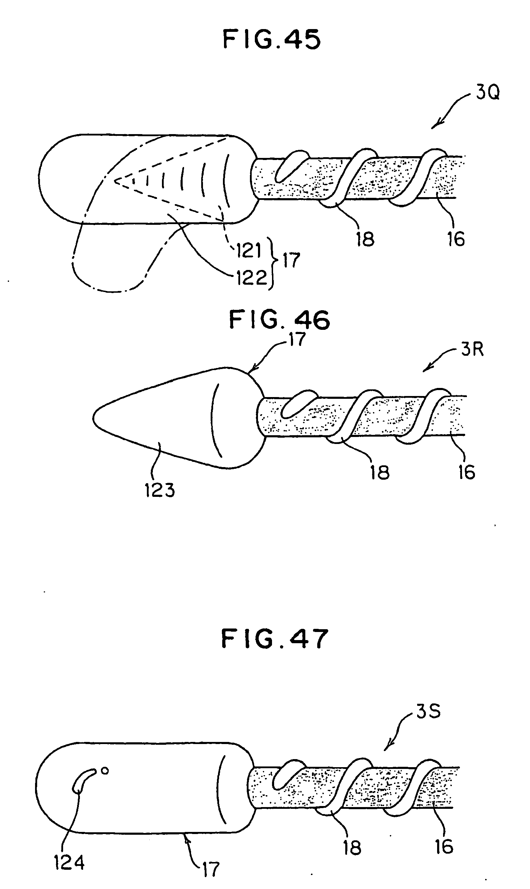

Next, a fourth embodiment of the present invention will be described. Fig. 45 shows the structure of the distal end of an endoscope insertion aiding device 3Q according to the fourth embodiment of the present invention. According to the fourth embodiment, the endoscope insertion aiding device 3Q does not have any spiral structures on the distal-end member 17.

In the endoscope insertion aiding device 3Q, the rigidity of the distal-end member 17 is softer near the distal end thereof, and it sequentially changes near the proximal end thereof.

Specifically, the distal-end member 17 comprises a conical member 121 with high rigidity as shown by a dotted line and a member 122 with low rigidity which covers the outer circumferential surface of the conical member 121 with high rigidity.

The distal end of the distal-end member 17 is smoothly inserted in the body cavity. When the tip end of the lumen is bent in the down direction, the distal end of the distal-end member 17 is bent in accordance with the bending operation as shown by an alternate long and short dash line to smoothly insert the distal end of the distal-end member 17. Other structures are the same as those according to the first embodiment.

With the above-described structure, advantageously, the change in rigidity of the distal-end member 17 according to the fourth embodiment is easily bent to improve the following operation in accordance with the bending operation.

Fig. 46 shows the structure of the distal end of an endoscope insertion aiding device 3R according to a first modification. The endoscope insertion aiding device 3R is shaped with a conical surface 123 which is reduced in outer diameter to more peripheral distal-end of the distal-end member 17, or is taper-shaped with the thinner portion near the distal end. Advantageously, according to the first modification, the passing property in the closed lumen is improved.

Fig. 47 shows the structure of the distal end of an endoscope insertion aiding device 3S according to a second modification. In the endoscope insertion aiding device 3S, a lubrication agent 124 coats the surface of the distal-end member 17 shown in Fig. 45 and thus the slipping performance of the surface of the distal-end member 17 is improved.

According to the second modification, the slipping performance of the distal-end member 17 is improved by the lubrication, thereby improving the insertability. The lubrication agent may be a fluoropolymer coating of Teflon (registered trademark) with high slipping performance or a hydrophilic lubrication agent of photocatalyst.

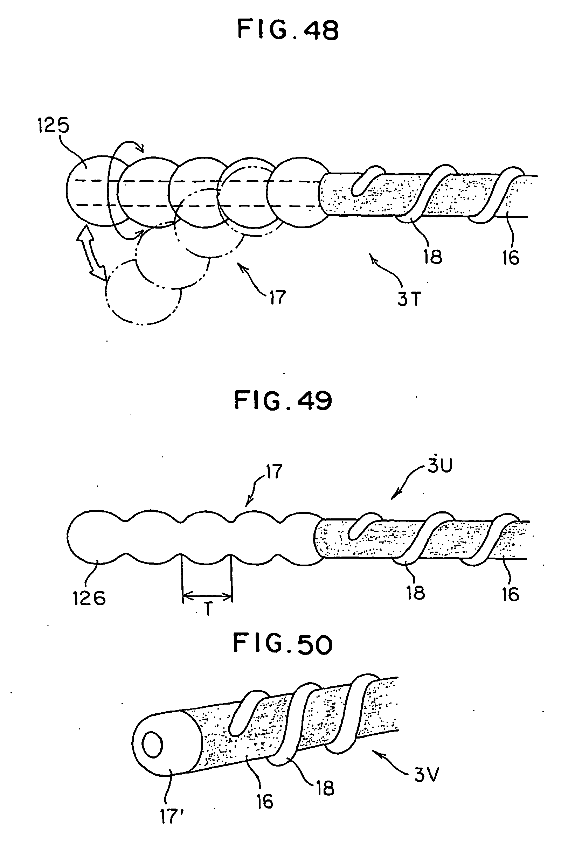

Fig. 48 shows the structure of the distal end of an endoscope insertion aiding device 3T according to a third modification. The endoscope insertion aiding device 3T has the distal-end member 17 in which a plurality of hollow beads 125 are freely rotatably connected. With the above-described structure, the distal-end member 17 is easily bent.

In the insertion into the body cavity, when the tip end is bent in the down direction, the endoscope is bent in the direction as shown by an alternate long and short dash line to improve the following property to the bent portion.

According to the third modification, the distal end is softly bent and, advantageously, the following property is improved.

Fig. 49 shows the structure of the distal end of an endoscope insertion aiding device 3Y according to a fourth modification. In the endoscope insertion aiding device 3Y, the rigidity of the member 125 forming the distal-end member 17 changes at a predetermined term T. Specifically, circular convexed portions and circular concaved portions are formed at the distal end of the tube 16 along the longitudinal direction of the tube 16 at the predetermined term T. The rigidity of the portion having the concaved portion is reduced to easily bend the distal-end member.

According to the fourth modification, the rigidity varies and thus, advantageously, the distal-end member is easily bent and the following property for bending operation.

According to the embodiments, the distal-end member 17 is thicker than the outer diameter of the tube 16. However, referring to Fig. 50, an endoscope insertion aiding device 3V may have a distal-end member 17' with the same outer diameter as that of the tube 16, serving as the distal-end member 17.

The endoscope insertion aiding device 3V has the distal-end member 17' with the same outer diameter as that of the tube 16 at the distal end of the tube 16 having the spiral structure 18. The endoscope 2 can be inserted in the hollow portion.

According to the modification, the insertability to the body cavity is preferably ensured.

The shape and rigidity of the distal-end member 17' shown in Fig. 50 may be applied to the distal-end member 17.

That is, according to the present invention, the distal-end member has approximately the same or more maximum outer diameter as that of the tube 16.

According to the present invention, the embodiments are partly combined and are partly changed.

### (Fifth embodiment)

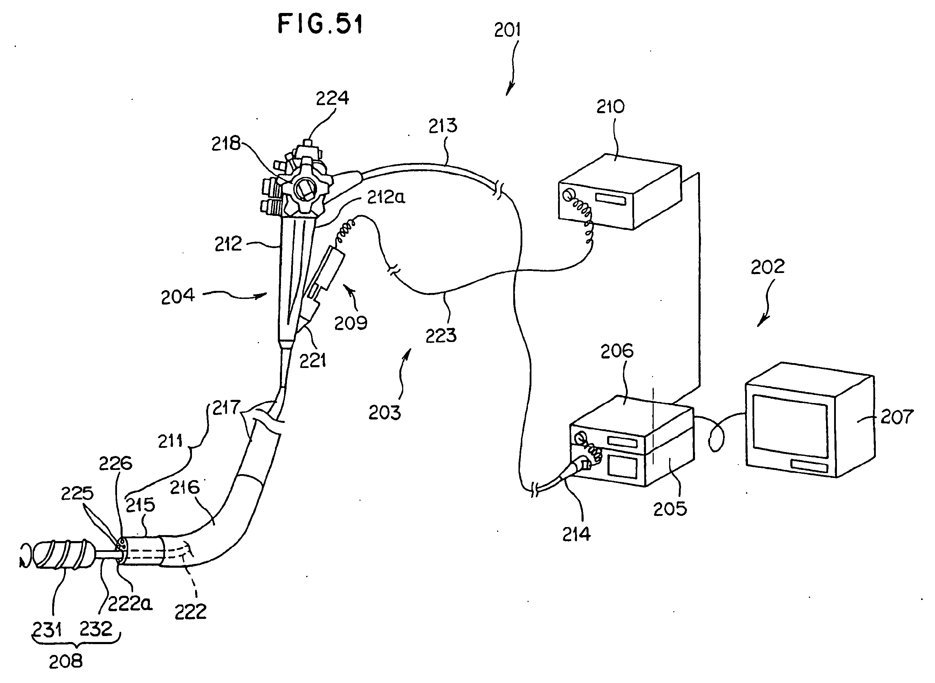

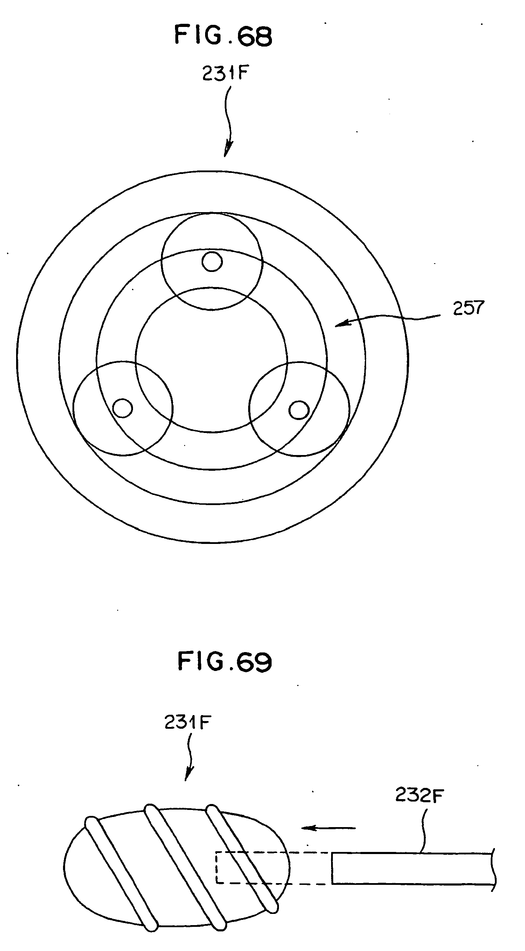

Next, a fifth embodiment of the present invention will be described with reference to Figs. 51 to 71.

Referring to Fig. 51, an endoscope insertion aiding system 201 comprises: an endoscope device 202 having an inserting portion, which will be described later, inserted in the body cavity; and an endoscope insertion aiding device 203 which improves the insertability of an inserting portion of the endoscope device 202.

The endoscope device 202 comprises: an endoscope 204 having an observing window, which will be described later; a light source device 205 which supplies illumination beam to the endoscope 204; a CCU (camera control unit) 206 which performs signal processing of an image pickup portion (not shown) of the endoscope 204; and a monitor 207 which receives a video signal from the CCU 206 and displays endoscope images.

The endoscope inserting aiding device 203 comprises: a spiral thrusting probe 208 which comes into contact with the inner wall of the body cavity and generates the thrust to guide an inserting portion of the endoscope 204 to the target portion in the body cavity; a spiral driving unit 209 which supplies driving force to a spiral thrusting unit 231, which will be described later, of the spiral thrusting probe 208; and a spiral-thrust control device 210 which controls the spiral driving unit 209.

First, the structure of the endoscope device 202 will be described.

The endoscope 204 comprises: an inserting portion 211 which is elongated and flexible; and an operating portion 212 which is continuously arranged to the proximal-end side of the inserting portion 211 and has a common function of a grip portion 212a. In the endoscope 204, a universal cord 213 is extended from the operating portion 212. A light guide and a signal line (which are not shown) are inserted into the universal cord 213. A connector portion 214 arranged to the end of the universal cord 213 is connected to the CCU 206.

The inserting portion 211 of the endoscope 204 has a rigid distal-end portion 215, a freely bendable bending portion 216, and a flexible tube portion 217 which is long and flexible are continuously arranged. The distal-end portion 215 is arranged to the distal end of the inserting portion 211. The bending portion 216 is arranged to the proximal end of the distal-end portion 215. The flexible portion 217 is arranged to the proximal end of the bending portion 216.

The operating portion 212 of the endoscope 204 has the grip portion 212a at the proximal end thereof. The grip portion 212a is gripped by an operator. A video switch (not shown) for remotely controlling the CCU 206 is arranged on the top side of the operating portion 212. A video switch (not shown) for operating the absorption and an air/water feed switch (not shown) for operating the air feed and the water feed are arranged to the operating portion 212. A bending operation knob 218 is arranged to the operating portion 212, and the bending portion 216 is bent by operating the bending operation knob 218 with the grip operation of the grip portion 212a.

The operating portion 212 comprises an inserting port 221 of the treatment tool in which a treatment tool such as biopsy forceps near the front end of the grip portion 212a. The inserting port 221 of the treatment tool is communicated with a channel 222 for inserting the treatment tool therein. The treatment tool (not shown) such as forceps is inserted into the inserting port 221 of the treatment tool and thus the distal-end side of the treatment tool is projected form a channel opening 222a formed to the distal-end portion 215 via a channel 222 for inserting the treatment tool for biopsy.

According to the fifth embodiment, the proximal end of a flexible tube, which will be described later, of the spiral thrusting probe 208 is inserted from the channel opening 222a of the channel 222 for inserting the treatment tool. The proximal end of the flexible tube is pulled-out from the inserting port 221 of the treatment tool and is connected to the spiral driving unit 209 attached to the operating portion 212. The spiral driving unit 209 and the spiral-thrust control device 210 are electrically connected by a connecting cable 223.

A driving switch 224 for on/off operation of the spiral driving unit 209 is arranged to the operating portion 212. An on-signal from the driving switch 224 is inputted to the spiral-thrust control device 210 via the CCU 206, then, the spiral driving unit 209 is driven by power and a control signal from the spiral-thrust control device 210, and the driving force is supplied to the spiral thrusting probe 208.

The driving switch 224 may be connected to the spiral-thrust control device 210 to be detachably attached to the operating portion 212.

In the endoscope 204, a light guide (not shown) is inserted into the universal cord 213, the inserting portion 211, and the operating portion 212. The proximal end of the light guide passes through the operating portion 212 and reaches the connector portion 214 of the universal cord 213 so as to transmit the illumination beam from the light source device 205. The illumination beam transmitted from the light guide illuminates a subject of the affected portion from an illuminating window 225 via an illuminating optical system (not shown) arranged to the distal-end portion 215 of the inserting portion.

The reflecting light of the illuminated subject is captured as a subject image from an observing window 226 arranged adjacently to the illuminating window 225. The captured subject image is picked-up by the image pickup portion of a CCD (charge-coupled device) arranged at the image forming position via the objective optical system, is photoelectrically converted, and is converted into an image pickup signal.

The image pickup signal is transmitted to a signal cable extended from the image pickup portion, passes through the operating portion 212, and reaches a video connector of the universal cord 213. Further, the signal is outputted to the CCU 206 via the connecting cable. The CCU 206 performs signal processing of the image pickup signal from the image pickup portion of the endoscope 204, generates a standard video signal, and displays endoscope image on the inserting portion 7.

Next, the detailed description will be given of the endoscope insertion aiding device 203.

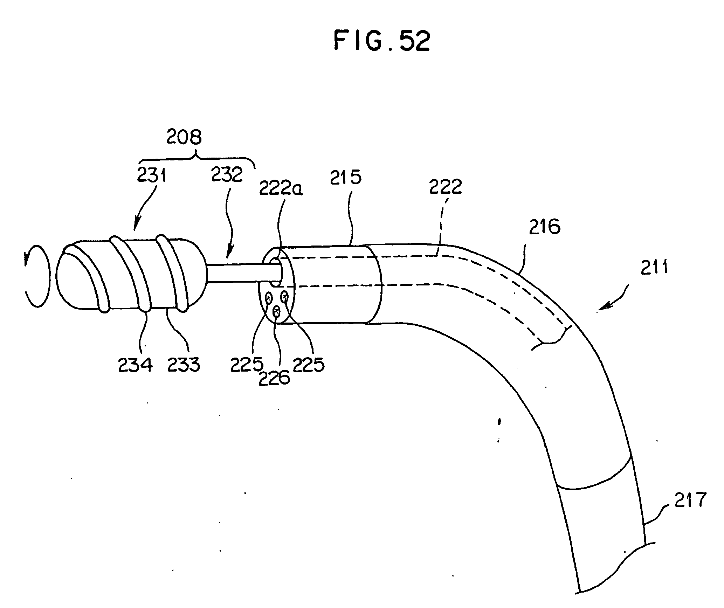

Referring to Fig. 52, the spiral thrusting probe 208 comprises: a cylindrical spiral thrusting unit 231; and a flexible tube 232 continuously arrange to the spiral thrusting unit 231.

The spiral thrusting unit 231 has a spiral projection 234, serving as a thrust generating structure portion, which generates the thrust by the rotation on the outer circumferential surface of an exterior container 233. The spiral projection 234 contains an elastic member such as rubber or rigid resin. Although the spiral projection 234 is formed in the center of the spiral thrusting unit 231 as shown in Fig. 52, up to the end of the cylindrical portion may be formed for the purpose of easy thrust.

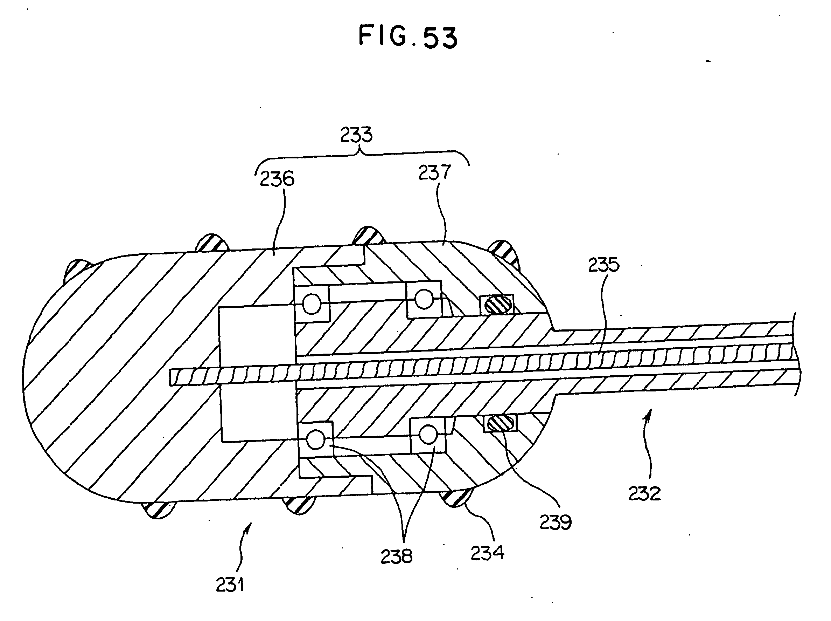

Referring to Fig. 53, a flexible shaft 235, serving as a flexible rotating shaft, is inserted to transmit the driving force for rotatably driving the spiral thrusting unit 231. The flexible rotating shaft may be a torque tube (such as a tube having a metallic net which is integrated to the inner wall of the tube by the resin-molding) or coil sheathe, in place of the flexible shaft 235.

The proximal end of the flexible tube 232 is connected to the spiral driving unit 209. The flexible shaft 235 transmits, to the spiral thrusting unit 231, the rotating force from a motor unit, which will be described later, arranged to the spiral driving unit 209.

The exterior container 233 is formed by integrally adhering and fixing a container 236 on the distal-end side and a container 237 on the proximal-end side. The distal end of the flexible shaft 235 inserted in the flexible tube 232 is pressed and fixed to the container 236 on the distal-end side. The driving force is transmitted from the flexible shaft 235.

The distal end of the flexible tube 232 is attached to the container 237 on the proximal-end side, thereby rotating the flexible tube 232 by a bearing 238. An O ring 239 allows the interval between the container 237 on the proximal-end side and the flexible tube 232 to be watertight.

In the exterior container 233, the driving force transmitted from the flexible shaft 235 to the flexible tube 232 integrally rotates the container 236 on the distal-end side and the container 237 on the proximal-end side.

Thus, the spiral projection 234 comes into contact with the body cavity to rotate the exterior container 233. Then, the spiral thrusting unit 231 can advance and retreat in the body cavity, thereby guiding the inserting portion 211 of the endoscope 204 into the body cavity. Since the spiral thrusting unit 231 is projected from the channel opening 222a of the channel 222 for inserting the treatment tool, the spiral thrusting probe 208 is within the range of the field of view of the observing window 226 of the endoscope 204. Thus, the contact state of the spiral thrusting unit 231 to the inner wall of the body cavity and the operating state are grasped.

Next, a description is given of the spiral driving unit 209 which generates the driving for rotating the spiral thrusting unit 231. As described above, the spiral driving unit 209 is attached to the inserting port 221 of the treatment tool.

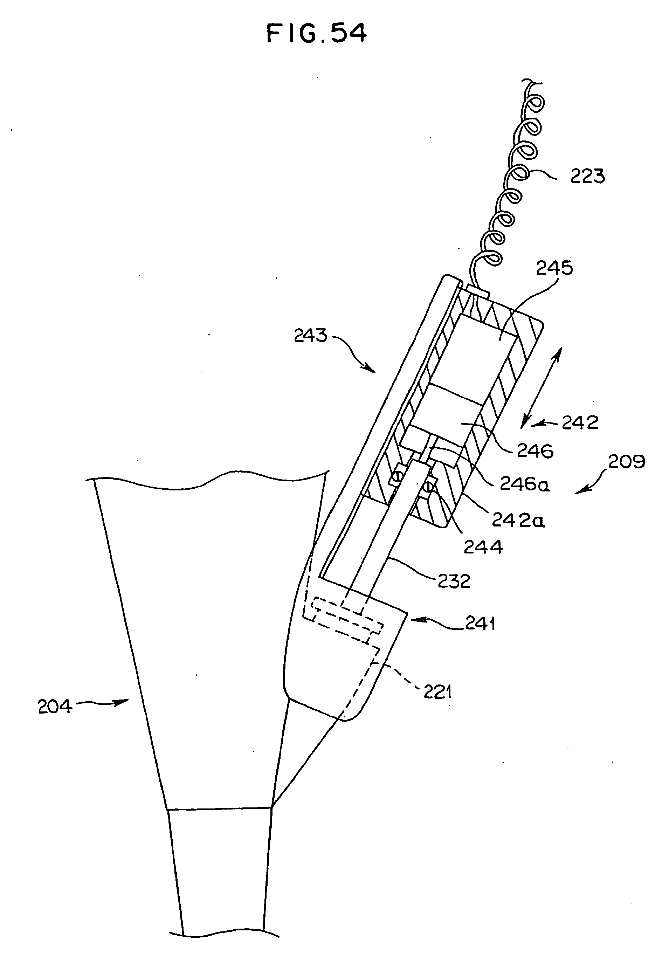

Referring to Fig. 54, the spiral driving unit 209 comprises: a motor-unit attaching portion 241 which is attached to the inserting port 221 of the treatment tool; a motor-unit portion 242 which generates the driving force for rotating the spiral thrusting unit 231 of the spiral thrusting probe 208; and a slider portion 243, serving as advancing and retreating means, which slides the motor-unit portion 242 in the vertical direction and advances and retreats the flexible tube 232.

The slide operation of the slider portion 243 advances and retreats the motor-unit portion 242, thereby advancing and retreating the flexible tube 232. Thus, the spiral thrusting unit 231 advances and retreats to a predetermined position. The spiral thrusting probe 208 advances and retreats to the position for preventing the spiral thrusting unit 231 from shielding the field of view for observation of the observing window 226 in the endoscope 204.

The slider portion 243 may be a mechanism for manually sliding the motor-unit portion 242 in the vertical direction or a mechanism for electrically sliding the motor-unit portion 242 in the vertical direction with a built-in motor. Although not shown, the slider portion 243 has a slide groove portion for sliding the motor-unit portion 242, and the slid groove portion has a slide projected portion of the motor-unit portion 242, which is slidable. Further, in the slider portion 243, the motor-unit portion 242 is positioned and is fixed at a predetermined position by a stop member such as a screw. Therefore, the spiral thrusting probe 208 is stopped to the inserting portion 211 of the endoscope 204.

The motor-unit portion 242 connects the proximal end of the flexible tube 232 pulled-out from the inserting port 221 of the treatment tool. The interval between an exterior portion 242a of the motor-unit portion 242 and the flexible tube 232 is watertight by an O ring 244.

The motor-unit portion 242 comprises: a motor 245 for generating the rotating force; and a gear 246 which inverts the rotating force of the motor 245 and communicates desired torque to an output shaft 246a. Power and a control signal are supplied from the spiral-thrust control device 210 to the motor 245 via the connecting cable 223, thereby driving the motor 245. Power may be supplied to the motor-unit portion 242 from a built-in battery.

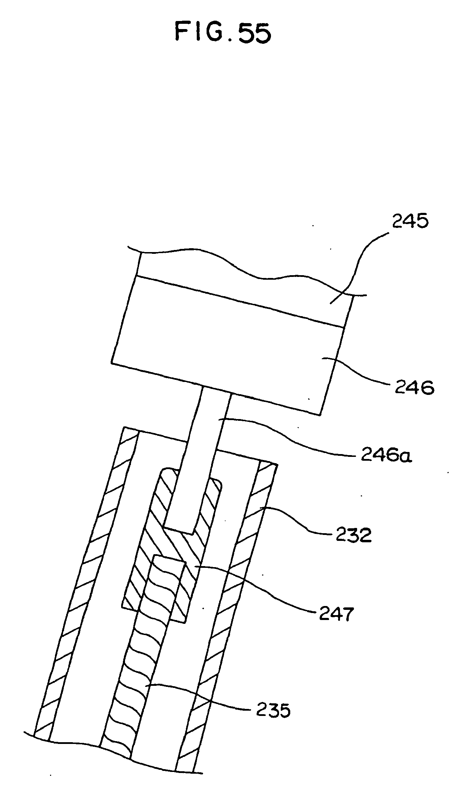

Referring to Fig. 55, in the flexible tube 232, the proximal end of the flexible shaft 235 is connected to the output shaft 246a of the motor-unit portion 242 by a connecting portion 247. The output shaft 246a is connected and fixed to the connecting portion 247 by D-cut fitting.

Thus, the spiral driving unit 209 communicates the driving force from the motor-unit portion 242 to the flexible shaft 235, thereby rotating the spiral thrusting unit 231 of the spiral thrusting probe 208.

The endoscope insertion aiding system 201 with the above-described structure is used as shown in Fig. 51. According to the fifth embodiment, the endoscope 204 is inserted from the anus.

The operator inserts the inserting portion 211 of the endoscope 204 from the anus of the patient. In this case, the inserting portion 211 of the endoscope 204 is elongated and flexible and therefore the operator presses and pulled-out the inserting portion 211 to insert the inserting portion 211 in the body cavity.

In the endoscope device 202, the endoscope image picked-up by the image pickup portion in the endoscope 204 is subjected to the signal processing by the CCU 206, and the endoscope image is displayed on the monitor 207. The operator inserts the inserting portion 211 of the endoscope 204 while viewing the endoscope image displayed on the monitor 207.

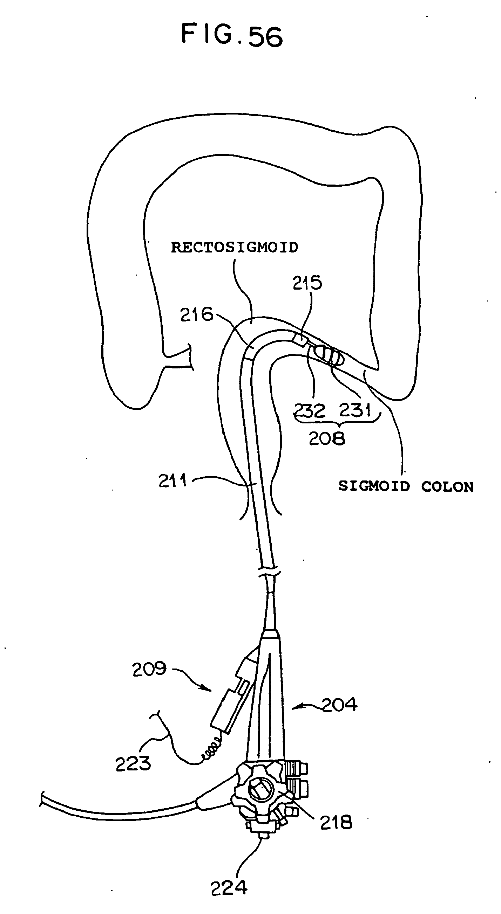

The distal-end portion 215 of the inserting portion of the endoscope 204 is inserted to the colon of the patient from the anus via the rectum. Referring to Fig. 56, in the middle of a state in which the distal-end portion 215 of the inserting portion of the endoscope 204 reaches the sigmoid colon from the sigmoid portion of the rectum, the friction force increases on the sliding surface between the outer circumferential surface of the inserting portion 211 and the inner wall of the body cavity in the direction of tangent line thereof and thus the distal-end portion 215 of the inserting portion is not inserted.

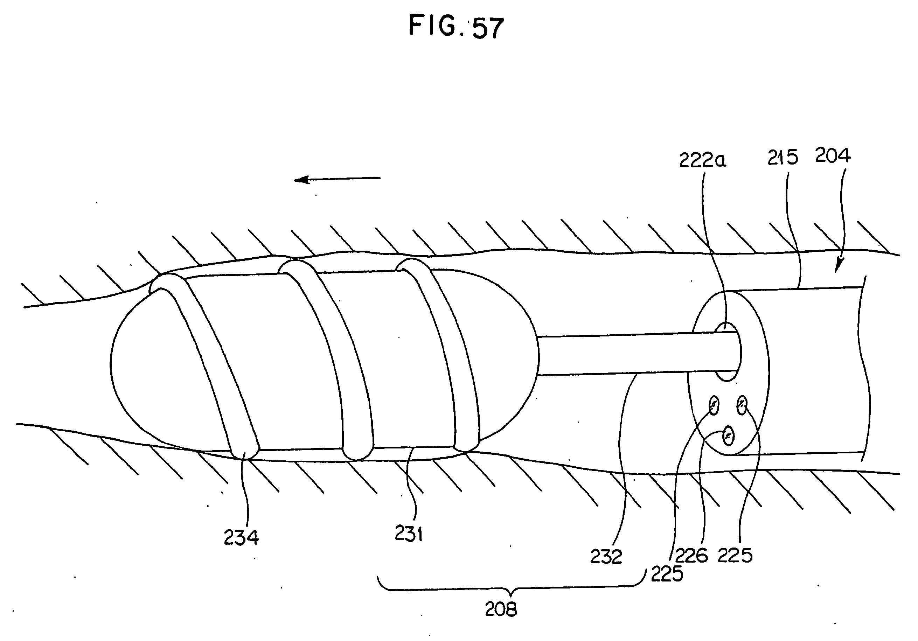

According to the fifth embodiment, as described above, the endoscope insertion aiding device 203 is arranged and the endoscope insertion aiding device 203 guides the inserting portion 211 of the endoscope 204 into the body cavity. Referring to Fig. 57, the endoscope insertion aiding device 203 projects the spiral thrusting unit 231 of the spiral thrusting probe 208 from the channel opening 222a of the channel 222 for inserting the treatment tool formed to the distal-end portion 215 of the inserting portion of the endoscope 204.

When the spiral thrusting unit 231 is out of the range of the field of view for observation of the observing window 226 in the endoscope 204, the contact state of the spiral thrusting unit 231 to the inner wall of the body cavity or the operating state is not grasped and the operating timing of the spiral thrusting unit 231 is not checked.

However, according to the fifth embodiment, the spiral thrusting unit 231 is within the range of the field of view for observation of the observing window 226 in the endoscope 204 and the body cavity is observed. Thus, the spiral thrusting unit 231 is operated at the desired timing.

That is, the operator checks the contact state and the operating state of the spiral thrusting unit 231 to the inner wall of the body cavity by the endoscope image displayed on the monitor 207. When the operator determines that the spiral thrusting unit 231 needs to be operated, he presses the driving switch 224 arranged to the operating portion 212 for on-operation.

The on-signal from the driving switch 224 is transmitted to the spiral-thrust control device 210 via the CCU 206. The spiral-thrust control device 210 outputs power and a control signal for driving the spiral driving unit 209.

The spiral driving unit 209 receives the power and the control signal from the spiral-thrust control device 210, thereby driving the motor-unit portion 242. The driving force from the motor-unit portion 242 is transmitted to the flexible shaft 235. The driving force transmitted from the flexible shaft 235 is transmitted to the spiral thrusting unit 231 of the spiral thrusting probe 208.

The container 236 on the distal-end side of the exterior container 233 receives the driving force from the flexible shaft 235 and thus the spiral thrusting unit 231 integrally rotates the flexible tube 232 together with the container 237 on the proximal-end side integrally adhered and fixed to the container 236 on the distal-end side.

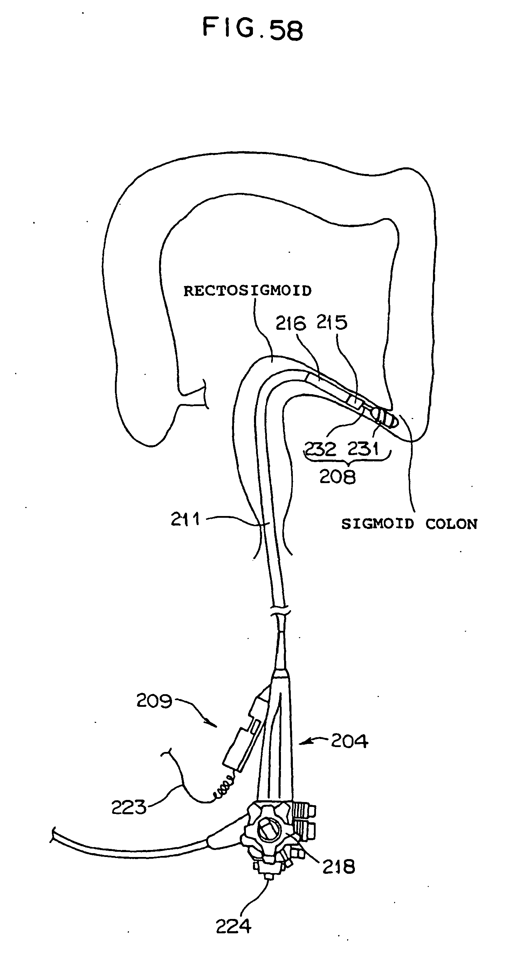

Referring to Fig. 57, the spiral projection 234 comes into contact with the inner wall of the body cavity and rotates in the lumen in the body cavity and thus the spiral thrusting unit 231 advances forward. The operator presses and advances forward the inserting portion 211 of the endoscope 204 integrally to the spiral thrusting unit 231 in accordance with the guide operation of the spiral thrusting unit 231. Further, referring to Fig. 58, the inserting portion 211 of the endoscope 204 passes through the sigmoid colon.

In the endoscope insertion aiding device 203, the slider portion 243 is slid and thus the spiral thrusting unit 231 advances the flexible tube 232, thereby advancing forward the spiral thrusting unit 231. Thus, the inserting portion 211 in the endoscope 204 may be inserted along the flexible tube 232.

As a result, the endoscope insertion aiding device 203 according to the fifth embodiment grasps the contact state of the spiral thrusting unit 231 to the inner wall of the body cavity and the operating state, thereby improving the insertability of the inserting portion 211 of the endoscope 204.

Further, the endoscope insertion aiding device 203 according to the fifth embodiment can be freely detachably attached to the endoscope 204 and thus the cleaning and the sterilization are easy. Although not shown, the spiral thrusting unit 231 comprises illuminating means such as LED (Light Emitting Diode) and image pickup means such as an image pickup portion.

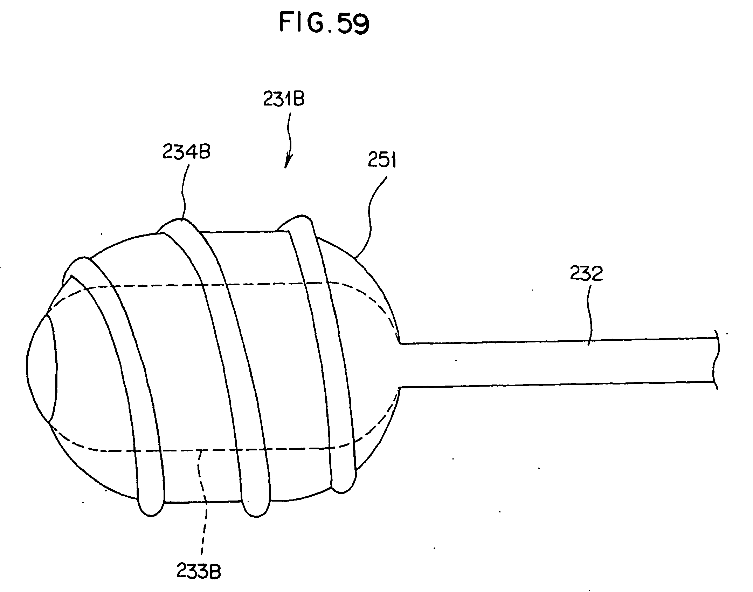

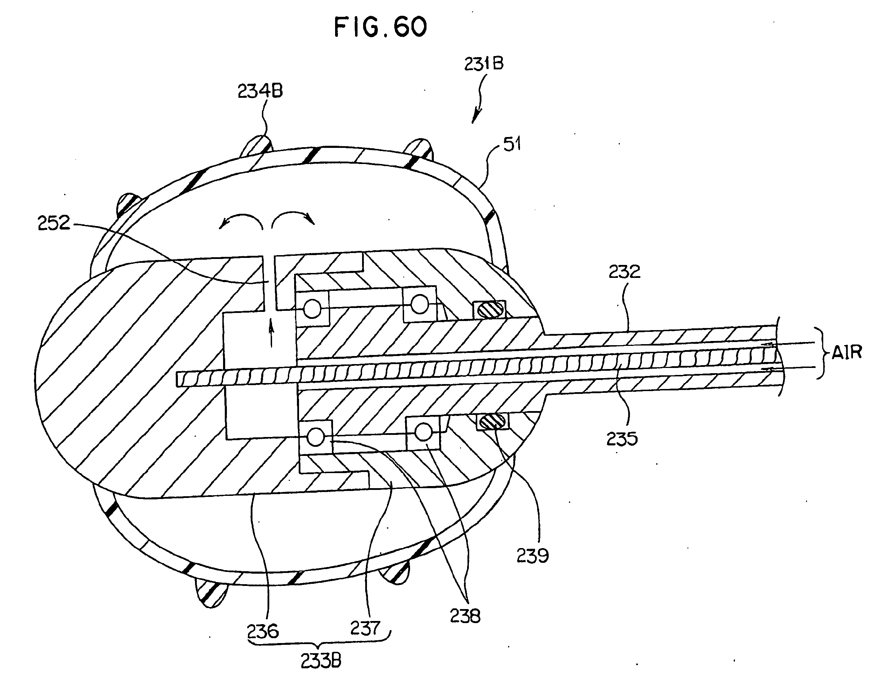

Referring to Figs. 59 and 60, the spiral thrusting unit may cover an exterior container by using a balloon.

As shown in Figs. 59 and 60, a spiral thrusting unit 231B covers an exterior container 233B by a balloon 251 having a spiral projection 234B. The spiral projection 234B contains an expandable material such as an elastic tube.

The exterior container 233B has a through-hole 252 from the inside to the outer circumferential surface in the container 236 on the distal-end side. Thus, the air is fed into the balloon 251 arranged onto the outer circumference. The flexible tube 232 is combinedly used as an air feed tube in addition to the tube of the flexible shaft 235.

Although not shown, the compressor for feeding the air is connected to the flexible tube 232. The compressor may be independent or may be arranged in the spiral driving unit 209.

The spiral thrusting unit 231B blows the balloon 251 at the portion with the large diameter of organ, thereby coming into contact with the inner wall of the body cavity. Since the diameter of lumen of the digestive tract varies depending on portions in the body cavity or persons, the contact state with the lumen (= thrust) is adjusted by controlling the amount of air filling the balloon 251.

The balloon 251 is blown when the driving switch 224 is pressed. Upon starting the air compressor and filling the balloon 251, the power and the control signal from the spiral-thrust control device 210 drive the spiral driving unit 209, thereby supplying the driving force to the spiral thrusting probe 208. Thus, the spiral thrusting unit 231B is rotated.

The spiral thrusting unit 231B absorbs the air so as to prevent that the balloon 251 becomes an obstacle when the endoscope image is obtained, the endoscope 204 observes the front portion, and the inserting portion 211 of the endoscope 204 is pulled-out. Thus, the balloon 251 is compressed.

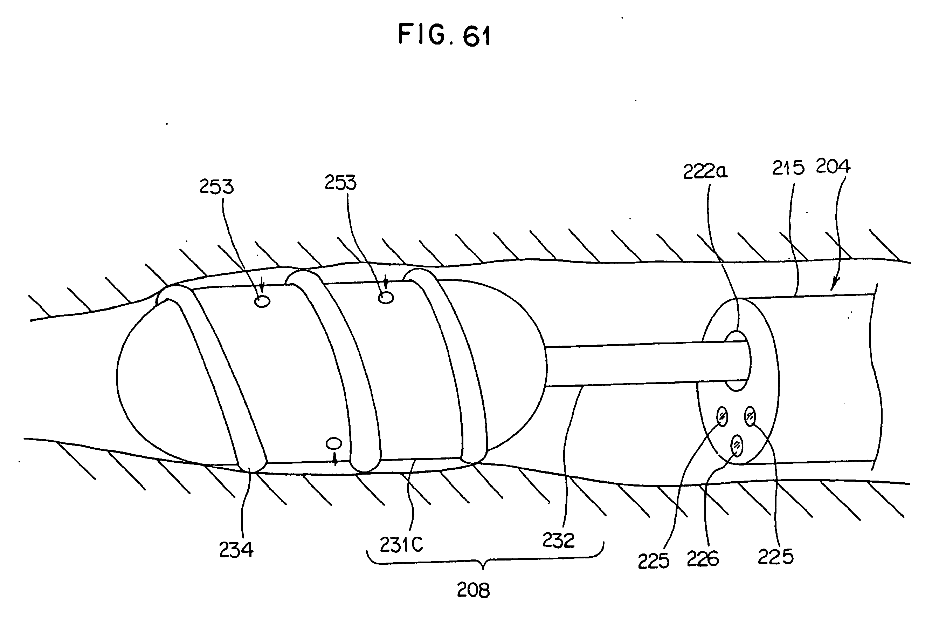

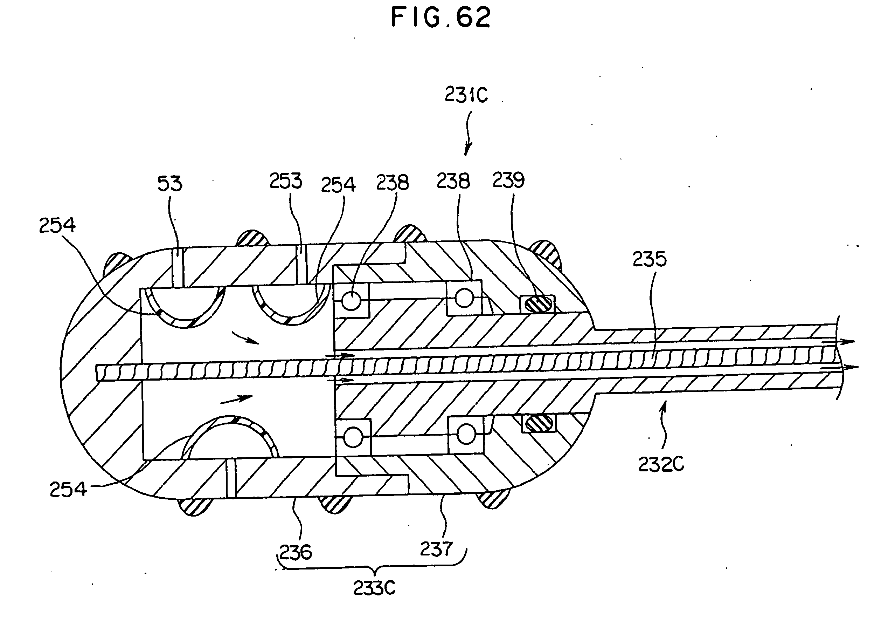

Referring to Figs. 61 and 62, the spiral thrusting unit 231 may have an absorbing hole for absorbing fluid in the gap formed between the inner wall of the body cavity and the exterior container.

Referring to Figs. 61 and 62, a spiral thrusting unit 231C has an absorbing hole 253 for absorbing the space formed between the inner wall of the body cavity and an exterior container 233C at the exterior container 233C.

The exterior container 233C has the absorbing hole 253 from the outer circumferential surface to the inside of the container 236 on the distal-end side. A balloon 254 serving as an elastic watertight film, arranged in the exterior container 233C prevents the influx of the body fluid or the like. Further, the flexible tube 232C has a common function of an absorbing line in addition to the line of the flexible shaft 235. The balloon 254 may not be arranged if the body fluid or the like is discharged out of the body via the absorbing line.

Although not shown, an absorbing device for absorption is connected to the flexible tube 232C. The absorbing device may independently be structured or may be arranged in the spiral driving unit 209.

Thus, the spiral thrusting unit 231C absorbs the space formed between the inner wall of the body cavity and the exterior container 233C, thereby increasing and reducing the friction force by the closely contact property between the inner wall of the body cavity and the exterior container 233C. Thus, the thrust can be adjusted.

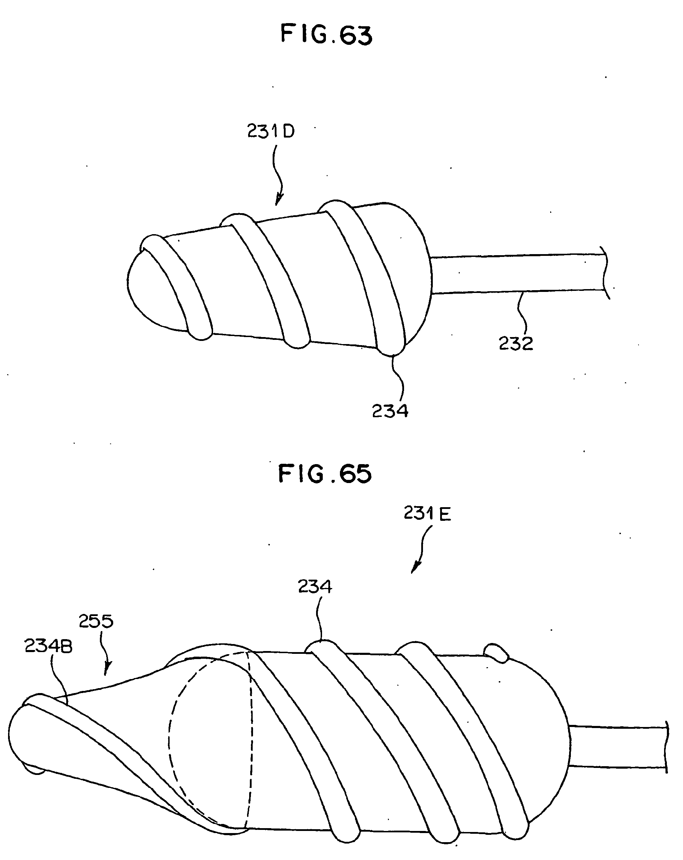

Referring to Fig. 63, the spiral thrusting unit may have the distal end which is taper-shaped for easy insertion in the thin lumen.

As shown in Fig. 63, a spiral thrusting unit 231D has the distal end which is taper-shaped. Consequently, the spiral thrusting unit 231D is easily inserted into the thin tract of the body cavity, and the tract in the body cavity is easily widened by pressing operation. Only the distal end of a spiral thrusting unit 231D may be elastic to easily advance in the tract of the body cavity.

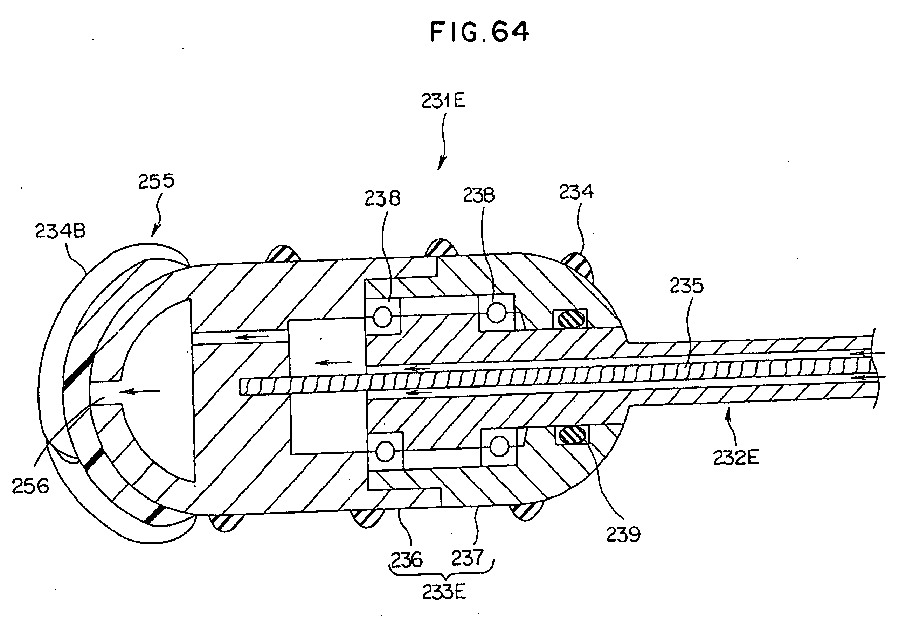

Referring to Figs. 64 to 66, the spiral thrusting unit may have a taper balloon at the distal end of a cylindrical exterior container.

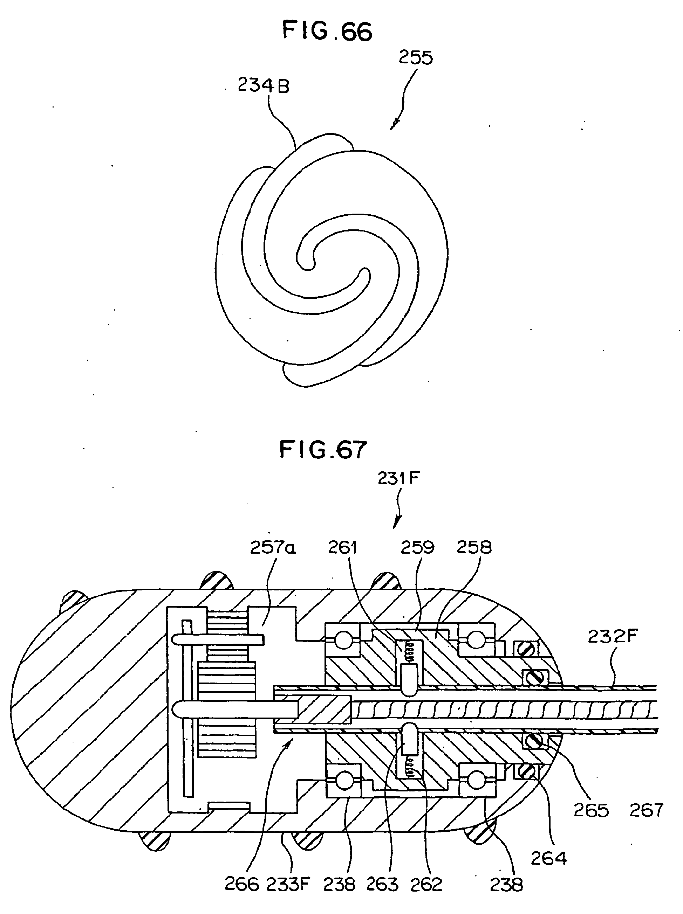

Referring to Figs. 64 to 66, a spiral thrusting unit 231E has a taper balloon 255 at the distal end of a cylindrical exterior container 233E. Referring to Figs. 65 and 66, the taper balloon 255 is expanded.

The exterior container 233E has a through-hole 256 from the outer circumferential surface of the distal end of the container 236 on the distal-end side to the inside thereof so that the air is fed to the taper balloon 255 arranged to the outer circumference of the distal end. The exterior container 233E has a common function of an air feed tube in addition to the tube of the flexible shaft 235. The container 236 on the distal-end side has the inner shape for passage of the air fed from the flexible tube 232, and may not be shaped described as shown in the drawing.

The spiral thrusting unit 231E has the same advantages as those of the spiral thrusting unit 231D. Further, as described above, when the spiral thrusting unit 231E impinges to the bending portion such as the sigmoid colon, the taper balloon 255 may be blown or may be blown and pass through the bent portion.

At the closing portion of the tract in the body cavity, the taper balloon 255 is blown, thereby extending the spiral thrusting unit 231E as compared with the case before blowing the taper balloon 255. By rotation, the spiral thrusting unit 231E easily advances.

The spiral thrusting unit 231E may blow the taper balloon 255 only at the necessary timing. For example, the taper balloon 255 may contract periodically, e.g., every second. Referring to Figs. 67 to 69, the spiral thrusting unit may be detachable to the flexible tube.

Referring to Figs. 67 to 69, a spiral thrusting unit 231F is detachable to a flexible tube 232F. Specifically, the spiral thrusting unit 231F has a planetary gear mechanism 257 for rotating an exterior container 233F therein integrally formed to the spiral thrusting unit 231F. In place of the planetary gear mechanism 257, a rotating mechanism may be arranged.

The spiral thrusting unit 231F has a locking mechanism 258 for pressing and fixing the distal end of the flexible tube 232F at a tube fixing member 259. The locking mechanism 258 has a groove portion 261 facing the inner circumferential surface of the tube fixing member 259. A coil spring 262 embedded into the groove portion 261 has a projection 263 for pressing and fixing the flexible tube 232F. The locking mechanism 258 may use the absorbability of a magnet, in stead of the above-described mechanical structure.

The bearing 238 is arranged between the inner circumferential surface of the exterior container 233F and the tube fixing member 259. The exterior container 233F can be rotated to the tube fixing member 259 by the bearing 238. The interval between the tube fixing member 259 and the inner circumferential surface of the exterior container 233F is watertight by an O ring 264. Further, the interval between the tube fixing member 259 and the flexible tube 232F is watertight by an O ring 265.

The flexible tube 232F that detachably attaches the spiral thrusting unit 231F has, on the distal-end side, a fitting portion 266 for fitting a shaft 257a of the planetary gear mechanism 257 of the spiral thrusting unit 231. In place of the flexible shaft 235, a torque tube 267 is inserted into the flexible tube 232F.

The spiral thrusting unit 231F is detachable to the flexible tube 232F.

Before detachably attaching the spiral thrusting unit 231F to the flexible tube 232F, the channel 222 for inserting the treatment tool of the endoscope 204 is inserted into the flexible tube 232F, thereby projecting the distal end of the tube from the channel opening 222a. Therefore, the spiral thrusting unit 231F is detachably and watertightly attached to the distal end of the flexible tube 232F.

Thus, when the spiral thrusting unit 231F is inserted in the channel 222 for inserting the treatment tool of the endoscope 204 while the spiral thrusting unit 231F is attached to the flexible tube 232F, it is possible to prevent a difficulty that the flexible tube 232F comes into contact with the branch of the channel 222 for inserting the treatment tool and is not inserted into the channel 222 for inserting the treatment tool.

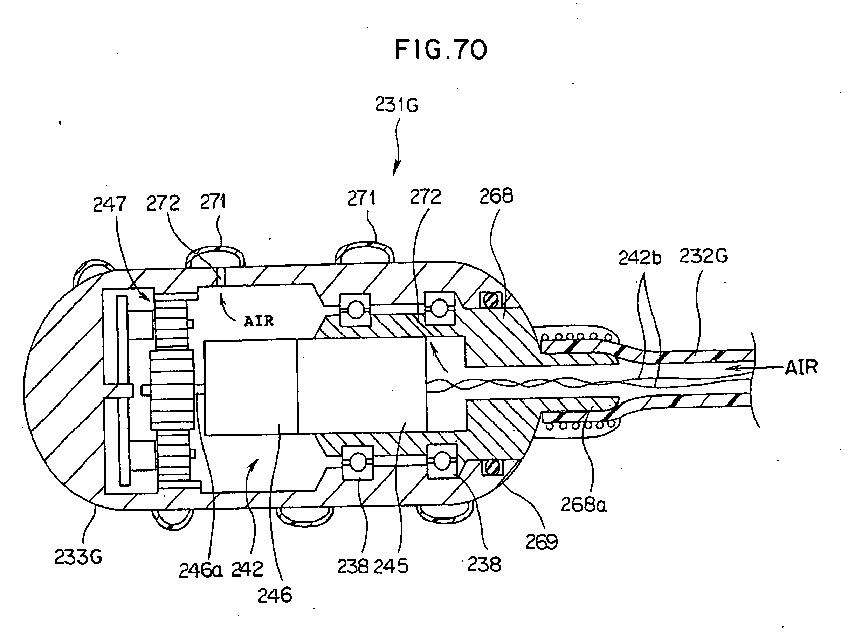

As shown in Fig. 70, the spiral thrusting unit may have an exterior container having therein a motor-unit portion.

Referring to Fig. 70, a spiral thrusting unit 231G has a motor-unit portion 242 in an exterior container 233G integrally formed to the spiral thrusting unit 231G. A motor fixing member 268 fixes and holds the motor-unit portion 242. The output shaft 246a of the motor-unit portion 242 is connected to the planetary gear mechanism 257.

The bearing 238 is arranged between the inner circumferential surface of the exterior container 233G and the motor fixing member 268. The exterior container 233G is rotated to the motor fixing member 268 by the bearing 238. Further, the interval between the inner circumferential surface of the exterior container 233G and the motor fixing member 268 is watertight by an O ring 269.

An attaching portion 268a of the flexible tube 232G is formed on the proximal-end side of the motor fixing member 268. The distal end of the flexible tube 232G is fit into the attaching portion 268a by the adhesion and fixing like a bobbin. A signal line 242b extended from the motor-unit portion 242 is inserted in the flexible tube 232G. The motor-unit portion 242 receives the power and the control signal from the spiral-thrust control device 210 via the signal line 242b and thus is driven.

Further, the outer circumferential surface of the exterior container 233G has a balloon projection 271 containing a balloon serving as the spiral projection. Therefore, the exterior container 233G and the motor fixing member 268 have a through-hole 272 which guides the air fed from the flexible tube 232G to the balloon projection 271.

The balloon projection 271 adjusts the height of the projection depending on the amount of fed air. Thus, the spiral thrusting unit 231G optimizes the thrust in accordance with the change in diameter of the tract in the body cavity.

The spiral thrusting unit 231G absorbs the air so as to prevent a state in which the balloon 254 becomes the obstacle upon pulling-out the inserting portion 211 of the endoscope 204 or upon observing the front portion by the endoscope 204 with the obtained endoscope image, thereby deflating the balloon projection 271.

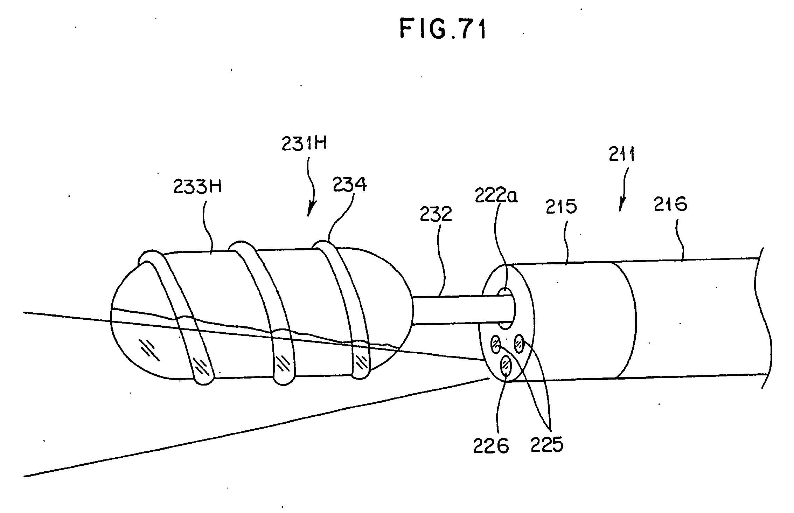

Referring to Fig. 71, the spiral thrusting unit may be partly transparent, as means for ensuring the field of view, so as to prevent a state in which the spiral thrusting unit becomes the obstacle of the range of the field of view for observation of the endoscope 204.

Referring to Fig. 71, a spiral thrusting unit 231H contains an exterior container 233H and a part of the spiral projection 234 having a transparent material. The spiral thrusting unit 231H may have the component of the planetary gear or the like that is partly transparent.

Thus, when the endoscope 204 observes the tract in the body cavity, e.g., digestive tract, the spiral thrusting unit 231H adjusts the angle so that the transparent portion enters the range of the field of view for observation, thereby preventing the state in which the spiral thrusting unit 231H becomes the obstacle of the illumination beam or field of view for observation of the endoscope 204.

The spiral thrusting unit 231 may be structured by removing the portion corresponding to the transparent portion of the spiral thrusting unit 231H and arranging a balloon, as means for ensuing the field of view (not shown), at the removing portion thereof.

In this case, the spiral thrusting unit 231 is cylindrically shaped by blowing the balloon in the spiral thrust. The balloon is deflated in the observation of the endoscope 204. Thus, the spiral thrusting unit 231 does not become the obstacle of the range of the field of view for observation of the endoscope 204.

In the spiral thrusting unit 231, a forceps stand-up function may be arranged to the channel opening 222a of the channel 222 for inserting the treatment tool, as means for ensuring the field of view (not shown) to stand-up the spiral thrusting unit 231 in the observation. Thus, the spiral thrusting unit 231 is out of the range of the field of view for observation.

### (Sixth embodiment)

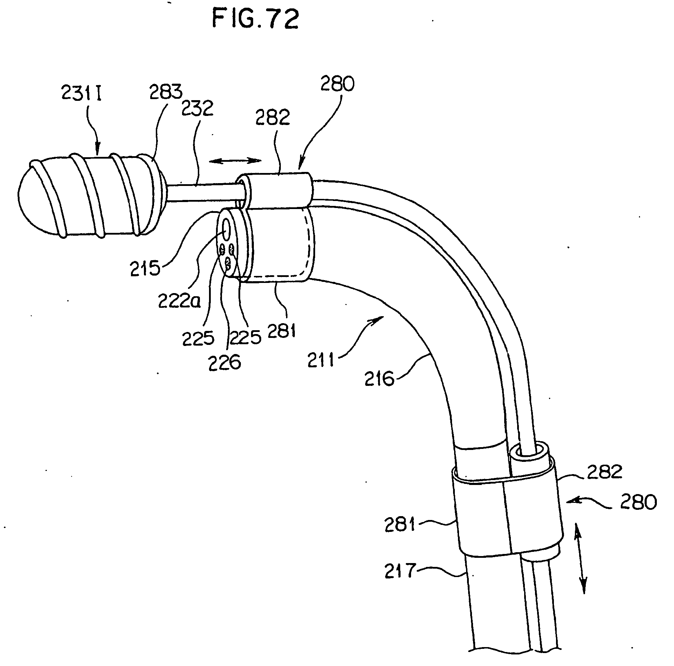

Next, a sixth embodiment of the present invention will be described with reference to Figs. 72 to 81.

According to the fifth embodiment, the spiral thrusting probe 208 is inserted in the channel 222 for inserting the treatment tool of the endoscope 204. However, according to the sixth embodiment, the spiral thrusting probe 208 is attached to a detachable unit along the outer circumference of the endoscope 204. Other structures are the same as those according to the fifth embodiment, a description thereof is omitted, and the same components as those according to the fifth embodiment are designated by the same reference numerals.

Referring to Fig. 72, in an endoscope insertion aiding device according to the sixth embodiment, the spiral thrusting probe 208 is attached to the inserting portion 211 of the endoscope 204 by an attachable/detachable unit 280 serving as a detachably attached unit.

An attachable/detachable unit 280 is ring-shaped like the figure of 8, and comprises: a ring 281 with thick diameter into which the distal-end side of the inserting portion 211 of the endoscope 204 is fit and a ring 282 with fine diameter into which the flexible tube 232 of the spiral thrusting probe 208 is fit.

In the attachable/detachable unit 280, the distal-end side of the inserting portion 211 of the endoscope 204 is fit into the ring 281 with thick diameter to be attached to the inserting portion 211 of the endoscope 204. After that, the flexible tube 232 of the spiral thrusting probe 208 is fit into the ring 282 with fine diameter. Thus, the spiral thrusting probe 208 is freely detachably attached to the distal-end side of the inserting portion 211 of the endoscope 204.

According to the sixth embodiment, two attachable/detachable units 280 are slidably arranged to at least two portions of the distal-end portion 215 of the inserting portion of the endoscope 204 and the flexible portion 217.

Thus, in the spiral thrusting probe 208, the flexible tube 232 advances and returns by the operating portion 212 of the endoscope 204 and thus the flexible tube 232 is slid to the inserting portion 211 of the endoscope 204 and the attachable/detachable unit 280. The spiral thrusting probe 208 is slid forward and backward.

A spiral thrusting unit 231I has a proximal-end side balloon 283 on the proximal-end side thereof.

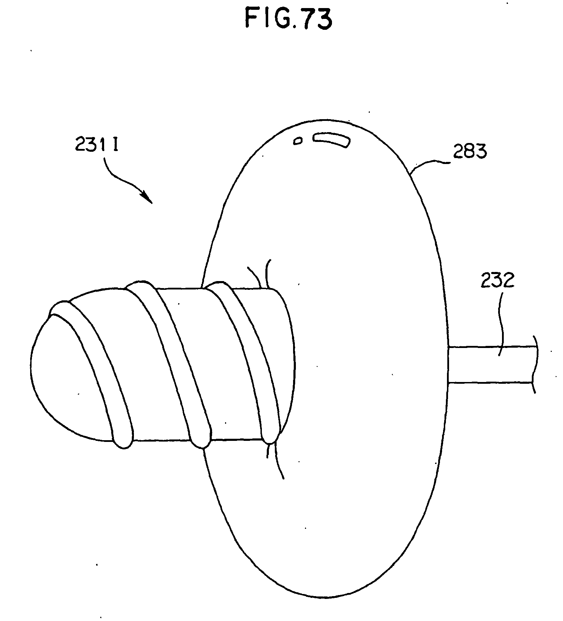

Referring to Fig. 73, the balloon 283 on the proximal-end side is formed to be expanded with the same diameter as that of the tract in the body cavity. Thus, the balloon 283 on the proximal-end side stops the spiral thrusting unit 231I at the position in the tract of the body cavity, as will be described later. The air is fed to the balloon 283 on the proximal-end side from the flexible tube 232.

The endoscope insertion aiding system with the above-described structure is used as described above according to the fifth embodiment. The operator inserts the inserting portion 211 of the endoscope 204 from the anus. In this case, the inserting portion 211 of the endoscope 204 is elongated and flexible. Therefore, the inserting portion 211 is pressed and pulled-out to be inserted in the body cavity.

In the endoscope insertion aiding device, similarly to the fifth embodiment, the spiral driving unit 209 is driven by pressing the driving switch 224 under the control of the spiral-thrust control device 210, thereby thrusting the spiral thrusting unit 231I.

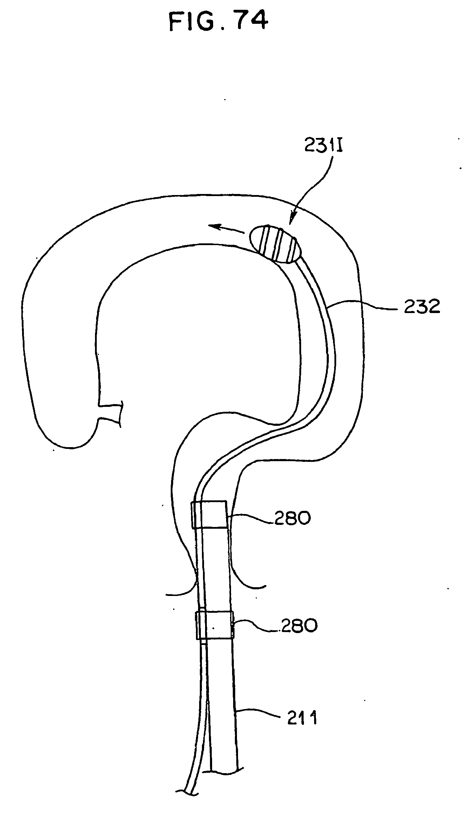

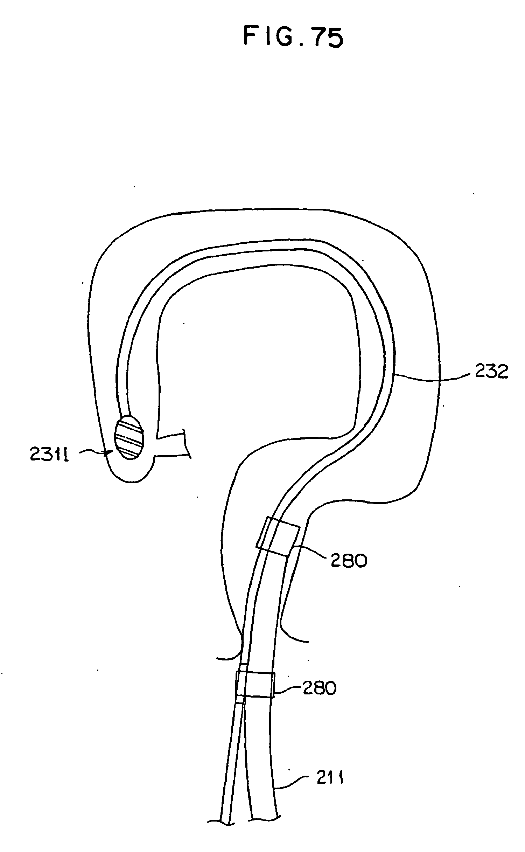

According to the sixth embodiment, referring to Fig. 74, only the spiral thrusting unit 231I thrusts in advance. Referring to Fig. 75, when the spiral thrusting unit 231I reaches the cecum, the balloon 283 on the proximal-end side is blown.

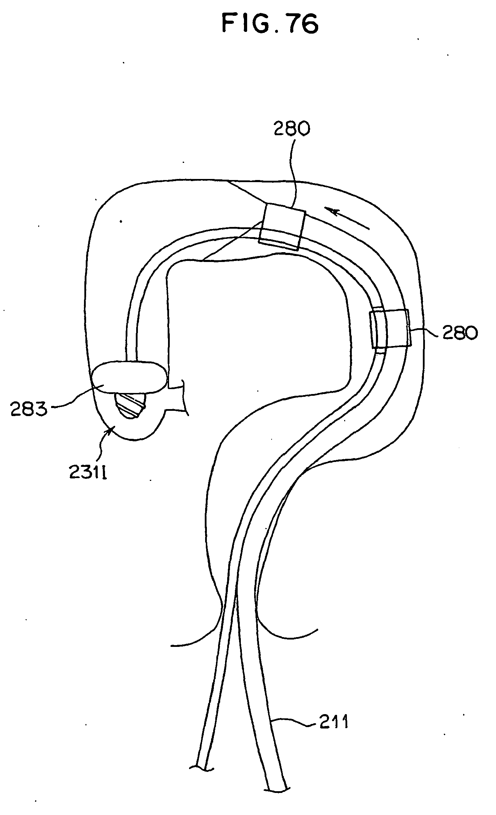

In the spiral thrusting probe 208, the balloon 283 on the proximal-end side is blown with the diameter of lumen of the cecum, thereby stopping the spiral thrusting unit 231I to the cecum. Referring to Fig. 76, the spiral thrusting probe 208 uses the flexible tube 232 as a guide wire, thereby inserting the endoscope 204 to the cecum. In the spiral thrusting probe 208, the endoscope 204 feeds the air into the large intestine before inserting the endoscope 204 so that the spiral thrusting probe 208 is blown to ensure the field of view for observation and then the endoscope 204 may be inserted.

Although not shown, the spiral thrusting probe 208 may have the flexible tube 232 including a rigidity varying function (coil sheath) (not shown). In the spiral thrusting probe 208 in this case, the spiral thrusting unit 231I reaches the cecum and the balloon 283 on the proximal-end side stops the spiral thrusting unit 231I, then, the rigidity of the flexible tube 232 increases to easily insert the endoscope 204. The spiral thrusting probe 208 may properly switch-on/off the rigidity varying function even in the insertion of the spiral thrusting unit 231I and consequently the insertability is improved.

As a result, the endoscope insertion aiding device according to the sixth embodiment has the same advantages as those according to the fifth embodiment. In addition, the attachable/detachable unit 280 is attached to the inserting portion 211 of the endoscope 204, thereby structuring an endoscope without the channel 222 for inserting the treatment tool or a (thin) endoscope with the fine diameter.

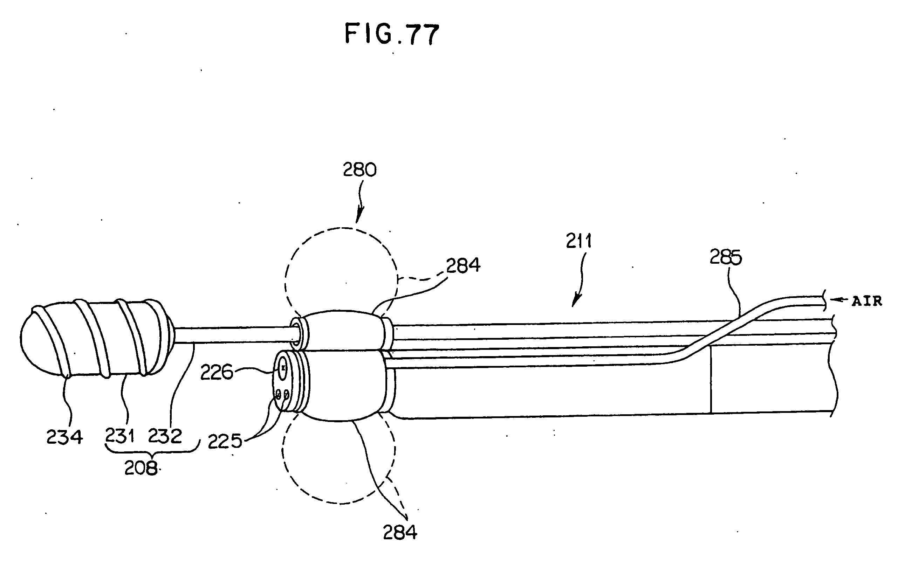

Referring to Fig. 77, the endoscope insertion aiding device may have the attachable/detachable unit having a balloon.

Referring to Fig. 77, the attachable/detachable unit 280 has two balloons 284 on the side of the ring 281 with large diameter and the side of the ring 282 with small diameter. An air feed tube 285 is extended to the attachable/detachable unit 280 to feed the air to the balloons 284. The air feed tube 285 is connected to a compressor (not shown).

The endoscope insertion aiding system with the above-described structure is used as described above according to the fifth embodiment. The operator inserts the inserting portion 211 of the endoscope 204 from the anus of the patient. In this case, since the inserting port 221 of the treatment tool of the endoscope 204 is elongated and flexible, the inserting portion 211 is pressed and pulled-out to be inserted in the body cavity.

In the endoscope insertion aiding device, first, the balloons 284 of the attachable/detachable unit 280 is blown, thereby fixing the distal-end portion 215 of the inserting portion of the endoscope 204. After that, the spiral thrusting unit 231 is thrust.

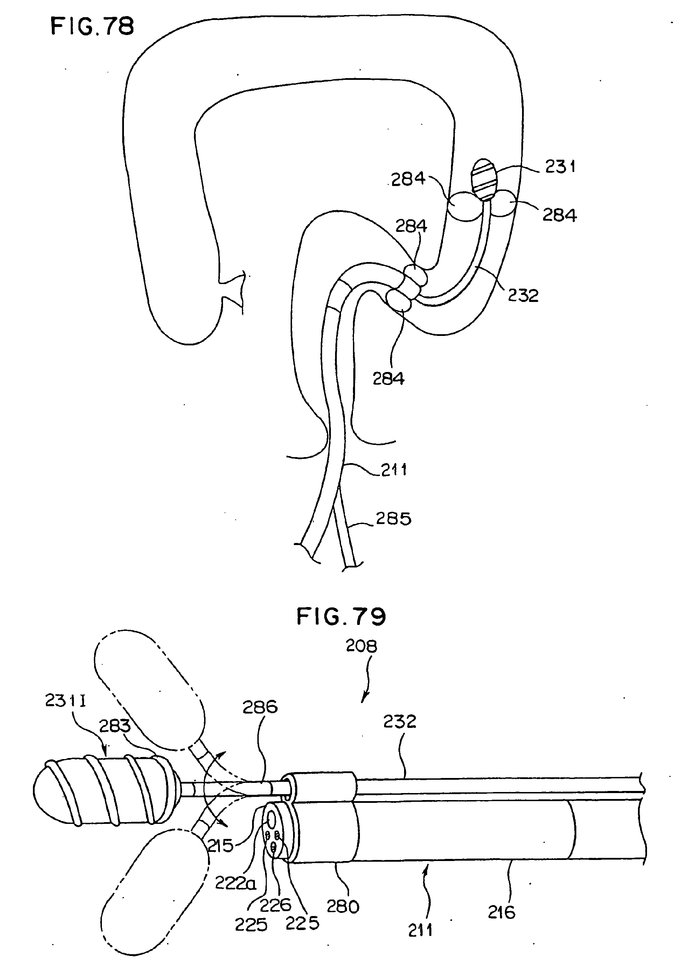

Referring to Fig. 78, the endoscope insertion aiding device blows the balloon 234 of the spiral thrusting unit 231 as descried with reference to Fig. 73 after advancing the spiral thrusting unit 231. Next, the endoscope insertion aiding device deflates the balloon 284 of the endoscope 204, thereby inserting the endoscope 204 by using the spiral thrusting unit 231 as the guide. The above operation repeats and thus the distal-end portion 215 of the inserting portion of the endoscope 204 reaches the cecum in the endoscope insertion aiding device 203.

In the endoscope insertion aiding device 203, the inserting portion 211 of the endoscope 204 is inserted into the tract of the body cavity, like the motion of an inchworm.

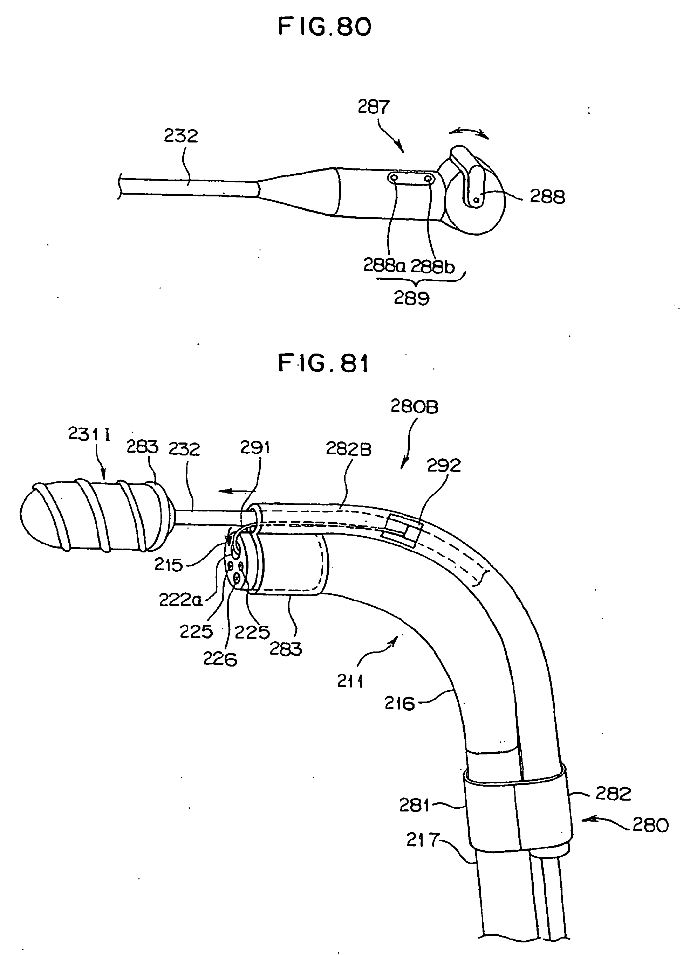

Referring to Figs. 79 and 80, the endoscope insertion aiding device may have a bending portion which is freely bendable to the flexible tube 232.

Referring to Fig. 79, the spiral thrusting probe 208 has a probe bending portion 286 which is freely bendable to the flexible tube 232. The probe bending portion 286 is arranged to the proximal-end portion in proximity to the spiral thrusting unit 231 for the tracing operation.

Referring to Fig. 80, the spiral thrusting probe 208 has a probe operating portion 287 on the proximal-end side thereof. The probe operating portion 287 has a motor-unit portion forming the spiral driving unit. The probe operating portion 287 comprises: a bending operation knob 288 for bending the probe bending portion 286; and a switch portion 289 including an on/off switch 289a for switching on/off the rotation of the spiral thrusting unit 231 and a rotational-direction and speed adjusting switch 289b for adjusting the direction of rotation of the spiral thrusting unit 231 and the rotating speed.

Thus, the endoscope insertion aiding device actively directs the spiral thrusting unit 231 to the running direction of the lumen. The easiness of advancing the spiral thrusting unit 231 is improved. Upon observing the digestive tract by the endoscope 204, the spiral thrusting unit 231 is arranged out of the field of view for observation of the endoscope 204 in the endoscope insertion aiding device. Thus, the body cavity is easily observed by bending the probe bending portion 286.

Referring to Fig. 81, the endoscope insertion aiding device may have an advance and retreat mechanism for advancing and retreating the flexible tube 232.

Referring to Fig. 81, in the endoscope insertion aiding device, a pulling string 291 passing through the channel 222 for inserting the treatment tool is connected to the flexible tube 232 via a string connecting portion 292. An attachable/detachable unit 280B has a ring 282B with small diameter which is extended throughout the entire inserting portion 211 of the endoscope 204. The flexible tube 232 is held and fixed to the inserting portion 211.

Consequently, in the endoscope insertion aiding device, the pulling string 291 is pulled from the hand side of the endoscope 204, thereby pulling the flexible tube 232 forward. The spiral thrusting unit 231 advances. The flexible tube 232 is pulled backward from the hand side, thereby retreating the spiral thrusting unit 231.

Therefore, the endoscope insertion aiding device is improved in the problem that the "pressing" operation is not transmitted due to the long flexible tube 232.

### (Seventh embodiment)

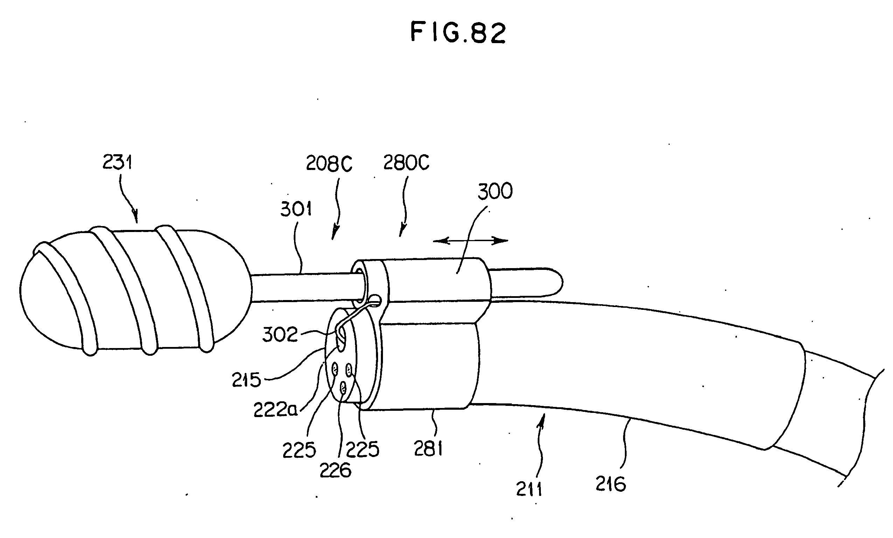

Next, a seventh embodiment of the present invention will be described with reference to Figs. 82 to 87.

According to the seventh embodiment, an advance and retreat mechanism is arranged to the attachable/detachable unit 280 according to the sixth embodiment. Other structures are the same as those according to the fifth embodiment, a description thereof is omitted, and the same reference numerals denote the same components.

Referring to Fig. 82, in an endoscope insertion aiding device according to the seventh embodiment, an attachable/detachable unit 280C for attaching a spiral thrusting probe 208C to the distal-end portion 215 of the inserting portion of the endoscope 204 has an advance and retreat mechanism unit 300.

The spiral thrusting probe 208C has a flexible tube 301 which is short. The spiral thrusting unit 231 has the motor-unit portion 242 similarly to the spiral thrusting unit 231G described with reference to Fig. 70. The flexible tube 301 optimizes its rigidity and elasticity so that the flexible tube 301 promptly becomes straight when the force is not applied though the elasticity is strong and the flexible tube 301 traces the running of the lumen.

Power and a control signal supplied to the spiral thrusting probe 208C are fed via a cable 302 passing through the channel 222 for inserting the treatment tool of the endoscope 204. The cable 302 is connected to the spiral-thrust control device 210 on the hand side. The cable 302 may be along the outside of the endoscope 204 without passing through the channel 222 for inserting the treatment tool.

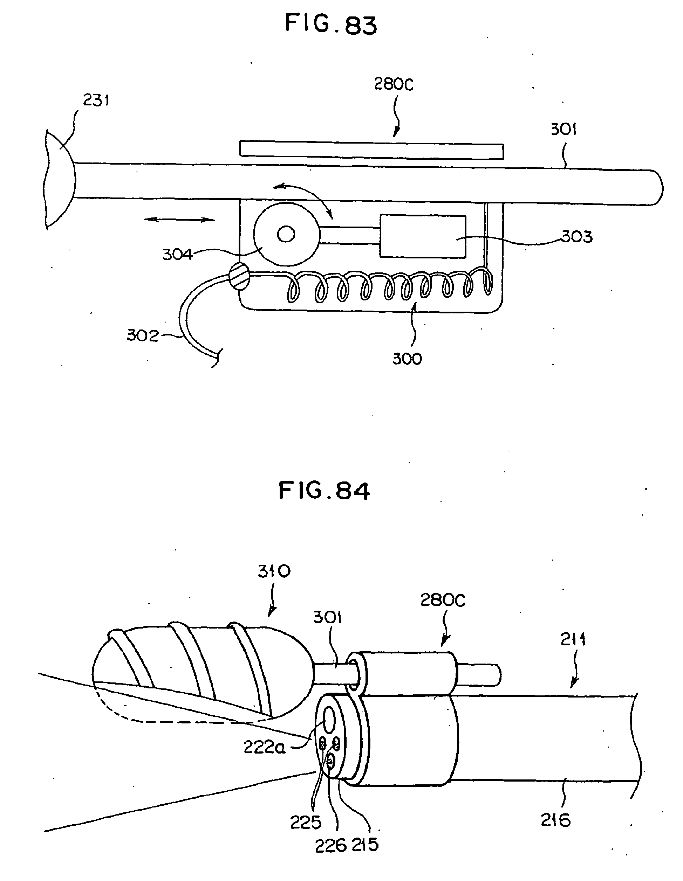

Referring to Fig. 83, the advance and retreat mechanism unit 300 comprises: a motor 303 which generates driving force for advancing and retreating the flexible tube 301; an umbrella gear (not shown) for reducing the driving force from the motor 303; and a roller 304 which transmits the rotation from the umbrella gear to the flexible tube 301 to advance and retreat the flexible tube 301. The advance and retreat mechanism unit 300 may have a rotating motor and mechanism of the spiral thrusting unit 231.

The endoscope insertion aiding system with the above-described structure is used as described according to the fifth embodiment. The operator inserts the inserting portion 211 of the endoscope 204 from the anus of the patient. In this case, the inserting portion 211 of the endoscope 204 is elongated and flexible and therefore the inserting portion 211 is pressed and pulled to be inserted in the body cavity.

In the endoscope insertion aiding device, similarly to the fifth embodiment, the spiral driving unit 209 is driven by pressing the driving switch 224 under the control of the spiral-thrust control device 210, thereby thrusting the spiral thrusting unit 231. In this case, in the endoscope insertion aiding device 203, the advance and retreat mechanism unit 300 is driven, thereby advancing the flexible tube 301.

Alternatively, in the endoscope insertion aiding device 203, when the endoscope image is obtained and the endoscope 204 observes the front portion or the inserting portion 211 of the endoscope 204 is pulled out, the advance and retreat mechanism unit 300 is driven to the predetermined position for preventing a state in which the spiral thrusting unit 231 becomes the obstacle to advance and retreat the flexible tube 301.

As a result, the endoscope insertion aiding device has the same advantages as those according to the sixth embodiment. In addition, since the spiral thrusting probe 208 is short, the endoscope insertion aiding device is reduced in size to be easily handled.

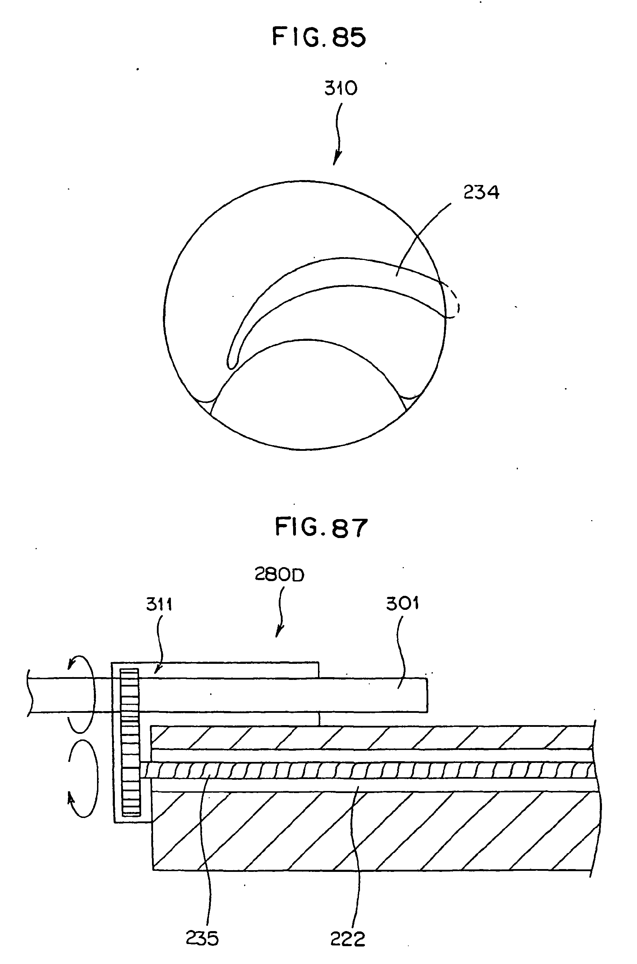

Referring to Figs. 84 and 85, the spiral thrusting unit may partly be removed, as means for ensuring the field of view, so as to prevent the state in which the endoscope insertion aiding device becomes the obstacle of the range of the field of view of the endoscope 204.

Referring to Fig. 84, a spiral thrusting unit 310 is structured by removing a part thereof, as the means for ensuring the field of view, so as to prevent the state in which the spiral thrusting unit 310 becomes the obstacle of the range of the field of view for observation.

Thus, referring to Fig. 85, the spiral thrusting unit 310 does not enter the range of the field of view for observation of the endoscope 204 as much as possible. Further, in the endoscope observation, the angle of the spiral thrusting unit 310 is adjusted to be a predetermined one.

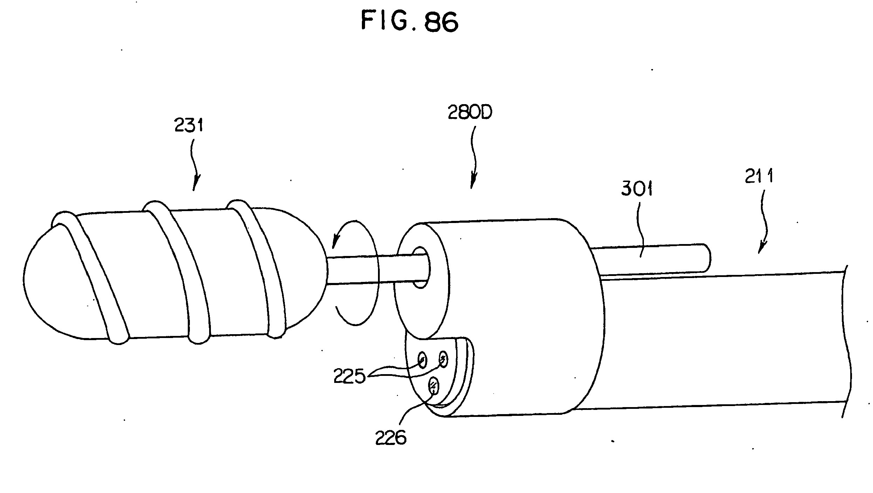

Referring to Figs. 86 and 87, the attachable/detachable unit may not have the motor unit.

Referring to Figs. 86 and 87, an attachable/detachable unit 280D transmits the driving force, as the rotation, transmitted from a torque tube by using a flexible shaft 235 passing through the channel 222 for inserting the treatment tool of the endoscope 204 or a gear 311.

Consequently, the endoscope insertion aiding device has the simple structure and the assemblity is improved.

### (Eighth embodiment)

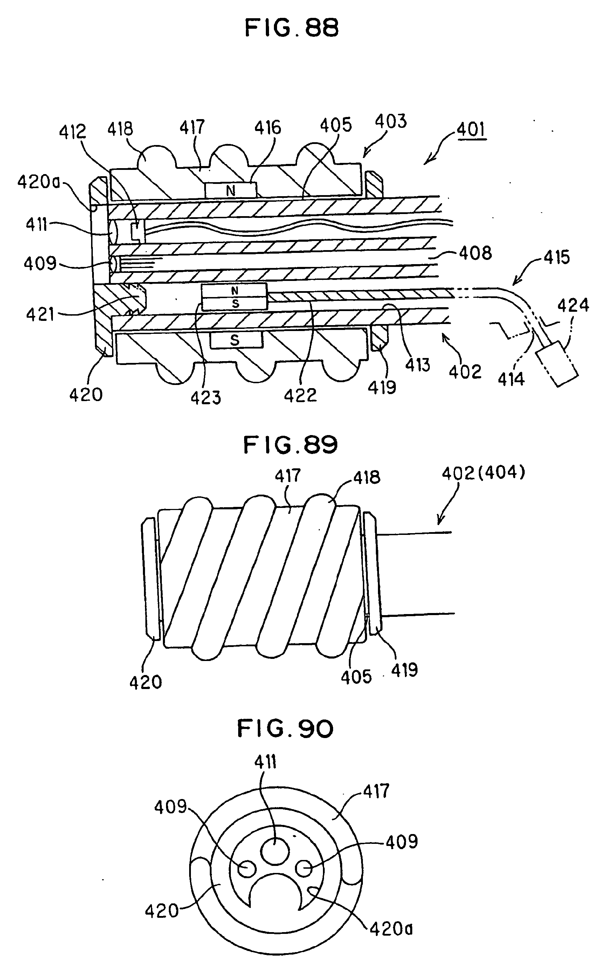

Next, an eighth embodiment of the present invention will be described with reference to Figs. 88 to 92.

Referring to Fig. 88, an endoscope device 401 comprises: an endoscope 402; and an endoscope insertion aiding device (or advancing device for the endoscope in the examinee) 403 which is freely detachably attached to the distal end of the endoscope 402 and smoothly guides or inserts the endoscope 402 into the examinee such as the body cavity.

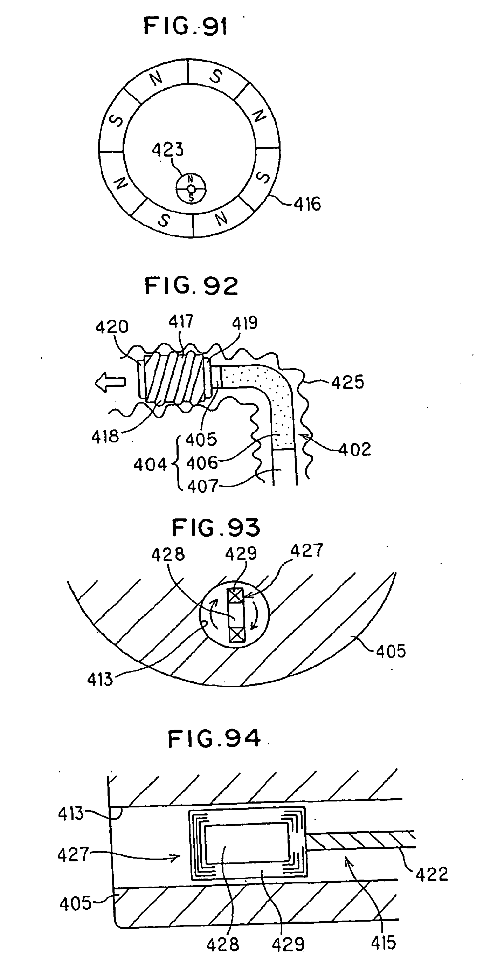

The endoscope 402 has an elongated inserting portion 404 that is inserted in the body cavity. The proximal-end side of the endoscope 402 has an operating portion (not shown). The inserting portion 404 comprises: a rigid distal-end portion 405 arranged to the distal end of the inserting portion 404; a bendable bending portion 406 arranged to the proximal end of the distal-end portion 405; and a long soft portion 407 reaching the front end of the operating portion from the proximal end of the bending portion 406 (refer to Fig. 92).

The user operates a bending operation knob (not shown) arranged to the operating portion, thereby bending the bending portion 406 in the desired direction.

A light guide 408 for transmitting the illumination beam is inserted into the inserting portion 404. The illumination beam is supplied from a light source device (not shown) to an incident end of the illumination beam serving as the proximal end of the light guide 408. The distal-end surface of the light guide 408 becomes an emitting distal-end surface of the illumination beam. The illumination beam transmitted by the light guide 408 passes through an illuminating lens 409 from the output end-surface is outputted to the frontward, and illuminates the body cavity on the frontward.

Referring to Fig. 88, the distal-end portion 405 of the inserting portion 404 has an observing window (image pickup window) adjacent to an illuminating window having the illuminating lens 409. An objective lens 411 attached to the observing window forms an optical image of the illuminated body cavity. A charge-coupled device (hereinafter, abbreviated to a CCD) 412, serving as an image pickup element, is arranged to the image forming position, and the CCD 412 photoelectrically converts the formed optical image.

The CCD 412 is connected to a signal processing device (not shown) via a signal line. The signal processing converts an output signal from the CCD 412 into a video signal, the image picked-up by the CCD 412 displays on a display surface of a monitor.

The inserting portion 404 of the endoscope 402 has the channel 413 into which the treatment tool such as forceps can be inserted. The proximal-end side of the channel 413 is branched near the proximal end of the inserting portion 404. One branched-portion is communicated with an inserting port 414 of the treatment tool and another reaches an absorbing cap connected to an absorbing device (not shown).

From the inserting port 414 of the treatment tool, a rotating member 417 and a magnetic field applying member 415 independent thereof, which will be described later, are inserted. The rotating member 417 and the magnetic field applying member 415 constitute the endoscope insertion aiding device 403.

The rotating member 417 having a magnet 416 is freely rotatably attached to the outer circumferential surface of the distal-end portion 405 of the inserting portion 404.

The rotating member 417 is cylindrical. Referring to Fig. 89, the rotating member 417 has a projected portion 418 that is spiral-shaped on the outer circumferential surface of the rotating member 417. The rotation together with the fixing member results in obtaining the thrust by the projected portion 418. The projected portion 418 may be formed by spirally attaching a hollow tube or by spirally attaching a solid string. Or, the number of spiral lines may be one, two, or three.

When attaching the rotating member 417 to the outer circumferential surface of the distal-end portion 405, a ring-shaped fixing member 419 fit and fixed to the outer circumferential surface near the proximal end of the distal-end portion 405 and a disc-shaped fixing member 420 having a hollow opening 420a fixed to the distal-end surface are used. The fixing member 420 has a projected portion 421 attached to the opening on the distal end of the channel 413 by compression.

That is, the fixing members 419 and 420 are attached to the distal-end portion 405 at both sides of the rotating member 417, thereby freely rotatably attaching the rotating member 417 to the distal-end portion 405. In this case, referring to Fig. 90, the fixing member 420 has the opening 420a which ensures the field of view at the position facing the distal-end surface of the endoscope 402 so as to prevent the illuminating window and the observing window from shielding.

The ring-shaped magnet 416 is fixed in the center of the inner circumferential surface of the rotating member 417 in the longitudinal direction. Referring to Fig. 91, the magnet 416 is energized such that the N and S magnetic poles are alternately arranged in the circumferential direction.

The magnetic field applying member 415 inserted in the channel 413 has a magnet 423 at the distal end of a flexible shaft 422 for transmitting the rotating force. The proximal end of the flexible shaft 422 is attached to a rotating shaft of a motor 424. The motor 424 rotates, thereby rotating the magnet 423 at the distal end of the flexible shaft 422.

Referring to Fig. 91, the magnet 423 has the N and S magnetic poles in the circumferential or diameter direction. The rotating magnet system, thus, enables rotating the rotating member 417.

That is, in the ring-shaped magnet 416 alternately having the N and S magnetic poles, the stick-shaped magnet 423 having the poles in the diameter direction is rotated, thereby rotating the ring-shaped magnet 416 on the outer-circumference side due to the attraction and repulsion between the magnets 416 and 423.

According to an eighth embodiment, the endoscope 402 is a normal endoscope having the channel 413 and therefore the endoscope 402 has a watertight structure in which the cleaning and sterilization are possible.

The rotating member 417 is constituted of a resin member or the like for cleaning and sterilization, the resin member having the ring-shaped magnet 416. The fixing members 419 and 420 are also constituted of a resin member for cleaning and sterilization.

The magnetic field applying member 415 has the simple structure and therefore is easily structured to be watertight for cleaning and sterilization.

According to the eighth embodiment, as described above, the rotating member 417 freely rotatably arranged onto the outer circumferential surface of the distal-end portion 405 is arranged separately from the magnetic field applying member 415 for rotating the magnet 416 arranged to the rotating member 417, the magnetic field being arranged in the channel 413 of the endoscope 402. Thus, the diameter of the distal-end portion 405 is not excessively increased and the distal-end portion 405 can be applied to the endoscope 402 having a channel 413. The separating structure of the rotating member 417 and the magnetic field applying member 415 results in the individual simple structures in which it is easily watertight.

The operation with the above-described structure will be described with reference to Fig. 92 according to the eighth embodiment. First, the fixing member 419 is attached near the proximal end on the outer circumferential surface of the distal-end portion 405 of the inserting portion 404 in the endoscope 402. Then, the rotating member 417 is fit to the outer circumferential surface of the distal-end portion 405. After that, the projected portion 421 of the fixing member 420 is pressed and entered in the opening of the distal end of the channel 413, thereby attaching the fixing member 420. Thus, the user can attach the rotating member 417 freely rotatably to the outer circumferential surface of the distal-end portion 405.

Referring to Fig. 88, the distal end of the magnetic field applying member 415 is inserted from the inserting port 414 of the treatment tool. The magnet 423 arranged to the distal end of the magnetic field applying member 415 is set to the position facing the magnet 416 of the rotating member 417 near the inner circumference thereof.

The graduations are arranged to the proximal end of the flexible shaft 422. A mark or the like is put on the position of the graduations in the case of presetting the magnet 423 at the position facing the central portion of the magnet 416 on the inner circumference (in the longitudinal direction). At the mark position, the proximal end of the flexible shaft 422 may be freely rotatably fixed to the inserting port 414 of the treatment tool.

The inserting portion 404 of the endoscope 402 having the rotating member 417 is inserted in the body cavity. The operator of the endoscope examination inserts the distal-end side of the inserting portion 404 from the anus for example.

The operator switches-on a switch (not shown) for driving the motor 424 of the magnetic field applying member 415, thereby rotating the motor 424. The rotation of the motor 424 rotates the flexible shaft 422 and the magnet 423 at the distal end thereof. The rotating magnetic field of the magnet 423 exerts the rotating force on the ring-shaped magnet 416 arranged on the outer-circumference side. Then, the rotating member 417 rotates together with the magnet 416.

The rotating member 417 has the spiral projected portion 418 on the outer circumferential surface thereof. Referring to Fig. 92, the projected portion 418 rotates, thereby being engaged with the inner wall in contact with the projected portion 418, specifically, the inner-wall surface having folds (concaved and convexed) of the large intestine 425. The thrust is exerted on the rotating member 417. That is, the rotation of screw acts such that the screw is screwed to the deep portion of a member to which the screw is to be attached.

The rotation of the rotating member 417 exerts the thrust on the rotating member 417. The rotation of the rotating member 417 smoothly thrusts or guides the distal-end portion 405 freely rotatably attached to the deep portion of the large intestine 425.

The eighth embodiment has the following advantages.

With the above-described structure, the distal-end portion 405 has, on the outer-circumference side, the cylindrical-shaped rotating member having the magnet 416. The magnetic field applying member 415 for magnetically rotating the rotating member in the non-contact state is arranged in the channel 413. Therefore, the excessive increase in outer diameter of the distal-end portion 405 is prevented and the distal-end portion 405 is smoothly thrust.

That is, the cylindrical rotating member 417 having the magnet 416 is attached to the outer circumferential surface of the distal-end portion 405, and the magnetic field applying member 415 is arranged in the channel 413. Thus, the rotating member 417 is magnetically rotated in the non-contact state. The rotating member 417 and the magnetic field applying member 415 are independently formed and therefore the individual structures are simple and easily watertight.

The endoscope 402 is preset to be watertight. Further, the rotating member 417 has no problem regarding the contact state with the liquid. The rotating member 417 is easily detached or attached. With the above-described structure, the rotating member 417 has a property to be highly cleaned and so is surely cleaned and sterilized.

In the structure according to the eighth embodiment, the rotating member 417 can be attached to the existing endoscope 402. The function of the endoscope 402 except for those of the channel 413 is used without modification and therefore the endoscope 402 is smoothly thrust by using its bending function.

A first modification will be described with reference to Figs. 93 and 94. Fig. 93 is a lateral sectional view showing the periphery of the channel 413 in the distal-end portion 405 (of the endoscope 402). Fig. 94 is a longitudinal sectional view showing the periphery of an electromagnet 427 arranged in the channel 413.

According to the eighth embodiment, the stick magnet 423, as the magnetic field applying member 415, is attached to the distal end of the flexible shaft 422. According to the first modification, the electromagnet 427 is attached to the distal end of the flexible shaft 422 as shown in Figs. 93 and 94.

At the distal end of the flexible shaft 422, the electromagnet 427 is formed by arranging a coil 429 to an iron core 428. A signal line connected to both ends of the coil 429 is inserted in the hollow portion of the flexible shaft 422, and the proximal end of the signal line is connected to a DC power supply such as a battery.

Similarly to the eighth embodiment, the motor 424 rotates the flexible shaft 422, thereby rotating the electromagnet 427 together with the flexible shaft 422.

The rotation of the electromagnet 427 rotates the direction of the magnetic field. Similarly to the case of rotating the magnet 423, the rotation of the electromagnet generates the force to rotate the magnet 416 arranged on the side of the outer circumference.

The electromagnet 427 may have a ferromagnetic member such as iron in the center of the coil 429. In this case, the magnetic field generated by the electromagnet 427 can be made strong and the magnet 416 is certainly rotated. According to the first modification, the same advantages as those according to the eighth embodiment are obtained.

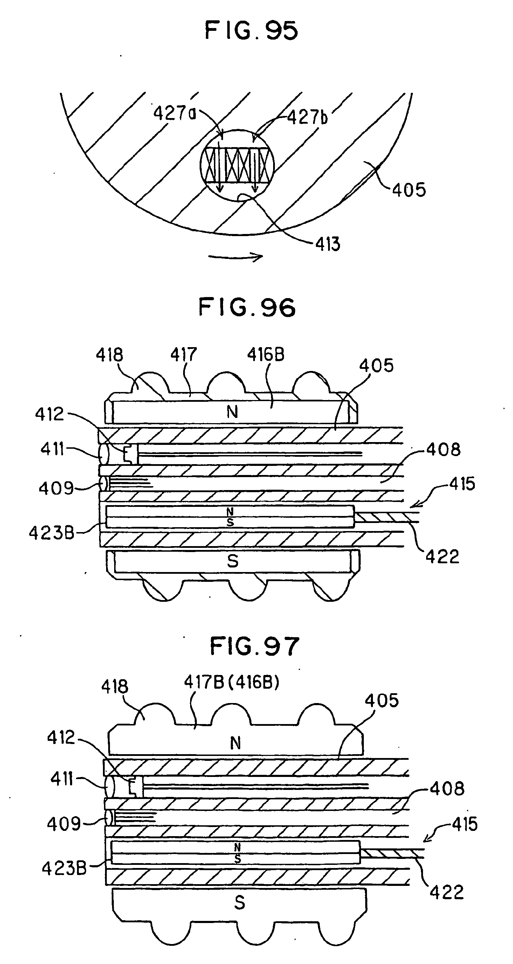

Fig. 95 shows a second modification. According to the second modification, a value of current flowing to electromagnets 427a and 427b arranged in parallel therewith in the channel 413 is changed, thereby applying the magnetic field for rotating the magnet 416. Referring to Fig. 95, for example, the value of current flowing to the two electromagnets 427a and 427b arranged adjacently thereto is changed, thereby operating the magnetic field for rotating the magnet 416 arranged on the side of the outer circumference. The direction of current may be changed.

According to the second modification, the rotation of the motor 424 is unnecessary. According to the second modification, there is a merit that the magnetic field applying member 415 does not need to be rotated. Except for this, the same advantages as those according to the eighth embodiment are obtained.

Fig. 96 shows a third modification. According to the third modification, a magnet 416B is formed by increasing the size of the magnet 416 arranged in the rotating member 417. Further, a magnet 423B is formed by increasing the size of the magnet 423 freely rotatably arranged in the channel 413 for treatment tool.

That is, the ring-shaped magnet 416B is used with the length approximate to the entire length of the rotating member 417 in the longitudinal direction. The magnet 423B has the similar length.

According to the third modification, the fixing members 419 and 420 are not used. That is, the rotating member 417 has the inner diameter to fit the rotating member 417 into the outer circumferential surface of the distal-end portion 405 so as to freely rotate the rotating member 417 on the outer circumferential surface of the distal-end portion 405. In this case, the rotating member 417 might be moved in the longitudinal direction thereof from the distal-end portion 405. However, since the magnet 423B is arranged on the side of the inner circumferential surface, the magnetic force between the magnet 416B and the magnet 423B regulates the movement in the longitudinal direction.

According to the third modification, the rotating force is improved. Advantageously, the rotating member 417 is freely rotatably fixed to the distal-end portion 405 without the mechanical restrictions of the fixing members 419 and 420.

According to the third modification, the structure is simple and the magnet 423B is rotated, thereby rotating the rotating member 417 with the large force. Further, the rotating member 417 is easily attachable and detachable to and from the distal-end portion 405 without the fixing members 419 and 420.

Fig. 97 shows a fourth modification. According to the fourth modification, the entire rotating member 417 according to the third modification is substituted by a magnet 416B. According to the fourth modification, the rotating force is improved. Except for this, the same advantages as those according to the third modification are obtained.

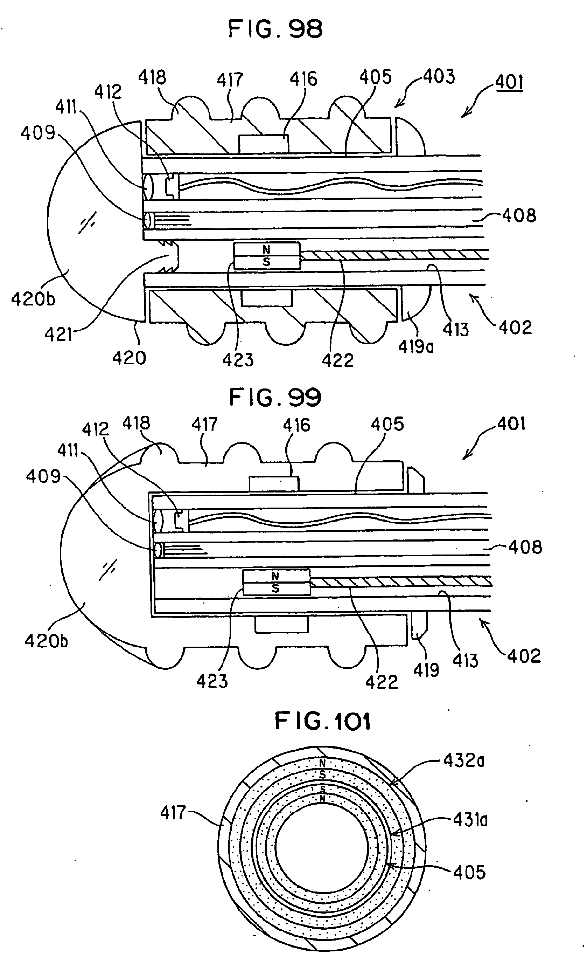

Fig. 98 shows a fifth modification. According to the fifth modification, the fixing member 420 at the distal end according to the eighth embodiment is substituted by a transparent member, and a semi-spherical portion 420b which is formed by semi-spherically shaping the distal-end side of the fixing member 420 is arranged. According to the fifth modification, the observation of the endoscope 402 is ensured. Further, the distal-end side is semi-spherical, thereby ensuring the smooth contact with the inner wall in the body cavity. Further, if the fixing member 419 is substituted by a spherical member 419a toward the rear side, the endoscope 402 is smoothly pulled out.

Fig. 99 shows a sixth modification. According to the sixth modification, the projected portion 421 is removed from the fixing member 420 according to the fifth embodiment, and the fixing member 420 is integrated to the rotating member 417 to be rotated (together with the rotating member 417). The rotating member 417 is formed by a transparent member, and the spiral projected portion 418 on the outer circumferential surface of the rotating member 417 is arranged up to the distal-end side.

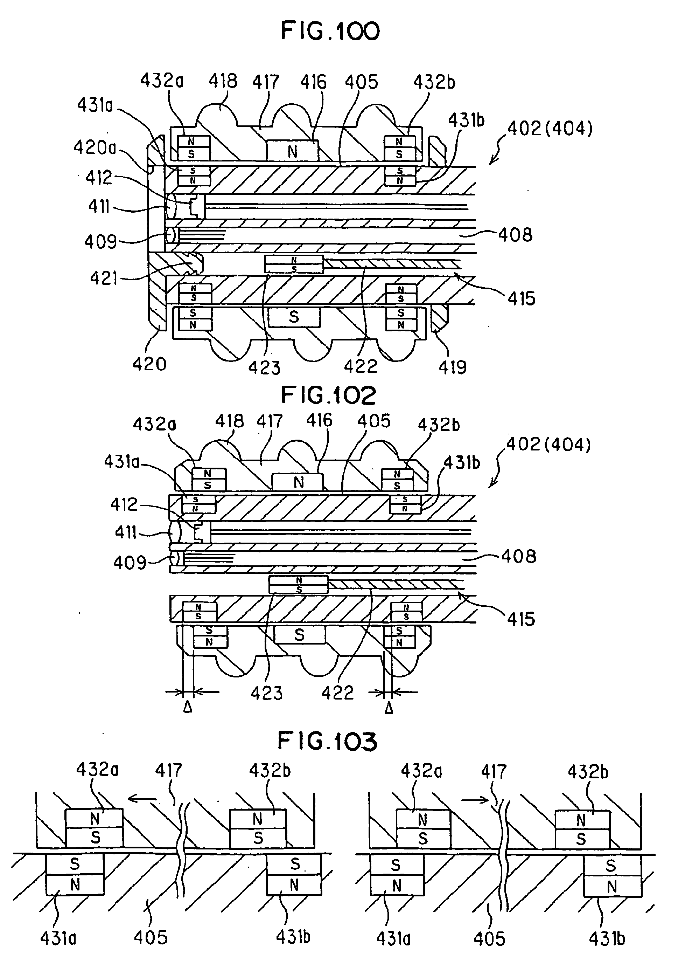

According to the sixth modification, the thrust is improved. Except for this, the same advantages according to the fifth modification are obtained. Figs. 100 and 101 show a seventh modification. According to the seventh modification, the central axis for rotation is not deviated from the central axis of the endoscope 402 according to the eighth embodiment by arranging the magnetic shaft bearing. Specifically, ring-shaped concaved portions are arranged at the positions on the outer circumferential surface near the distal end and the proximal end of the distal-end portion 405 of the endoscope 402, and ring magnets 431a and 431b are attached to the concaved portions.

On the side of the rotating member 417, ring-shaped concaved portions are arranged on the inner circumferential surface constituting both distal- and proximal-end sides of the magnet 416 such that the rotating member 417 faces the magnets 431a and 431b, and ring magnets 432a and 432b are attached respectively.

The magnets 431a and 431b in this case have the magnetic poles different between the inside and the outside in the radial direction as shown in Fig. 101. Specifically, the inside is the N pole and the outside is the S pole. Referring to Fig. 101, the magnets 432a and 432b have magnetic poles different between the inside of the outside in the radial direction so that the force of repulsion acts against the magnets 431a and 431b. Specifically, the inside is the S pole and the outside is the N pole.

The force of repulsion acts on the magnets 431a and 432a which face each other on the side of the distal end. The force of repulsion acts on the magnets 431b and 432b which face each other on the side of the proximal end. The rotating member 417 is held, floating from the outer circumferential surface of the distal-end portion 405. Thus, the rotating member 417 is rotated in the non-contact state with the endoscope 402 and therefore the rotating efficiency is improved.

Figs. 102 and 103 show an eighth modification.

According to the eighth modification, referring to Fig. 102, the facing magnets 431a and 431b are deviated from the facing magnets 432a and 432b in the longitudinal direction of the distal-end portion 405 in the structure shown in Fig. 100. Specifically, the distance between the magnets 431a and 431b arranged on the side of the distal-end portion 405 of the endoscope 402 is larger than the distance between the magnets 432a and 432b arranged on the side of the rotating member 417. When the user attaches the rotating member 417 freely rotatably on the outer circumferential surface of the distal-end portion 405, referring to Fig. 102, the magnets 432a and 432b face each other, deviated to the inner positions from the magnets 431a and 431b of which distance is set larger therebetween (specifically, deviated by Δ).

With the above-described structure, the operation shown in Fig. 103 is obtained.

For example, referring to Fig. 103 on the left side, the external force for movement at the rotating member 417 side acts to the distal-end side. If the rotating member is deviated to the distal-end side as shown by an arrow in this case, the magnets 431a and 432a facing each other on the distal-end side act the higher magnetic force of repulsion (due to the close state of deviation). As shown in Fig. 103 on the right side, the magnetic force of repulsion returns the rotating member 417 to the state before deviation. When the rotating member 417 moves on the proximal-end side, the magnetic force of repulsion acts similarly.

Therefore, the fixing members 419 and 420 in the structure shown in Fig. 100 are not necessary.

According to the eighth modification, the rotating member 417 is freely rotatably held by the simple structure without the fixing members 419 and 420.

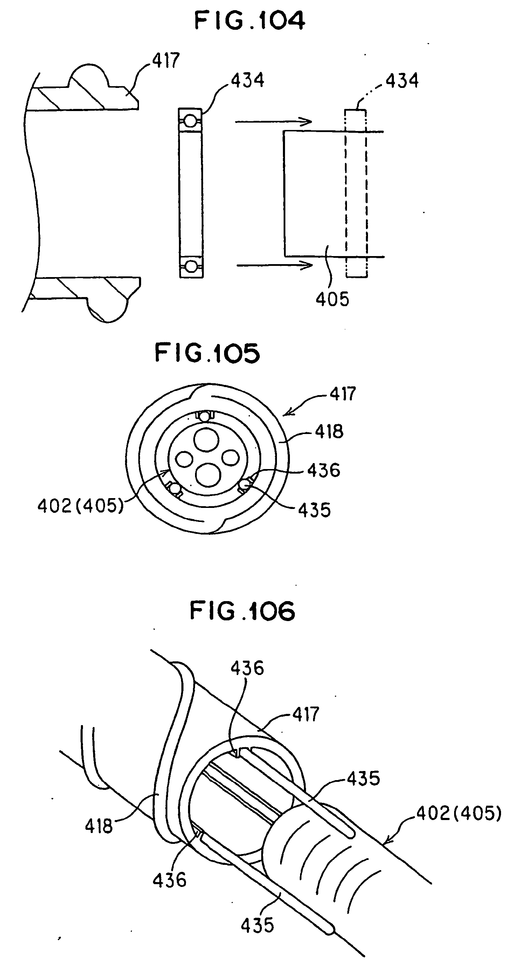

Fig. 104 shows a ninth modification. According to the ninth modification, the roller bearing holds the rotating member 417 so as to prevent the deviation of the central axis of the endoscope 402 and of the rotational central axis, similarly to the seventh modification. Specifically, referring to Fig. 104, the bearing 434 is used upon attaching the rotating member 417 to the distal-end portion 405.

That is, the bearing 434 is attached to the distal-end portion 405. Then, the rotating member 417 is attached such that bearing 434 is inserted between the distal-end portion 405 and the rotating member 417. According to the ninth modification, since the bearing 434 is hard to clean, the bearing 434 is made disposable.

According to the ninth modification, the rotating member 417 is freely rotatably held without fail, as compared with the case according to the eighth embodiment.

Figs. 105 and 106 show a tenth modification. According to the tenth modification, a plurality of rollers (needle bearings) 435 are used upon attaching the rotating member 417 because of the similar reason to that the seventh modification. Referring to Fig. 105, three rollers 435, for example, are freely rotatably held by stoppers 436 arranged at three positions on the inner circumferential surface of the rotating member 417.

In this case, referring to Fig. 106, the rollers 435 may be inserted into the stoppers 436 arranged on the inner circumferential surface of the rotating member 417 and then the distal-end portion 405 of the endoscope 402 may be inserted. In the state in which the rollers 435 are inserted in the halfway, the distal-end portion 405 of the endoscope 402 may be inserted.

According to the tenth modification, the rotating member 417 is freely rotatably held without fail, as compared with the case according to the eighth embodiment.

The number of the rollers 435 may increase.

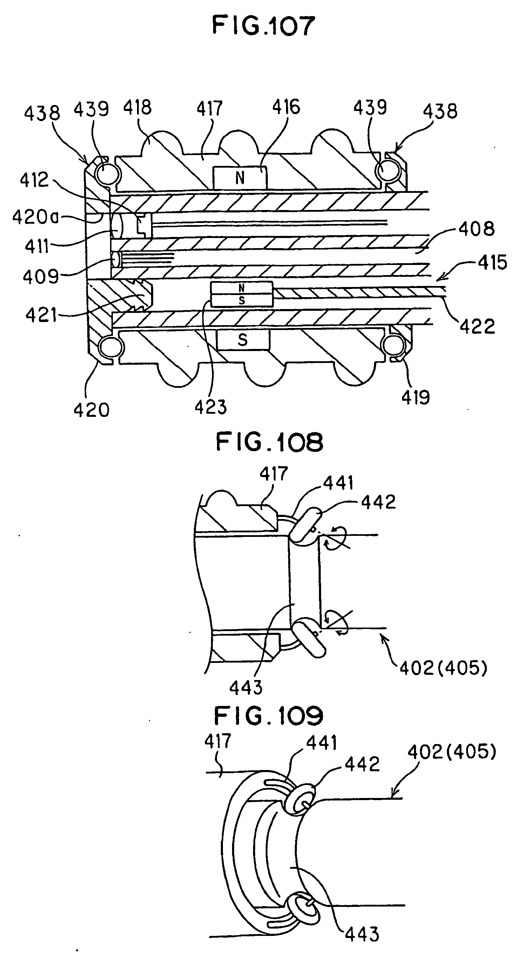

Fig. 107 shows an eleventh modification. According to the eleventh modification, a ball bearing 438 is used upon attaching the rotating member 417 because of the similar reason to that according to the seventh embodiment.

According to the eleventh modification, concaved portions slightly larger than the semi-spherical shape are formed at a plurality of positions, e.g., three or four positions on the surfaces facing the rotating member 417 of the fixing member 419 and the fixing member 420, and balls 439 are freely rotatably accommodated in the concaved portions.

Further, concaved portions slightly smaller than the semi-spherical shape are formed in the circumferential direction on the surfaces facing the fixing members 419 and 420 of the rotating member 417, and ball bearings 438 are formed to be freely rotatably in contact with the balls 439.

According to the eleventh modification, the rotating member 417 is freely rotatably held without fail.

Next, a twelfth modification will be described. According to the twelfth modification, a member with a small friction coefficient, e.g., Teflon (registered trademark) is formed by coating the contact portion between the outer circumferential surface of the distal-end portion 405 of the endoscope 402 and the rotating member 417. According to the twelfth modification, the friction is reduced, the slipping property is improved, and the rotating member 417 is smoothly rotated. Next, a thirteenth modification will be described with reference to Fig. 108. The thirteenth modification corresponds to the modification shown in Fig. 109. Since the movement of the rotating member 417 to the distal-end side is not mechanically regulated in the structure shown in Fig. 99, the rotating member 417 is moved from the desired position if there is not the large magnet shown in Fig. 96.

Then, according to the thirteenth modification, the rotating member 417 is regulated not so as to move to the distal-end side, even in the case of using the small magnet.

Referring to Figs. 108 and 109, at a plurality of positions on the rear surface of the rotating member 417 in the circumferential direction, a shaft portion 441 constituting of an elastic member is diagonally projected to the central axis of the distal-end portion 405 of the endoscope 402 from the axial direction of the rotating member 417. A roller or a tire 442 is freely rotatably attached to the shaft portion 441.

The tire 442 is energized to be engaged with a circumferential groove 443 formed by spherically cutting the outer circumferential surface of the distal-end portion 405 of the endoscope 402. Therefore, the tire 442 is elastically compressed to the inner wall of the circumferential groove 443 and is freely rotatably engaged with the circumferential groove 443. Further, the movement of the rotating member 417 to the distal-end side is regulated.

According to the thirteenth modification, there is provided a function of a movement prevention mechanism for preventing the forward/backward movement of the rotating member 417, and the rotating member 417 is smoothly and freely rotatably held as if the tire 442 was using the bearing.

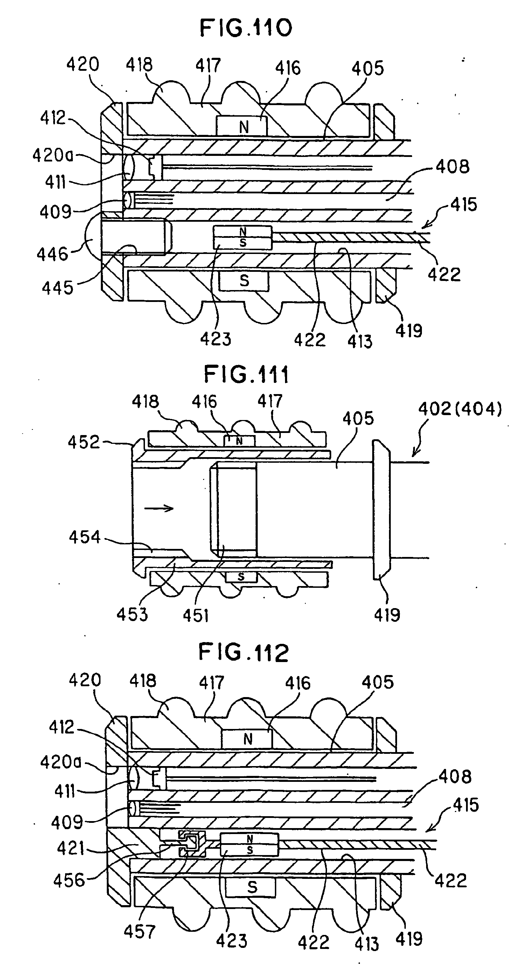

Fig. 110 shows a fourteenth modification. According to the fourteenth modification, a screw hole portion 445 is formed at the opening portion at the distal end of the channel 413 according to the eighth embodiment. A fixing screw 446 fixes the fixing member 420 on the side of the distal end thereof to the distal-end portion 405 via a hole or a screw hole of the fixing member 420.

That is, according to the eighth embodiment, the fixing member 420 on the side of the distal end is fixed by fitting, e.g., by pressing the fixing member 420 into the opening at the distal end of the channel 413. However, according to the fourteenth modification, the screw hole portion 445 is arranged by screw fixing at the opening at the distal end of the channel 413.

According to the fourteenth modification, the fixing member 420 is strongly fixed to the distal-end portion 405 and therefore the movement of the rotating member 417 to the distal-end side is prevented without fail.

Fig. 111 shows a fifteenth modification. According to the fifteenth modification, a male screw portion 451 is arranged onto the outer circumferential surface on the side of the distal end of the distal-end portion 405 of the endoscope 402. The male screw portion 451 is screwed to a female screw portion 454 arranged onto the inner circumferential surface of a cylinder 453 having a collar (flange portion) 452 on the outer circumference of the distal end, thereby fixing the cylinder 453 to the outer circumferential surface of the distal-end portion 405.

The collar 452 of the cylinder 453 and the fixing member 419 regulate the movement of the rotating member 417 in the longitudinal direction, thereby freely rotatably holding the rotating member 417. According to the fifteenth modification, it is possible to assuredly prevent the fixing member 420 from moving from the desired rotating position.

Fig. 112 shows a sixteenth modification. According to the sixteenth modification, the projected portion 421 according to the eighth embodiment is shaped to be fit into the opening of the distal end of the channel 413, and a projected portion 456 is arranged backward from the projected portion 421. The projected portion 456 is freely rotatably connected by a connecting member 457 projected from the distal end of the magnet 423 inserted in the channel 413. Specifically, a large-diameter portion is arranged to the proximal end of the projected portion 456, and a hollow portion for accommodating the large-diameter portion is arranged to the distal end of the connecting member 457, thereby freely rotatably connecting the projected portion 456 and the connecting member 457. Therefore, the magnet 423 is freely rotatably held to the extended portion 456. According to the sixteenth modification, the magnet 423 in the channel 413 is easily arranged at the position of the magnet 416 of the rotating member 417. The sixteenth modification has the similar advantages to those according to the fifteenth modification.

### (Ninth embodiment)

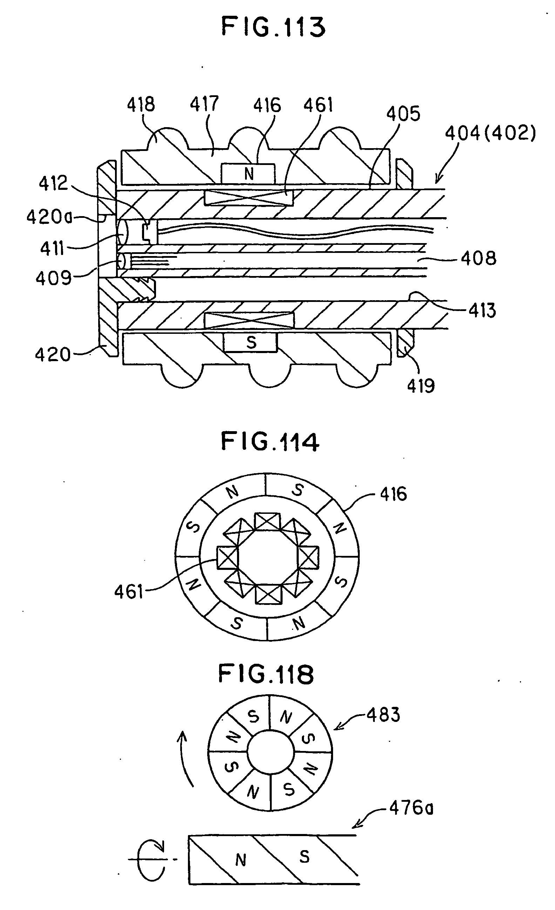

Next, a ninth embodiment of the present invention will be described with reference to Figs. 113 and 114. Fig. 113 shows an endoscope insertion aiding device according to the ninth embodiment of the present invention. The endoscope insertion aiding device 403 according to the ninth embodiment has the rotating member 417 and the fixing members 419 and 420, similarly to the eighth embodiment.

An electromagnet 461 having a function of the magnetic field applying member 415 according to the eighth embodiment is arranged at the position facing the magnet 416 arranged to the rotating member 417 on the side of the outer circumference of the electromagnet 461, on the outer circumferential surface of the distal-end portion 405 of the endoscope 402, thereby rotating the magnet 416 of the rotating member 417 by the direct driving system.

That is, the rotating member 417 and the fixing members 419 and 420 according to the eighth embodiment are used. According to the ninth embodiment, unlike the eighth embodiment, the endoscope 402 includes an electromagnet 461 having the operation for generating the rotating magnetic field. The electromagnet 461 is sealed so as to prevent the invasion of water from the outside.

Fig. 114 is an operation principle diagram of the direct driving system in this case.

Similarly to the rotating-magnet system, a plurality of the electromagnets 461 for generating the magnetic field in the diameter direction are arranged in the ring magnet 416. The magnetic field generated by the electromagnet 461 is changed, thereby rotating the ring magnet 416. As shown in Fig. 113, the electromagnet 461 is arranged to the endoscope 402 side, thereby forming a rotating mechanism for rotating the rotating member 417 having the magnet 416. A signal line connected to the electromagnet 461 is inserted in the endoscope 402, and is connected to a power supply device for generating the rotating magnetic field.

Other structures are the same as those according to the eighth embodiment, the same reference numerals denote the same components, and a description thereof is omitted. The side view and the front view according to the ninth embodiment are the same as Figs. 89 and 90 according to the eighth embodiment and therefore are not shown.

The ninth embodiment has the following advantages.

That is, the endoscope 402 is exclusively designed. However, similarly to the eighth embodiment, the rotating member 417 and the endoscope 402 is easily watertight-structured.

One modification of the ninth embodiment can use the fourth to fifteenth modifications, excluding the first to third modifications of the eighth embodiment.

### (Tenth embodiment)

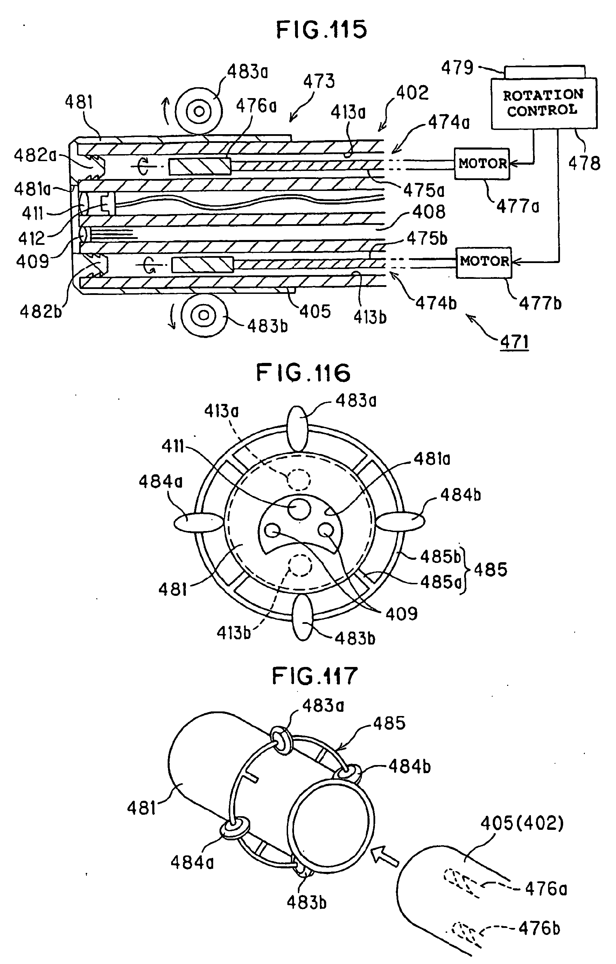

Next, a tenth embodiment of the present invention will be described with reference to Figs. 115 to 118. Fig. 115 shows a sectional structure when the endoscope insertion aiding device according to the tenth embodiment is attached to the endoscope. Fig. 116 is a front view of Fig. 115. Fig. 117 is a perspective view showing the state of attaching the endoscope insertion aiding device to the endoscope. Fig. 118 is a principle diagram showing the rotation.

An endoscope device 471 according to the tenth embodiment comprises: the endoscope 402 and an endoscope insertion aiding device 473 that is freely attachable and detachable to and from the endoscope 402.

The endoscope 402 according to the tenth embodiment is formed by arranging a plurality of channels 413a and 413b, in place of the one channel 413 of the endoscope 402 according to the eighth embodiment. In this case, referring to Fig. 116, the channels 413a and 413b are symmetrically arranged in the vertical direction of the central axis on the distal-end surface of the distal-end portion 405. Other structures in the endoscope 402 are similar to those of the endoscope 402 according to the eighth embodiment and therefore a description is given by using the same reference numerals.

Rotating magnetic-field applying members 474a and 474b are inserted in the channels 413a and 413b. In the rotating magnetic-field applying members 474a and 474b, stick magnets 476a and 476b are attached to the distal ends of flexible shafts 475a and 475b, and the proximal ends of the flexible shafts 475a and 475b are connected to motors 477a and 477b.

The motors 477a and 477b are connected to a rotation control circuit 478. An operating panel 479 arranged to the rotation control circuit 478 is operated, thereby synchronously rotating the motors 477a and 477b with the same phase and the inverse phase.

According to the tenth embodiment, a cylinder 481 is attached onto the outer circumferential surface of the distal-end portion 405 of the endoscope 402. The cylinder 481 has the inner diameter that is fit to the outer circumferential surface of the distal-end portion 405, and the distal-end portion 405 is inserted in the cylinder 481.

Projected portions 482a and 482b are arranged onto end surfaces (front-end surfaces) serving as the deep portion upon inserting the distal-end portion 405 in the cylinder 481. The projected portions 482a and 482b are pressed in the channels 413a and 413b, thereby fixing the cylinder 481 to the distal-end portion 405. Referring to Fig. 116, an opening 481a is arranged at least at portions of the illuminating window and the observing window on the front-end surface of the cylinder 481.

Referring to Fig. 117, on the side of the outer circumference of the cylinder 481, a supporting frame member 485 freely rotatably holds magnet tires (or rollers) 483a and 483b serving as rotating members and a non-magnet dummy tires (rollers) 484a and 484b.

Specifically, supporting frame members 485a projected in the radial outer direction are arranged at four positions in the circumferential direction on the outer circumferential surface of the cylinder 481. Ring supporting frame members 485b are continuously arranged to the distal ends of the supporting frame members 485a. The ring supporting frame members 485b freely rotatably have magnet circular disc tires 483a and 483b and non-magnet dummy tires 484a and 484b at the two facing positions in the vertical direction and at the two facing positions in the horizontal direction.

Referring to Fig. 116, therefore, the magnet tires 483a and 483b closely face the magnets 476a and 476b arranged in the channels 413a and 413b of the endoscope 402 therein. The motors 477a and 477b rotate the magnets 476a and 476b arranged in the channels 413a and 413b, thereby rotating the magnet tires 483a and 483b. In this case, the motors 477a and 477b are mutually rotated in the opposite directions and therefore the magnet tires 483a and 483b are rotated in the opposite directions each other.

Fig. 118 is a principle diagram of the rotation and the structure of the magnetic poles of the magnet 476a (similarly applied to the magnet 476b) and the tire 483a (similarly applied to the tire 483b).

The stick magnet 476a rotated around the shaft in the longitudinal direction is magnetized so as to alternately generate the N and S magnetic poles diagonally to the rotating shaft. On the contrary, the ring magnet forming the tire 483a is magnetized so as to alternately generate the N and S magnetic poles in the circumferential direction.

Therefore, the stick magnet 476a is rotated. Thus, in the ring magnet forming the tire 483a, the magnetic field is periodically changed at the magnet portion close to the magnet 476a. The periodically changed magnetic field rotates the tire 483a as shown by an arrow.

The operations according to the tenth embodiment are as follows. The inserting portion 404 of the endoscope 402 is inserted in the body cavity from the distal-end side. The user operates an operating panel 479, thereby rotating motors 477a and 477b in the opposite direction.

Then, the stick magnets 476a and 476b arranged in the channels 413a and 413b are rotated in the opposite direction each other. As shown in the principle diagram of Fig. 118, the magnet tires 483a and 483b are rotated in the opposite directions each other.

Accordingly, the side of the outer circumferences of the tires 483a and 483b operate the cylinder 481 and the distal-end portion 405 serving as the inside of the inner-wall surface of the body cavity to be thrust forward.

Since the tires 483a and 483b are individually operated, the advancing direction can be changed.

The operating panel 479 is operated, thereby setting the rotating speed of the motor 477a to be lower than the rotating speed of the motor 477b. Thus, the rotating speed of the upper tire 483a at the distal-end portion 405 is lower than the rotating speed of the down tire 483b and thus the distal-end portion 405 can be thrust in the up-bending direction.

The tenth embodiment has the following advantages.

That is, roller bearings of the tires 483a and 483b have higher cleaning property with the simple structure such as a slipping roller-bearing containing a low-friction material. Further, the tires 483a and 483b are individually operated and therefore the advancing direction can be changed.

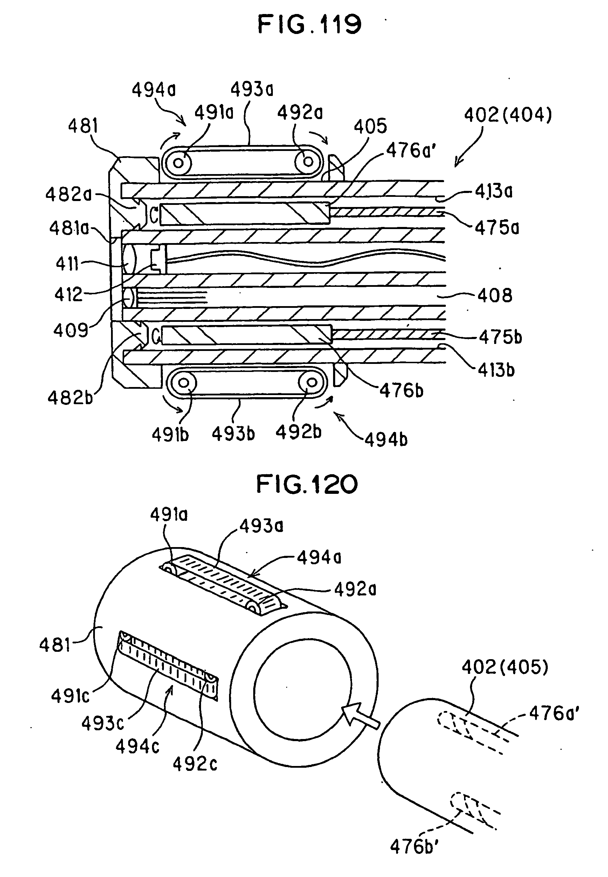

The first modification will be described with reference to Figs. 119 and 120. Fig. 119 is a sectional view showing the structure according to a first modification. According to the first modification, in place of the tires 483a and 483b according to the tenth embodiment, magnet rollers 491a and 492a and 491b and 492b serving as the pairs in the longitudinal direction are freely rotatably attached.

That is, concaved portions (groove portions) are arranged in the longitudinal direction of the cylinder 481 at the positions corresponding to the up and down directions (facing the channels 413a and 413b) on the outer circumferential surface of the cylinder 481. The grooves accommodate therein the magnet rollers 491a and 492a and 491b and 492b to be supported freely rotatably.

A belt caterpillar 493a is bridged between the pair of the rollers 491a and 492a, and a caterpillar 493b is bridged between the pair of the rollers 491b and 492b, thereby forming caterpillar driving mechanisms 494a and 494b.

Referring to Fig. 120, in place of the tires 484a and 484b according to the tenth embodiment, the non-magnet rollers 491c, 492c, 491d, and 492d serving as the pairs in the longitudinal direction are freely rotatably attached. Referring to Fig. 120, the rollers 491d and 492d are opposite to the rollers 491c and 492c and therefore are not shown.

A caterpillar 493c is bridged between the pair of the rollers 491c and 492c, and a caterpillar 493d is bridged between the pair of the rollers 491d and 492d, thereby forming dummy caterpillar driving mechanisms 494c and 494d. The caterpillar 493d and the caterpillar driving mechanism 494d are not shown.

According to the tenth embodiment, the stick magnets 476a and 476b are magnetized near the portions facing the tires 483a and 483b. However, according to the first modification, stick magnets 476a' and 476b' are formed by diagonally magnetizing the portions facing the rollers 491a and 492a and the rollers 491b and 492b.

Other structures are the same as those according to the tenth embodiment. According to the first modification, the rollers 491a and 492a are arranged serving as the pair in the longitudinal direction of the distal-end portion 405. Therefore, the distal-end portion 405 is stably thrust, as compared with the case according to the tenth embodiment. Except for this, the first modification has the same advantages as those according to the tenth embodiment.

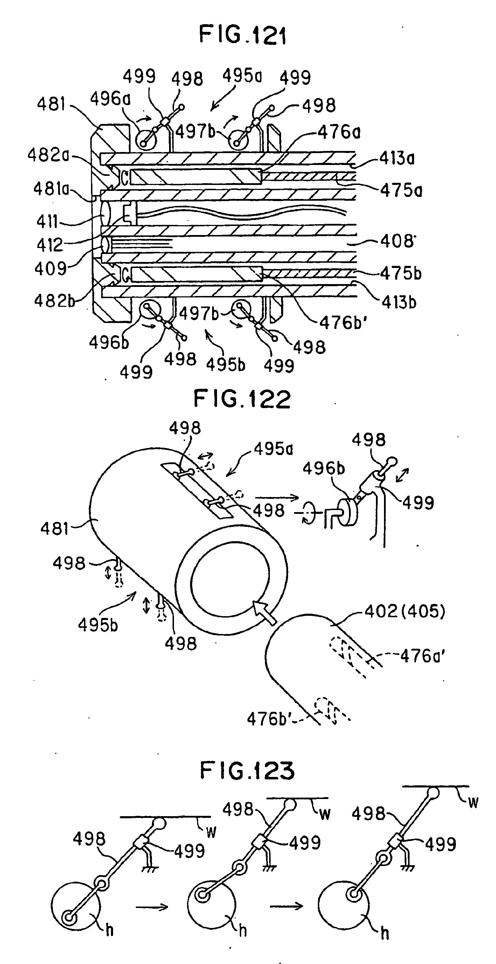

A second modification will be described with reference to Figs. 121 to 123. Fig. 121 is a sectional view showing the structure according to the second modification. According to the second modification, crank-pressing driving mechanism 495a and 495b are arranged, in place of the caterpillar driving mechanisms according to the first modification.

Referring to Figs. 121 and 122, concaved portions (groove portions) are arranged in the longitudinal direction of the cylinder 481 at the position corresponding of the cylinder 481 in the vertical direction. The concaved portions individually accommodates therein magnet wheels 496a, 497a, 496b, and 497b at two positions in the front and rear directions. Wheels h (h = 496a, 497a, 496b, and 497b) are freely rotatably supported in the cylinder 481.

Crank mechanisms are arranged in each of the wheels h. The rotation of the wheels h enables push rods 498 connected to the wheels h at first ends thereof to freely be projected and pulled (that is, the amount of projection is variable). The push rods 498 are inserted in rod holding cylinders 499 and are freely slidably held by the rod holding cylinders 499.

Fig. 123 is a principle diagram showing the crank-pressing driving mechanisms. Referring to Fig. 123, the wheels h are substantially half rotated, thereby projecting the push rods 498 such that the amount of projection gradually increases in the diagonally rear direction. The distal ends of the push rods 498 press a body cavity inner wall w in the diagonally rear direction. Thus, the body cavity inner wall w presses the cylinder 481 having the wheels h and the distal-end portion 405 in the front direction constituting the diagonally down direction.

As shown in Fig. 123, the wheels 496a and 497a are arranged to the top of the outer circumferential surface of the distal-end portion 405. Similarly, the wheels 496b and 497b arranged to the bottom of the outer circumferential surface of the distal-end portion 405 press the cylinder 481 and the distal-end portion 405 in the front direction constituting the diagonally up direction. That is, the cylinder 481 and the distal-end portion 405 are thrust and moved in the front direction.

As described according to the tenth embodiment, the rotating speeds of the motors 477a and 477b are controlled by operating the operating panel 479, thereby changing the thrust direction. Except for this, the second modification has the same advantages as those according to the first modification.

The embodiments may partly be combined and the present invention includes the combined embodiment.

### Industrial Applicability

The distal-end member and the like provided with the spiral structure are rotated, thereby making it possible to obtain a large thrust and smoothly insert the endoscope in the body or the like.

## Claims

1. An endoscope insertion aiding device (3) comprising:
a flexible tube (16);
a distal-end member (17) that is arranged to the distal end of the tube (16) and has the outer diameter larger than the outer diameter of the tube (16); and
a spiral structure (18) that is arranged onto the outer circumferential surface of the tube (16),
**characterised in that** the spiral structure (19) is also arranged to the outer circumferential surface of the distal-end member (17).

2. The endoscope insertion aiding device (3) according to Claim 1, wherein the distal-end member has a through-hole (17a) communicated with a hollow portion (16a) of the tube (16), and an inserting portion (7) of an endoscope (2) can be inserted into the through-hole (17a) from the proximal-end side of the tube (16).

3. The endoscope insertion aiding device (3) according to Claim 1, further comprising:
rotation driving means (21) that rotatably drives the tube (16).

4. The endoscope insertion aiding device (3) according to Claim 2, further comprising:
rotation driving means (21) that rotatably drives the tube (16).

5. The endoscope insertion aiding device (3) according to Claim 1, further comprising:
varying means that varies the height of projection from the outer circumferential surface of the spiral structure (18, 19) arranged onto the outer circumferential surface of at least one of the tube (16) and the distal-end member (17).

6. The endoscope insertion aiding device (3) according to Claim 2, further comprising:
varying means that varies the height of projection from the outer circumferential surface of the spiral structure (18, 19) arranged onto the outer circumferential surface of at least one of the tube (16) and the distal-end member (17).

7. The endoscope insertion aiding device (3) according to Claim 1, wherein the outer diameter of the distal-end member (17) varies.

8. The endoscope insertion aiding device according to Claim 1, wherein the spiral structure (18) arranged onto the outer circumferential surface of the tube (16) has a hollow structure.

9. The endoscope insertion aiding device (3) according to Claim 5, wherein the spiral structure (18, 19) arranged onto the outer circumferential surface of at least one of the tube (16) and the distal-end member (17) has a hollow structure, and fluid fed from a proximal-end operating portion to the hollow portion (16a) drives the varying means.

10. The endoscope insertion aiding device (3) according to Claim 1, further comprising:
a bending mechanism that bends at least one of the tube (16) and the distal-end member (17).

11. The endoscope insertion aiding device (3) according to Claim 10, wherein the bending mechanism contains a member contracted by applying a voltage.

12. The endoscope insertion aiding device (3) according to Claim 10, wherein the bending mechanism is arranged near the distal end of the tube (16).

13. The endoscope insertion aiding device (3) according to Claim 10, wherein the bending mechanism is bent in at least one direction.

14. The endoscope insertion aiding device (3) according to Claim 10, wherein the bending mechanism is bent by contracting a wire (78) on the side of the proximal-end operating portion.

15. The endoscope insertion aiding device (3) according to Claim 10, wherein the bending mechanism can be bent in a plurality of directions, and control means that controls the bending direction to be constant is arranged upon rotating the tube (16) while the bending mechanism is bent

16. The endoscope insertion aiding device (3) according to Claim 10, wherein the bending mechanism can be bent in only one direction, and control means that controls the insertion through a bent passage by repeating the bending, the rotation of the tube (16), the stop of rotation, and the release of bending is arranged.

17. The endoscope insertion aiding device (3) according to Claim 2, wherein the periphery of an overlapping portion of the tube in which the bending portion of the endoscope (2) is inserted contains a member softer than another portions.

18. The endoscope insertion aiding device (3) according to Claim 2, wherein the periphery of a connecting portion between the tube (16) and the distal-end member (17) contains a bendable soft member.

19. The endoscope insertion aiding device (3) according to Claim 3, wherein the rotation driving means (21) drives the rotation of the tube (16) by rotating force of a motor (23).

20. The endoscope insertion aiding device (3) according to Claim 19, wherein the motor (23) has a hollow rotating shaft (44a), and can insert the inserting portion of the endoscope (2) therein.

21. The endoscope insertion aiding device (3) according to Claim 3, wherein the rotation driving means (21) contains a plurality of electromagnets (47) arranged onto the outer circumferential surface of the tube (16) and a plurality of electromagnets (48) arranged onto the outer circumferences of the plurality of electromagnets.

22. The endoscope insertion aiding device (3) according to Claim 3, wherein the rotation driving means (21) has rotation regulating means that regulates the rotation of the tube (16) side when torque at a predetermined value or more is exerted.

23. The endoscope insertion aiding device according to Claim 22, wherein the rotation regulating means (62) comprises two disc members (62a, 62b) having friction surfaces in contact therewith by pressure.

24. The endoscope insertion aiding device (3) according to Claim 22, wherein the rotation regulating means (62) comprises the two disc members (62a, 62b) in contact therewith by pressure and a connecting member that keeps the connecting state of the two disc members (62a, 62b) and separates them by proper torque.

25. The endoscope insertion aiding device (3) according to Claim 22, wherein the rotation diving means (21) comprises a sensor (83) that detects the torque and control means (65) that stops the driving of rotation of the rotation driving means (21) by an output of the sensor (83).

26. The endoscope insertion aiding device (3) according to Claim 22, wherein the rotation regulating means (62) comprises a plurality of cylindrical members (46) arranged onto the outer circumferential surface of the tube (16) in the longitudinal direction thereof and spiral structures arranged to the outer circumferential surfaces of the cylindrical members (46).

27. The endoscope insertion aiding device (3) according to Claim 1, wherein the distal-end member (17) has the outer diameter that is reduced toward the distal end thereof.

28. The endoscope insertion aiding device (3) according to Claim 1, wherein the distal-end member (17) is taper-shaped with the outer diameter that is reduced as the distal-end member is near the distal end thereof.

29. The endoscope insertion aiding device (3) according to Claim 1, wherein the distal-end member (17) contains a soft material that can be bent by external force.

30. The endoscope insertion aiding device (3) according to Claim 1, wherein the outer diameter of the distal-end member (17) periodically changes.

31. The endoscope insertion aiding device (3) according to Claim 1, wherein the rigidity of the distal-end member (17) periodically changes.

32. The endoscope insertion aiding device (3) according to Claim 1, wherein the rigidity of the distal-end member (17) is softer near the distal end and continuously changes toward the proximal end.

33. The endoscope insertion aiding device (3) according to Claim 1, wherein the surface of the distal-end member (17) is lubricated.

34. The endoscope insertion aiding device (3) according to Claim 1, wherein the distal-end member (17) has a plurality of freely rotatably-connected hollow bead members (125) with the outer diameter equal to or more than that of the tube (16).

35. The endoscope insertion aiding device (3) according to Claim 9, wherein the spiral structure (18) arranged onto the outer circumferential surface of the tube (16) and the spiral structure (19) arranged onto the outer circumferential surface of the distal-end member (17) are respectively composed of a hollow tube (16a,17a), and both the hollow tubes (16a, 17a) are communicated with each other.

36. The endoscope insertion aiding device according to Claim 7, wherein means for varying the outer diameter of the distal-end member (17) comprises a balloon (52) arranged onto the outer circumferential surface of the distal-end member (17) and feed/discharge means that feeds/discharges fluid to/from the balloon (52).

37. The endoscope insertion aiding device (3) according to Claim 2, wherein the fluid is fed between the outer circumferential surface of the inserting portion (7) and the inner circumferential surface of the tube (16).

38. The endoscope insertion aiding device (3) according to Claim 2, wherein the interval between the outer circumferential surface of the inserting portion (7) and the inner circumferential surface of the tube (16) is freely rotatably sealed and an inner portion is filled with a lubrication agent.

39. The endoscope insertion aiding device (3) according to Claim 2, wherein a tube (16) freely rotatably held is inserted between the outer circumferential surface of the inserting portion (7) and the inner circumferential surface of the tube (16).

40. The endoscope insertion aiding device (3) according to Claim 1, further comprising;
a holder (22) that is attached to the side surface of the inserting portion (7) of the endoscope (2) on the distal end thereof and that movably holds the tube (16) having the spiral structure (18).

41. The endoscope insertion aiding device (3) according to Claim 40, wherein the holder(22) comprises rotation driving means (21) that drives the rotation of the tube (16).

42. The endoscope insertion aiding device (3) according to Claim 1, wherein the tube (16) having the spiral structure (18) can be inserted into a channel of the endoscope(2).

43. The endoscope insertion aiding device (3) according to Claim 5, further comprising:
a mechanism that detaches the spiral structure from the tube (16) after the insertion in the body cavity, the mechanism constituting means for removing the height of the spiral structure and flattening the tube (16).

44. The endoscope insertion aiding device (3) according to Claim 1, wherein the endoscope inserted after the endoscope (2) inserting aiding device (3) being inserted is a dedicated one having the cross-sectional shape wherein the endoscope insertion aiding device (3) is inserted to or detached from the down direction.

45. The endoscope insertion aiding device (3) according to Claim 1, wherein a concaved and convexed portion of the tube (16) made by the spiral structure is removed by overlaying another tube (16) to the tube (16) to smoothly insert the endoscope (2) after the endoscope insertion aiding device (3) being inserted.

46. The endoscope insertion aiding device (3) according to Claim 2, wherein a treatment tool can be inserted into the through-hole serving as an endoscope channel (91) and communicated with the distal-end member (17) and the tube (16).

## Patentansprüche

1. Endoskopeinführungs-Hiflsvorrichtung (3) mit:
- einem flexiblen Rohr (16);
- einem distalen Endelement (17), das an dem distalen Ende des Rohrs (16) angeordnet ist und dessen Außendurchmesser größer ist als der Außendurchmesser des Rohrs (16); und
- einer Spiralstruktur (18), die an der Außenumfangsfläche des Rohrs (16) angeordnet ist,
**dadurch gekennzeichnet, dass** die Spiralstruktur (19) auch an der Außenumfangsfläche des distalen Endelements (17) angeordnet ist.

2. Endoskopeinführungs-Hilfsvorrichtung (3) nach Anspruch 1, wobei das distale Endelement eine Durchgangsöffnung (17a) aufweist, die mit einem hohlen Abschnitt (16a) des Rohres (16) in Kommunikation steht, und ein Einführabschnitt (7) eines Endoskops (2) von der proximalen Endseite des Rohrs (16) durch die Durchgangsöffnung (17a) einführbar ist.

3. Endoskopeinführungs-Hilfsvorrichtung (3) nach Anspruch 1, ferner aufweisend:
- ein Rotationsantriebsmittel (21), das das Rohr (16) drehbar antreibt.

4. Endoskopeinführungs-Hilfsvorrichtung (3) nach Anspruch 2, ferner aufweisend:
- ein Rotationsantriebsmittel (21), das das Rohr (16) drehbar antreibt.

5. Endoskopeinführungs-Hilfsvorrichtung (3) nach Anspruch 1, ferner aufweisend:
- ein Änderungsmittel, das die Höhe ändert, um die die Spiralstruktur (18, 19), die an der Außenumfangsfläche des Rohrs (16) und/oder des distalen Endelements (17) angeordnet ist, von der Außenumfangsfläche vorsteht.

6. Endoskopeinführungs-Hilfsvorrichtung (3) nach Anspruch 2, ferner aufweisend:
- ein Änderungsmittel, das die Höhe ändert, um die die Spiralstruktur (18, 19), die an der Außenumfangsfläche des Rohres (16) und/oder des distalen Endelements (17) angeordnet ist, von der Außenumfangsfläche vorsteht.

7. Endoskopeinführungs-Hilfsvorrichtung (3) nach Anspruch 1, wobei der Außendurchmesser des distalen Endelements (17) variiert.

8. Endoskopeinführungs-Hilfsvorrichtung (3) nach Anspruch 1, wobei die Spiralstruktur (18), die an der Außenumfangsfläche des Rohrs (16) angeordnet ist, eine hohle Struktur aufweist.

9. Endoskopeinführungs-Hilfsvorrichtung (3) nach Anspruch 5, wobei die Spiralstruktur (18, 19), die an der Außenumfangsfläche des Rohrs (16) und/oder des distalen Endelements (17) angeordnet ist, eine hohle Struktur aufweist, und ein von einem Betätigungsabschnitt am proximalen Ende zu dem hohlen Abschnitt (16a) zugeführtes Fluid das Änderungsmittel antreibt.

10. Endoskopeinführungs-Hilfsvorrichtung (3) nach Anspruch 1, ferner aufweisend:
- einen Biegemechanismus, der das Rohr (16) und/oder das distale Endelement (17) biegt.

11. Endoskopeinführungs-Hilfsvorrichtung (3) nach Anspruch 10, wobei der Biegemechanismus ein Element umfasst, das durch das Anlegen einer Spannung zusammenzieht.

12. Endoskopeinführungs-Hilfsvorrichtung (3) nach Anspruch 10, wobei der Biegemechanismus nahe dem distalen Ende des Rohrs (16) angeordnet ist.

13. Endoskopeinführungs-Hilfsvorrichtung (3) nach Anspruch 10, wobei der Biegemechanismus in wenigstens eine Richtung gebogen ist.

14. Endoskopeinführungs-Hilfsvorrichtung (3) nach Anspruch 10, wobei der Biegemechanismus durch Zusammenziehen eines Drahts (7) an der Seite des Betätigungsabschnitts am proximalen Ende gebogen wird.

15. Endoskopeinführungs-Hilfsvorrichtung (3) nach Anspruch 10, wobei der Biegemechanismus in eine Mehrzahl von Richtungen biegbar ist, und ein Steuermittel angeordnet ist, das die Biegerichtung beim Drehen des Rohrs (16) so steuert, dass sie konstant ist, während der Biegemechanismus gebogen wird.

16. Endoskopeinführungs-Hilfsvorrichtung (3) nach Anspruch 10, wobei der Biegemechanismus nur in eine Richtung biegbar ist, und ein Steuermittel angeordnet ist, das die Einführung durch eine gebogene Passage durch wiederholtes Biegen, die Rotation des Rohrs (16), das Stoppen der Rotation, und die Rücknahme der Biegung steuert.

17. Endoskopeinführungs-Hilfsvorrichtung (3) nach Anspruch 2, wobei der Umfang eines überlappenden Abschnitts des Rohrs, in das der Biegeabschnitt des Endoskops (2) eingeführt ist, ein Element aufweist, das weicher ist als andere Abschnitte.

18. Endoskopeinführungs-Hilfsvorrichtung (3) nach Anspruch 2, wobei der Umfang eines Verbindungsabschnitts zwischen dem Rohr (16) und dem distalen Endelement (17) ein biegbares, weiches Element aufweist.

19. Endoskopeinführungs-Hilfsvorrichtung (3) nach Anspruch 3, wobei das Rotationsantriebsmittel (21) die Rotation des Rohrs (16) durch eine Rotationskraft eines Motors (23) antreibt.

20. Endoskopeinführungs-Hilfsvorrichtung (3) nach Anspruch 19, wobei der Motor (23) eine hohle Rotationswelle (44a) aufweist und den Einführabschnitt des Endoskops (2) darin aufnehmen kann.

21. Endoskopeinführungs-Hilfsvorrichtung (3) nach Anspruch 3, wobei das Rotationsantriebsmittel (21) eine Mehrzahl von Elektromagneten (47), die an der Außenumfangsfläche des Rohrs (16) angeordnet sind, und eine Mehrzahl von Elektromagneten (48) aufweist, die an den Außenumfängen der Mehrzahl von Elektromagneten angeordnet sind.

22. Endoskopeinführungs-Hilfsvorrichtung (3) nach Anspruch 3, wobei das Rotationsantriebsmittel (21) ein Rotationsregelmittel aufweist, das die Rotation der Seite des Rohrs (16) regelt, wenn ein Drehmoment von einem vorbestimmten Wert oder größer angelegt wird.

23. Endoskopeinführungs-Hilfsvorrichtung (3) nach Anspruch 22, wobei das Rotationsregelmittel (62) zwei Scheibenelemente (62a, 62b) aufweist, die durch Druck in Kontakt stehende Reibflächen umfassen.

24. Endoskopeinführungs-Hilfsvorrichtung (3) nach Anspruch 22, wobei das Rotationsregelmittel (62) die zwei durch Druck in Kontakt stehende Scheibenelemente (62a, 62b) und ein Verbindungselement aufweist, das den Verbindungszustand der zwei Scheibenelemente (62a, 62b) aufrechterhält und sie durch ein passendes Drehmoment voneinander trennt.

25. Endoskopeinführungs-Hilfsvorrichtung (3) nach Anspruch 22, wobei das Rotationsantriebsmittel (21) einen Sensor (83), der das Drehmoment erfasst, und ein Steuermittel (65) aufweist, das den Antrieb des Rotationsantriebsmittels (21) durch eine Ausgabe des Sensors (83) anhält.

26. Endoskopeinführungs-Hilfsvorrichtung (3) nach Anspruch 22, wobei das Rotationsregelmittel (62) eine Mehrzahl von zylindrischen Elementen (46) aufweist, die an der Außenumfangsfläche des Rohrs (16) in dessen Längsrichtung angeordnet sind und die Spiralstrukturen an den Außenumfangsflächen der zylindrischen Elemente (46) angeordnet sind.

27. Endoskopeinführungs-Hilfsvorrichtung (3) nach Anspruch 1, wobei sich der Außendurchmesser des distalen Endelements (17) in Richtung des distalen Endes des distalen Endelements (17) verkleinert.

28. Endoskopeinführungs-Hilfsvorrichtung (3) nach Anspruch 1, wobei das distale Endelement konusförmig mit einem Außendurchmesser ausgebildet ist, der nahe dem distalen Ende des distalen Endelements verkleinert ist.

29. Endoskopeinführungs-Hilfsvorrichtung (3) nach Anspruch 1, wobei das distale Endelement (17) ein weiches Material enthält, das durch eine externe Kraft biegbar ist.

30. Endoskopeinführungs-Hilfsvorrichtung (3) nach Anspruch 1, wobei sich der Außendurchmesser des distalen Endelements (17) sich periodisch verändert.

31. Endoskopeinführungs-Hilfsvorrichtung (3) nach Anspruch 1, wobei sich die Steifigkeit des distalen Endelements (17) periodisch verändert.

32. Endoskopeinführungs-Hilfsvorrichtung (3) nach Anspruch 1, wobei die Steifigkeit des distalen Endelements (17) nahe dem distalen Ende weicher ist und sich kontinuierlich in Richtung des proximalen Endes verändert.

33. Endoskopeinführungs-Hilfsvorrichtung (3) nach Anspruch 1, wobei die Fläche des distalen Endelements (17) geschmiert ist.

34. Endoskopeinführungs-Hilfsvorrichtung (3) nach Anspruch 1, wobei das distale Endelement (17) eine Mehrzahl von frei drehbar miteinander verbundenen hohlen Kugelelementen (25) mit einem Außendurchmesser aufweist, der gleich dem oder größer als der Außendurchmesser des Rohrs (16) ist.

35. Endoskopeinführungs-Hilfsvorrichtung (3) nach Anspruch 9, wobei die Spiralstruktur (18), die an der Außenumfangsfläche des Rohrs (16) angeordnet ist, und die Spiralstruktur (19), die an der Außenumfangsfläche des distalen Endelements (17) angeordnet ist, jeweils von einem hohlen Rohr (16a, 17a) gebildet werden und beide hohle Rohre (16a, 17a) miteinander in Kommunikation stehen.

36. Endoskopeinführungs-Hilfsvorrichtung (3) nach Anspruch 7, wobei ein Mittel zum Verändern des Außendurchmessers des distalen Endelements (17) einen Ballon (52), der an der Außenumfangsfläche des distalen Endelements (17) angeordnet ist, und ein Zuführ-/Ablassmittel, das ein Fluid zu/von dem Ballon (52) zuführt/abführt, aufweist.

37. Endoskopeinführungs-Hilfsvorrichtung (3) nach Anspruch 2, wobei das Fluid zwischen der Außenumfangsfläche des Einführabschnitts (7) und der Innenumfangsfläche des Rohrs (16) zugeführt wird.

38. Endoskopeinführungs-Hilfsvorrichtung (3) nach Anspruch 2, wobei das Intervall zwischen der Außenumfangsfläche des Einführabschnitts (7) und der Innenumfangsfläche des Rohrs (16) frei drehbar abgedichtet ist und ein Innenabschnitt mit einem Schmiermittel gefüllt ist.

39. Endoskopeinführungs-Hilfsvorrichtung (3) nach Anspruch 2, wobei ein frei drehbar gehaltenes Rohr (16) zwischen der Außenumfangsfläche des Einführabschnitts (7) und der Innenumfangsfläche des Rohrs (16) einführbar ist.

40. Endoskopeinführungs-Hilfsvorrichtung (3) nach Anspruch 1, ferner aufweisend:
- einen Halter (22), der an der Seitenfläche des Einführabschnitts (7) des Endoskops (2) an dessen distalem Ende angeordnet ist, und der das Rohr (16) mit der Spiralstruktur (18) bewegbar hält.

41. Endoskopeinführungs-Hilfsvorrichtung (3) nach Anspruch 40, wobei der Halter (22) ein Rotationsantriebsmittel (21) aufweist, das die Rotation des Rohrs (16) antreibt.

42. Endoskopeinführungs-Hilfsvorrichtung (3) nach Anspruch 1, wobei das Rohr (16) mit der Spiralstruktur (18) in einen Kanal des Endoskops (2) eingeführt werden kann.

43. Endoskopeinführungs-Hilfsvorrichtung (3) nach Anspruch 5, ferner aufweisend:
- einen Mechanismus, der die Spiralstruktur von dem Rohr (16) nach der Einführung in die Körperhöhle abkoppelt, wobei der Mechanismus ein Mittel zum Entfernen der Höhe der Spiralstruktur und zum Abflachen des Rohrs (16) bildet.

44. Endoskopeinführungs-Hilfsvorrichtung (3) nach Anspruch 1, wobei das nach der Endoskopeinführungs-Hilfsvorrichtung (3) eingeführte Endoskop ein vobestimmtes Endoskop mit einer Querschnittsform ist, in die die Endoskopeinführungs-Hilfsvorrichtung (3) von unten einführbar oder abnehmbar ist.

45. Endoskopeinführungs-Hilfsvorrichtung (3) nach Anspruch 1, wobei ein konkaver und konvexer Abschnitt des Rohrs (16), der durch die Spiralstruktur entsteht, durch Überlappen eines anderen Rohrs (16) mit dem Rohr (16) entfernt wird, um das Endoskop (2) nach der Endoskopeinführungs-Hilfsvorrichtung (3) einfach einzuführen.

46. Endoskopeinführungs-Hilfsvorrichtung (3) nach Anspruch 2, wobei ein Behandlungsinstrument durch die Durchgangsöffnung eingeführt werden kann, die als Endoskopkanal (91) dient und mit dem distalen Endelement (17) und dem Rohr (16) kommuniziert.

## Revendications

1. Dispositif d'aide à l'insertion d'un endoscope (3) comprenant :
un tube flexible (16) ;
un élément d'extrémité distale (17) qui est disposé sur l'extrémité distale du tube (16) et présente un diamètre externe supérieur au diamètre externe du tube (16) ; et
une structure en spirale (18) qui est prévue sur la surface circonférentielle externe du tube (16),
**caractérisé en ce que** la structure en spirale (19) est également prévue sur la surface circonférentielle externe de l'élément d'extrémité distale (17).

2. Dispositif d'aide à l'insertion d'un endoscope (3) selon la revendication 1, dans lequel l'élément d'extrémité distale comporte un trou traversant (17a) en communication avec une partie creuse (16a) du tube (16), et une partie d'insertion (7) d'un endoscope (2) peut être insérée dans le trou traversant (17a) à partir du côté d'extrémité proximale du tube (16).

3. Dispositif d'aide à l'insertion d'un endoscope (3) selon la revendication 1, comprenant en outre :
un moyen d'entraînement en rotation (21) qui entraîne le tube (16) en rotation.

4. Dispositif d'aide à l'insertion d'un endoscope (3) selon la revendication 2, comprenant en outre :
un moyen d'entraînement en rotation (21) qui entraîne le tube (16) en rotation.

5. Dispositif d'aide à l'insertion d'un endoscope (3) selon la revendication 1, comprenant en outre :
un moyen de variation qui fait varier la hauteur de saillie depuis la surface circonférentielle externe de la structure en spirale (18, 19) prévue sur la surface circonférentielle externe d'au moins l'un parmi le tube (16) et l'élément d'extrémité distale (17).

6. Dispositif d'aide à l'insertion d'un endoscope (3) selon la revendication 2, comprenant en outre :
un moyen de variation qui fait varier la hauteur de saillie depuis la surface circonférentielle externe de la structure en spirale (18, 19) prévue sur la surface circonférentielle externe d'au moins l'un parmi le tube (16) et l'élément d'extrémité distale (17).

7. Dispositif d'aide à l'insertion d'un endoscope (3) selon la revendication 1, dans lequel le diamètre externe de l'élément d'extrémité distale (17) varie.

8. Dispositif d'aide à l'insertion d'un endoscope selon la revendication 1, dans lequel la structure en spirale (18) prévue sur la surface circonférentielle externe du tube (16) comporte une structure creuse.

9. Dispositif d'aide à l'insertion d'un endoscope (3) selon la revendication 5, dans lequel la structure en spirale (18, 19) prévue sur la surface circonférentielle externe d'au moins l'un parmi le tube (16) et l'élément d'extrémité distale (17) comporte une structure creuse, et l'alimentation en fluide d'une partie d'actionnement d'extrémité proximale à la partie creuse (16a) entraîne le moyen de variation.

10. Dispositif d'aide à l'insertion d'un endoscope (3) selon la revendication 1, comprenant en outre :
un mécanisme de cintrage qui cintre au moins l'un parmi le tube (16) et l'élément d'extrémité distale (17).

11. Dispositif d'aide à l'insertion d'un endoscope (3) selon la revendication 10, dans lequel le mécanisme de cintrage contient un élément contracté en appliquant une tension.

12. Dispositif d'aide à l'insertion d'un endoscope (3) selon la revendication 10, dans lequel le mécanisme de cintrage est prévu près de l'extrémité distale du tube (16).

13. Dispositif d'aide à l'insertion d'un endoscope (3) selon la revendication 10, dans lequel le mécanisme de cintrage est cintré dans au moins une direction.

14. Dispositif d'aide à l'insertion d'un endoscope (3) selon la revendication 10, dans lequel le mécanisme de cintrage est cintré en contractant un câble (78) sur le côté de la partie d'actionnement d'extrémité proximale.

15. Dispositif d'aide à l'insertion d'un endoscope (3) selon la revendication 10, dans lequel le mécanisme de cintrage peut être cintré dans une pluralité de directions, et un moyen de commande qui commande la direction de cintrage pour qu'elle soit constante est disposé sur rotation du tube (16) alors que le mécanisme de cintrage est cintré.

16. Dispositif d'aide à l'insertion d'un endoscope (3) selon la revendication 10, dans lequel le mécanisme de cintrage peut être cintré dans seulement une direction, et un moyen de commande est prévu qui commande l'insertion à travers un passage cintré en répétant le cintrage, la rotation du tube (16), l'arrêt de la rotation et le relâchement du cintrage.

17. Dispositif d'aide à l'insertion d'un endoscope (3) selon la revendication 2, dans lequel la périphérie d'une partie de chevauchement du tube dont la partie de cintrage de l'endoscope (2) est insérée contient un élément plus souple que les autres parties.

18. Dispositif d'aide à l'insertion d'un endoscope (3) selon la revendication 2, dans lequel la périphérie d'une partie de raccordement entre le tube (16) et l'élément d'extrémité distale (17) contient un élément souple pouvant être cintré.

19. Dispositif d'aide à l'insertion d'un endoscope (3) selon la revendication 3, dans lequel le moyen d'entraînement en rotation (21) entraîne la rotation du tube (16) par la force de rotation d'un moteur (23).

20. Dispositif d'aide à l'insertion d'un endoscope (3) selon la revendication 19, dans lequel le moteur (23) comporte un arbre rotatif creux (44a), et peut insérer la partie d'insertion de l'endoscope (2) dans celui-ci.

21. Dispositif d'aide à l'insertion d'un endoscope (3) selon la revendication 3, dans lequel le moyen d'entraînement en rotation (21) contient une pluralité d'électroaimants (47) disposés sur la surface circonférentielle externe du tube (16) et une pluralité d'électroaimants (48) disposés sur les circonférences externes de la pluralité d'électroaimants.

22. Dispositif d'aide à l'insertion d'un endoscope (3) selon la revendication 3, dans lequel le moyen d'entraînement en rotation (21) comporte un moyen de régulation de rotation qui régule la rotation du côté du tube (16) lorsqu'un couple à une valeur prédéterminée ou plus est exercé.

23. Dispositif d'aide à l'insertion d'un endoscope selon la revendication 22, dans lequel le moyen de régulation de rotation (62) comprend deux éléments circulaires (62a, 62b) comportant des surfaces de frottement en contact par pression.

24. Dispositif d'aide à l'insertion d'un endoscope (3) selon la revendication 22, dans lequel le moyen de régulation de rotation (62) comprend les deux éléments circulaires (62a, 62b) en contact par pression et un élément de raccordement qui maintient l'état de raccordement des deux éléments circulaires (62a, 62b) et les sépare par un couple approprié.

25. Dispositif d'aide à l'insertion d'un endoscope (3) selon la revendication 22, dans lequel le moyen d'entraînement en rotation (21) comprend un capteur (83) qui détecte le couple et un moyen de commande (65) qui arrête l'entraînement en rotation du moyen d'entraînement en rotation (21) par une sortie du capteur (83).

26. Dispositif d'aide à l'insertion d'un endoscope (3) selon la revendication 22, dans lequel le moyen de régulation de rotation (62) comprend une pluralité d'éléments cylindriques (46) disposés sur la surface circonférentielle externe du tube (16) dans son sens longitudinal et des structures en spirale disposées sur les surfaces circonférentielles externes des éléments cylindriques (46).

27. Dispositif d'aide à l'insertion d'un endoscope (3) selon la revendication 1, dans lequel l'élément d'extrémité distale (17) possède le diamètre externe qui est réduit vers l'extrémité distale de celui-ci.

28. Dispositif d'aide à l'insertion d'un endoscope (3) selon la revendication 1, dans lequel l'élément d'extrémité distale (17) est de forme conique avec le diamètre externe qui est réduit à mesure que l'élément d'extrémité distale se rapproche de l'extrémité distale de celui-ci.

29. Dispositif d'aide à l'insertion d'un endoscope (3) selon la revendication 1, dans lequel l'élément d'extrémité distale (17) contient un matériau souple qui peut être cintré par une force externe.

30. Dispositif d'aide à l'insertion d'un endoscope (3) selon la revendication 1, dans lequel le diamètre externe de l'élément d'extrémité distale (17) change périodiquement.

31. Dispositif d'aide à l'insertion d'un endoscope (3) selon la revendication 1, dans lequel la rigidité de l'élément d'extrémité distale (17) change périodiquement.

32. Dispositif d'aide à l'insertion d'un endoscope (3) selon la revendication 1, dans lequel la rigidité de l'élément d'extrémité distale (17) est plus souple près de l'extrémité distale et change de manière continue vers l'extrémité proximale.

33. Dispositif d'aide à l'insertion d'un endoscope (3) selon la revendication 1, dans lequel la surface de l'élément d'extrémité distale (17) est lubrifiée.

34. Dispositif d'aide à l'insertion d'un endoscope (3) selon la revendication 1, dans lequel l'élément d'extrémité distale (17) comporte une pluralité d'éléments de perles creuses raccordées en rotation libre (125) avec le diamètre externe est égal ou supérieur à celui du tube (16).

35. Dispositif d'aide à l'insertion d'un endoscope (3) selon la revendication 9, dans lequel la structure en spirale (18) prévue sur la surface circonférentielle externe du tube (16) et la structure en spirale (19) prévue sur la surface circonférentielle externe de l'élément d'extrémité distale (17) sont respectivement composées d'un tube creux (16a, 17a), et les deux tubes creux (16a, 17a) sont en communication l'un avec l'autre.

36. Dispositif d'aide à l'insertion d'un endoscope selon la revendication 7, dans lequel un moyen destiné à faire varier le diamètre externe de l'élément d'extrémité distale (17) comprend un ballonnet (52) disposé sur la surface circonférentielle externe de l'élément d'extrémité distale (17) et un moyen d'alimentation/refoulement qui délivre/refoule du fluide vers/depuis le ballonnet (52).

37. Dispositif d'aide à l'insertion d'un endoscope (3) selon la revendication 2, dans lequel le fluide est délivré entre la surface circonférentielle externe de la partie d'insertion (7) et la surface circonférentielle interne du tube (16).

38. Dispositif d'aide à l'insertion d'un endoscope (3) selon la revendication 2, dans lequel l'intervalle entre la surface circonférentielle externe de la partie d'insertion (7) et la surface circonférentielle interne du tube (16) est fermé de manière étanche en rotation libre et une partie interne est remplie d'un agent lubrifiant.

39. Dispositif d'aide à l'insertion d'un endoscope (3) selon la revendication 2, dans lequel un tube (16) maintenu en rotation libre est inséré entre la surface circonférentielle externe de la partie d'insertion (7) et la surface circonférentielle interne du tube (16).

40. Dispositif d'aide à l'insertion d'un endoscope (3) selon la revendication 1, comprenant en outre :
un dispositif de maintien (22) qui est fixé à la surface latérale de la partie d'insertion (7) de l'endoscope (2) sur l'extrémité distale de celui-ci et qui maintient de manière mobile le tube (16) comportant la structure en spirale (18).

41. Dispositif d'aide à l'insertion d'un endoscope (3) selon la revendication 40, dans lequel le dispositif de maintien (22) comprend un moyen d'entraînement en rotation (21) qui entraîne la rotation du tube (16).

42. Dispositif d'aide à l'insertion d'un endoscope (3) selon la revendication 1, dans lequel le tube (16) comportant la structure en spirale (18) peut être inséré dans un canal de l'endoscope (2).

43. Dispositif d'aide à l'insertion d'un endoscope (3) selon la revendication 5, comprenant en outre :
un mécanisme qui détache la structure en spirale du tube (16) après l'insertion dans la cavité de corps, le mécanisme constituant un moyen destiné à supprimer la hauteur de la structure en spirale et à aplatir le tube (16).

44. Dispositif d'aide à l'insertion d'un endoscope (3) selon la revendication 1, dans lequel l'endoscope inséré après que le dispositif (3) d'aide à l'insertion d'un endoscope (2) ait été inséré est un endoscope spécialisé présentant une forme de section transversale dans laquelle le dispositif d'aide à l'insertion d'un endoscope (3) est inséré vers ou détaché de la direction en aval.

45. Dispositif d'aide à l'insertion d'un endoscope (3) selon la revendication 1, dans lequel une partie concave et convexe du tube (16) composé par la structure en spirale est enlevée en superposant un autre tube (16) sur le tube (16) pour insérer en douceur l'endoscope (2) après que le dispositif d'aide à l'insertion d'un endoscope (3) ait été inséré.

46. Dispositif d'aide à l'insertion d'un endoscope (3) selon la revendication 2, dans lequel un outil de traitement peut être inséré dans le trou traversant servant de canal d'endoscope (91) et communique avec l'élément d'extrémité distale (17) et le tube (16).
